# EUROPEAN PATENT APPLICATION

(11) **EP 4 631 976 A1**
(43) Date of publication of application: **15.10.2025**
(21) Application number: 23902380.7
(22) Date of filing: 10.11.2023
(51) Int. Cl.: C07K 16/28, C12N 15/13, C12N 5/10, C12N 15/63, A61K 39/395, A61P 35/00, A61P 37/00, A61P 31/00, G01N 33/68

(54) **ANTI-CD155 ANTIBODY AND USE THEREOF**

(30) Priority: 12.12.2022 CN 202211600706
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Studio Torta S.p.A.
(86) International application number: PCT/CN2023/131016
(87) International publication number: WO 2024/125180

(57) **Abstract**

Provided in the present invention are an anti-CD155 antibody and the use thereof. Moreover, an obtained anti-CD155 chimeric antibody, a humanized anti-CD155 antibody, and a humanized affinity-matured anti-CD155 antibody can only specifically target and bind to a CD155 receptor and block the binding of CD155 to receptors TIGIT, CD96, and CD226 thereof, but also have the characteristic of not inducing the apoptosis of CD155 cells.

## Description

### FIELD

The present disclosure relates to the field of biomedicine, and more particularly, to an anti-CD155 antibody and use thereof.

### BACKGROUND

CD155, also known as NECL-5 or PVR, is a type I transmembrane protein with an extracellular segment containing one IgV domain and two IgC domains and an intracellular segment containing an ITIM motif. Studies have revealed that CD155 can be shed from the surface of the cell to form soluble CD155 protein. CD155 is expressed on the surface of tissue cells such as monocytes and dendritic cells, and is upregulated in a variety of tumor tissues. Studies have shown that CD155 promotes tumor invasion and metastasis, and the high expression of CD155 is associated with poor tumor prognosis.

NK cells and T cells express a variety of CD155 receptors on their surfaces, including TIGIT, CD96, and CD226, of which TIGIT and CD96 are inhibitory receptors, and CD226 is an activating receptor. The immune checkpoint antibodies targeting TIGIT and CD96 have entered the clinical trial stage, among them, the TIGIT monoclonal antibody from Roche is the most advanced, currently in the Phase III clinical stage. The CD155 shed from the surface of the cells can bind to CD226, downregulate CD226, and weaken the anti-cancer function of NK cells and T cells. Eli Lily is currently developing an agonistic CD226 antibody.

Studies have found that patients suffering from non-small cell lung cancer and melanoma with high expression of CD155 have a worse prognosis and a poorer response to PD-1/L1 therapy. Therefore, it is necessary to develop an antibody medicament targeting CD155, which can be suitable for PD-L1-negative patients on the one hand, and improve the response to PD-1/L1 therapy on the other hand. To date, only one CD155 antibody has entered the clinical trial stage, namely Nectin's NTX-1088 antibody (named NB1088 in the early discovery stage). This antibody is of the IgG4 subtype and can block CD155 from binding to its receptors TIGIT, CD96, and CD226. Our research found that this antibody strongly induced apoptosis of CD155-positive cells, while human peripheral blood mononuclear cells and a variety of normal tissue cells expressed CD155, suggesting that this antibody may have safety risks in clinical application. Another CD155 antibody SKII.4 commonly used in scientific research is also a blocking antibody that can block the binding of CD155 to its receptor. Our research found that this antibody also strongly induces apoptosis of CD155-positive cells.

Therefore, thereof is still a need to continue to research and develop antibody medicaments targeting CD155 with high safety.

### SUMMARY

The present disclosure is based on the inventor's discovery of the following issues and facts.

CD155 is expressed on the surface of tissue cells such as monocytes and dendritic cells, and is upregulated in a variety of tumor tissues. CD155 promotes tumor invasion and metastasis, and high expression of CD155 is associated with poor tumor prognosis. However, since CD155 binds to three receptors, TIGIT, CD96, and CD226, it is relatively difficult to screen blocking anti-CD155 monoclonal antibodies. Secondly, the existing antibodies that block the CD155 receptor still have the safety risk of inducing apoptosis of CD155-positive normal tissue cells. Therefore, it is relatively difficult to design and develop monoclonal antibodies against CD155.

The inventors of the present disclosure successfully obtained a blocking CD155 antibody that does not induce cell apoptosis through screening. Specifically, the inventors screened a series of high-affinity anti-CD155 monoclonal antibodies that block the binding of CD155 to its receptors TIGIT, CD96, CD226, etc. through hybridoma technology. In addition, the constant regions of the murine anti-CD155 monoclonal antibodies were humanized, and the variable regions of the murine anti-CD155 monoclonal antibodies were retained, to obtain a series of anti-CD155 chimeric monoclonal antibodies. On this basis, the inventors further humanized the framework regions in the murine variable regions to obtain humanized CD155 antibodies, and further, the inventors mutated the CDR of the humanized CD155 antibody to obtain an affinity-matured humanized CD155 antibody. The inventors found that the chimeric antibody, the humanized antibody, and the affinity-matured humanized antibody obtained in the present disclosure can not only specifically target and bind to the CD155 receptor, block the binding of CD155 to its receptors TIGIT, CD96, CD226, etc., and effectively treat and/or prevent a CD155-mediated related disease, such as tumor, but also have the characteristic of not inducing CD155 cell apoptosis.

To this end, in a first aspect of the present disclosure, the present disclosure provides an antibody or antigen binding fragment. According to an embodiment of the present disclosure, the antibody or antigen binding fragment includes a CDR selected from at least one of the following sequences or an amino acid sequence having at least 80% identity thereto: heavy chain variable region CDR sequences: SEQ ID NO: 1 to 3, SEQ ID NO: 7 to 9, SEQ ID NO: 13 to 15, and SEQ ID NO: 19 to 21; and light chain variable region CDR sequences: SEQ ID NO: 4 to 6, SEQ ID NO: 10 to 12, SEQ ID NO: 16 to 18, and SEQ ID NO: 22 to 24. The antibody or antigen binding fragment according to the embodiments of the present disclosure is an affinity-matured humanized antibody. The antibody or antigen binding fragment has stronger specificity, longer half-life, and stronger efficacy, can bind to human or monkey CD155 protein, block the binding of CD155 to its receptors TIGIT, CD96, CD226, etc., effectively treat or prevent a CD155-mediated disease at a lower dosage without inducing apoptosis of CD155-positive cells, having low toxicity and side effects and higher safety.

In a second aspect of the present disclosure, the present disclosure provides a nucleic acid molecule. According to an embodiment of the present disclosure, the nucleic acid molecule encodes the antibody or antigen binding fragment according to the first aspect. The antibody or antigen binding fragment encoded by the nucleic acid molecule according to the embodiments of the present disclosure has stronger specificity, longer half-life, and stronger efficacy; can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

In a third aspect of the present disclosure, the present disclosure provides an expression vector. According to an embodiment of the present disclosure, the expression vector carries the nucleic acid molecule according to the second aspect. The expression vector may include optional control sequences operably linked to the nucleic acid molecule. The control sequence is one or more control sequences that can direct the expression of the nucleic acid molecule in the host. The expression vector according to the embodiment of the present disclosure can efficiently express the above antibody or antigen binding fragment in a suitable host cell. The above antibody or antigen binding fragment has stronger specificity, longer half-life, and stronger efficacy; can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

In a fourth aspect of the present disclosure, the present disclosure provides a method for preparing the antibody or antigen binding fragment according to the first aspect. According to an embodiment of the present disclosure, the method includes: introducing the expression vector according to the third aspect into cells; and culturing the cells under conditions suitable for expression and secretion of protein, to obtain the antibody or antigen binding fragment. The method according to the embodiment of the present disclosure can effectively obtain the above antibody or antigen binding fragment, which has stronger specificity, longer half-life, and stronger efficacy; can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

In a fifth aspect of the present disclosure, the present disclosure provides a recombinant cell. According to an embodiment of the present disclosure, the recombinant cell carries the nucleic acid according to the second aspect or the expression vector according to the third aspect; or can express the antibody or antigen binding fragment according to the first aspect. The recombinant cell is obtained by transfecting or transforming the expression vector. According to some specific embodiments of the present disclosure, the recombinant cells can efficiently and massively express the above antibody or antigen binding fragment under appropriate conditions. The above antibody or antigen binding fragment has stronger specificity, longer half-life, and stronger efficacy; can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

In a sixth aspect of the present disclosure, the present disclosure provides an immunoconjugate. According to an embodiment of the present disclosure, the immunoconjugate includes the antibody or antigen binding fragment according to the first aspect, and a therapeutic agent. As mentioned above, the antibody or antigen binding fragment according to the embodiments of the present disclosure can effectively bind to CD155 protein and block the binding of CD155 protein to its receptor. Therefore, the immunoconjugate containing the above antibody or antigen binding fragment can also bind to human or monkey CD155 protein. The immunoconjugate has good effects in preventing and/or treating a CD155-mediated disease without inducing apoptosis of CD155-positive cells, having low toxicity and side effects and higher safety.

In a seventh aspect of the present disclosure, the present disclosure provides a composition. According to an embodiment of the present disclosure, the composition includes the above antibody, nucleic acid molecule, expression vector, or recombinant cell. As mentioned above, the above antibody or antigen binding fragment according to the embodiments of the present disclosure can effectively bind to CD155 protein, block the binding of CD155 protein to its receptor without inducing apoptosis of CD155-positive cells. Therefore, the composition containing the above substances can also effectively block the binding of CD155 protein to its receptor, has a good effect in preventing and/or treating a CD155-mediated disease without inducing apoptosis of CD155-positive cells.

In an eighth aspect of the present disclosure, the present disclosure provides use of the above-mentioned antibody or antigen binding fragment, nucleic acid molecule, expression vector, recombinant cell, or composition in the manufacture of a medicament for preventing and/or treating a CD155-related disease. As mentioned above, the above antibody or antigen binding fragment according to the embodiments of the present disclosure can effectively bind to CD155 protein, block the binding of CD155 protein to its receptor without inducing apoptosis of CD155-positive cells. Therefore, the medicament containing the above substances can also effectively block the binding of CD155 protein to its receptor, has a good effect in preventing and/or treating a CD155-mediated disease without inducing apoptosis of CD155-positive cells.

In a ninth aspect of the present disclosure, the present disclosure provides a medicament. According to an embodiment of the present disclosure, the medicament includes the above antibody, nucleic acid molecule, expression vector, recombinant cell, or composition. As mentioned above, the antibody or antigen binding fragment according to the embodiments of the present disclosure can effectively bind to CD155 protein and block the binding of CD155 protein to its receptor without inducing apoptosis of CD155-positive cells. Therefore, the medicament containing the above-mentioned substances can also effectively block the binding of CD155 protein to its receptor, has a good effect in preventing and/or treating a CD155-mediated disease without inducing apoptosis of CD155-positive cells.

In a tenth aspect of the present disclosure, the present disclosure provides a use of the above-mentioned antibody or antigen binding fragment in the manufacture of a kit. According to an embodiment of the present disclosure, the kit is used for detecting CD155. As mentioned above, the antibody or antigen binding fragment according to the embodiments of the present disclosure can effectively bind to human or monkey CD155 protein and block the binding of CD155 protein to its receptor. Therefore, the above antibody or antigen binding fragment can be used to manufacture a kit for detecting CD155 protein, which can effectively perform qualitative or quantitative detection of CD155 protein.

In an eleventh aspect of the present disclosure, the present disclosure provides a kit for detecting CD155. According to an embodiment of the present disclosure, the kit contains the above antibody or antigen binding fragment. As mentioned above, the antibody or antigen binding fragment according to the embodiments of the present disclosure can effectively bind to human or monkey CD155 protein and block the binding of the CD155 protein to its receptor. Therefore, the kit containing the afore antibody or antigen binding fragment can effectively perform qualitative or quantitative detection of CD155 protein.

In a twelfth aspect of the present disclosure, the present disclosure provides a method for preventing and/or treating CD155-related diseases. According to an embodiment of the present disclosure, the method includes: administering, to a subject, at least one of the above antibody or antigen binding fragment, the above nucleic acid molecule, the above expression vector, the above recombinant cell, the above immunoconjugate, or the above composition.

Additional aspects and advantages of the present disclosure will be provided in part in the following description, or will become apparent in part from the following description, or can be learned from practicing of the present disclosure.

### BRIEF DESCRIPTION OF THE DRAWINGS

The foregoing and/or additional aspects and advantages of the present disclosure will become apparent and readily appreciated from the following description of the embodiments in conjunction with the accompanying drawings of which:
FIG. 1 is a flow cytometry result graph of murine CD155 antibody at different concentrations binding to CHO-K1-human CD155 cells expressing human CD155 protein and CHO-K1-Rhesus CD155 cells expressing monkey CD155 protein according to a specific embodiment of the present disclosure.
FIG. 2 is a graph showing the results of murine CD155 antibody at different concentrations blocking TIGIT-Fc, CD96-Fc, and CD226-Fc from binding to CHO-K1-human CD155 cells expressing human CD155 protein according to a specific embodiment of the present disclosure.
FIG. 3 is a graph showing the ELISA results of murine CD155 antibody at different concentrations blocking TIGIT, CD96, and CD266 from binding to recombinantly expressed human CD155 protein according to a specific embodiment of the present disclosure.
FIG. 4 is a flow cytometry result graph of human-mouse chimeric CD155 antibody at different concentrations binding to CHO-K1-huamn CD155 and CHO-K1-Rhesus CD155 cells according to a specific embodiment of the present disclosure.
FIG. 5 is a graph showing the ELISA results of affinity-matured humanized CD155 antibody at different concentrations binding to human and monkey CD155 proteins according to a specific embodiment of the present disclosure.
FIG. 6 is a graph showing the ELISA results of affinity-matured humanized CD155 antibody at different concentrations blocking TIGIT, CD96, and CD226 from binding to human or monkey CD155 proteins expressed by CHO-K1-huamn CD155 and CHO-K1-Rhesus CD155 cells according to a specific embodiment of the present disclosure.
FIG. 7 is a flow cytometry result graph of affinity-matured humanized CD155 antibody at different concentrations binding to CHO-K1-human CD155 and CHO-K1-Rhesus CD155 according to a specific embodiment of the present disclosure.
FIG. 8 is a graph showing the result of humanized CD155 antibody promoting apoptosis of U-937-CD155 cells according to a specific embodiment of the present disclosure; and
FIG. 9 is a graph showing the detection results of humanized CD155 antibody promoting anti-human tumor activity in immune-reconstructed mice according to a specific embodiment of the present disclosure.

### DETAILED DESCRIPTION

Embodiments of the present disclosure are described in detail below. The embodiments described below are illustrative, and are merely used for explaining the present disclosure, and cannot be understood as a limitation to the present disclosure.

It should be noted that terms "first" and "second" are only used for descriptive purposes, and cannot be understood as indicating or implying relative importance, or implicitly indicating the number of indicated technical features. Therefore, the features defined with "first" and "second" may explicitly or implicitly include one or more of such features. Further, in the description of the present disclosure, unless otherwise specified, "plurality" means two or more.

The specific embodiments of the present disclosure are described in detail below. It should be understood that the specific embodiments described herein are only used to illustrate and explain the present disclosure, and are not used to limit the present disclosure.

The endpoint values or any values of the ranges disclosed herein are not limited to the precise ranges or values. These ranges or values should be understood to include values approximating such ranges or values. For numerical ranges, one or more new numerical ranges can be obtained by combining the endpoint values of the respective ranges, an endpoint value and an individual point value within the respective ranges, and individual point values within the respective ranges with each other, and these numerical ranges should be regarded as specifically disclosed herein.

For an easier understanding of the present disclosure, certain technical and scientific terms are specifically defined below. Unless obviously and clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

In the present disclosure, when not stated to the contrary, the term antigen binding fragment, i.e. "antibody fragment" used generally refers to an antigen binding antibody fragment and may include a portion of an intact antibody, typically an antigen binding or variable region. Examples of antibody fragments include Fab, Fab', F(ab')2, Fv or scFv, diabody, linear antibody, single-chain antibody molecules, etc.

The term "complementarity determining region" or "CDR" or "CDR sequence" refers to the amino acid sequence responsible for antigen binding in an antibody, for example, generally includes amino acid residues near 23-34 (L1), 50-56 (L2) and 89-97 (L3) in the light chain variable region, and 31-35B (H1), 50-65 (H2) and 95-102 (H3) in the heavy chain variable region (Kabat et al. Sequences of Proteins of Immunological Interest, 5th Ed. Public Health Service, National Institutes of Health, Bethesda, MD.(1991)); and/or amino acid residues from the "hypervariable loops" (e.g. near 26-32 (LI), 50-52 (L2), and 91-96 (L3) in the light chain variable region, and 26-32 (H1), 53-55 (H2), and 96-101 (H3) in the heavy chain variable region (Chothia and Lesk J. Mol.Biol.196: 901-917(1987)).

The term "an amino acid sequence in conservative modification form" refers to an amino acid modification that does not significantly affect or alter the binding characteristics of the antibody including the amino acid sequence, including the substitutions, additions and deletions of the amino acids. Modifications can be introduced into the antibodies of the present disclosure by standard techniques such as site-directed mutagenesis and PCR-mediated mutagenesis. Conservative amino acid substitution refers to the substitution of amino acid residues by amino acid residues with similar side chains. Families of amino acid residues having similar side chains have been identified in the art. These families include amino acids with basic side chains, e.g. lysine, arginine, and histidine; amino acids with acidic side chains, e.g. aspartic acid, and glutamic acid; amino acids with uncharged polar side chains, e.g. glycine, asparagine, glutamine, serine, threonine, tyrosine, cysteine, and tryptophan; amino acids with nonpolar side chains, e.g. alanine, valine, leucine, isoleucine, proline, phenylalanine, and methionine; amino acids with β-branched side chains, e.g. threonine, valine, and isoleucine, and amino acids with aromatic side chains, e.g. tyrosine, phenylalanine, tryptophan, and histidine. Thus, one or more of the amino acid residues in the CDR region of the antibody of the present disclosure can be replaced with other amino acid residues from the same side chain family, and the altered antibody can be tested for retained function using the functional assays described herein. Preferably, the number of conservative modifications does not exceed one or two.

For polypeptides, the term "(substantial) homology" means that at least about 80%, usually at least about 90% to 95%, and more preferably at least about 98% to 99.5% of the amino acids of two polypeptides or designated sequences thereof are identical when optimally aligned and compared (in which nucleotides are inserted or deleted appropriately).

The % identity between two sequences varies with the number of identical positions shared by the sequences when the sequences are optimally aligned (i.e.% homology = number of identical positions/total number of positions × 100), where optimal alignment is determined taking into account the number of gaps that need to be introduced and the length of each gap to achieve optimal alignment of the two sequences. Sequence comparison and percent identity determination between two sequences can be accomplished using mathematical algorithms, as described in the non-limiting examples below.

One skilled in the art may substitute, add and/or delete one or more (e.g. 1, 2, 3, 4, 5, 6, 7, 8, 9, or 10 or more) amino acids to a sequence of the present disclosure to obtain a variant of the sequence of the antibody or functional fragment thereof without substantially affecting the activity of the antibody (retaining at least 95% activity). They are considered to be included within the scope of the present disclosure. For example, amino acids having similar properties in the variable region may be substituted. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred in the present disclosure are shown from the N-terminus to the C-terminus.

As previously described, the antibody of the present disclosure may be full length (e.g. IgG1 or IgG4 antibodies) or may include only an antigen binding portion (e.g. Fab, F(ab')2 or scFv fragment), or may be modified to affect function. The present disclosure includes anti-CD155 antibodies having modified glycosylation patterns. In some applications, it may be useful to make modifications to remove undesired glycosylation sites, or to eliminate the presence of fucose moieties on the oligosaccharide chains, e.g. to enhance antibody-dependent ADCC function. In other applications, galactosylation modifications can be made to alter complement dependent cytotoxicity (CDC).

The term "functional fragment" as used herein refers in particular to fragment of an antibody such as Fv, scFv (sc means single chain), Fab, F(ab')2, Fab', scFv-Fc fragment or diabody, or any fragment which should be capable of increasing the half-life by chemical modification, e.g. addition of polyalkylene glycols, such as polyethylene glycol ("PEGylation, PEG") (referred to as pegylated fragments of Fv-PEG, scFv-PEG, Fab-PEG, F(ab')2-PEG or Fab'-PEG) ("PEG" means polyethylene glycol) or by incorporation into liposomes, and the fragment has CD155 binding activity. Preferably, the functional fragment will consist of or contain a partial sequence of the heavy chain variable region or light chain variable region of its source antibody, which is sufficient to retain the same binding specificity and sufficient affinity as its source antibody. For CD155, it is preferred to be at least 1/100 of its source antibody affinity, and in a more preferred manner, at least 1/10. Such function fragments will include a minimum of 5 amino acids, preferably 10, 15, 25, 50, and 100 consecutive amino acids of the antibody sequence from which they are derived.

In one aspect of the present disclosure, the present disclosure provides an antibody or antigen binding fragment, which includes a CDR selected from at least one of the following sequences or an amino acid sequence having at least 80% identity thereto: heavy chain variable region CDR sequences: SEQ ID NO: 1 to 3, SEQ ID NO: 7 to 9, SEQ ID NO: 13 to 15, and SEQ ID NO: 19 to 21; and light chain variable region CDR sequences: SEQ ID NO: 4 to 6, SEQ ID NO: 10 to 12, SEQ ID NO: 16 to 18, and SEQ ID NO: 22 to 24. The antibody or antigen binding fragment according to the embodiment of the present disclosure is an affinity-matured humanized antibody, which has stronger specificity, longer half-life, and stronger efficacy, can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

In a preferred embodiment of the present disclosure, in order to further improve the biological acceptability, specificity, and efficacy of the antibody, the antibody is humanized. Furthermore, the inventors performed affinity maturation treatment on the humanized antibody, such as mutating the CDR, to obtain an affinity-matured antibody. The term "chimeric antibody" refers to a recombinant antibody obtained by replacing the amino acid sequence of the constant region of a monoclonal antibody from one species (e.g. mouse) with that of the constant region of an antibody from another species (e.g. human) using recombinant DNA technology. The term "humanized antibody" refers to a recombinant antibody obtained by completely replacing the amino acid sequences of the constant region and the non-CDR (Fv framework region (FR)) in the variable region of a monoclonal antibody from one species (e.g. mouse) with the amino acid sequences of the constant region and the non-CDR in the variable region of an antibody from another species (e.g. human) using recombinant DNA technology. That is, when the constant region of an antibody is humanized, it is referred to as a chimeric antibody, and the amino acid sequences of the constant region and the non-CDR variable region are all humanized, it is referred to as a humanized antibody. Methods of humanization may be carried out with reference to conventional antibody engineering techniques and will not be described in detail herein. "Affinity maturation of antibody" refers to performing random mutation on the amino acids in the CDR region of the antibody, and obtaining the antibodies with higher affinity for the corresponding antigen by screening the mutant library.

According to some specific embodiments of the present disclosure, the above-mentioned antibody or antigen binding fragment further comprises at least one of the following additional technical features.

According to some specific embodiments of the present disclosure, the antibody or antigen binding fragment includes: a heavy chain variable region CDR1 selected from at least one of amino acid sequences as set forth in SEQ ID NO: 1, SEQ ID NO: 7, SEQ ID NO: 13, and SEQ ID NO: 19, and amino acid sequences in conservative modification form thereof; a heavy chain variable region CDR2 selected from at least one of an amino acid sequences as set forth in SEQ ID NO: 2, SEQ ID NO: 8, SEQ ID NO: 14, and SEQ ID NO: 20, and amino acid sequences in conservative modification form thereof; and a heavy chain variable region CDR3 selected from at least one of amino acid sequences as set forth in SEQ ID NO: 3, SEQ ID NO: 9, SEQ ID NO: 15, and SEQ ID NO: 21, and amino acid sequences in conservative modification form thereof.

According to some specific embodiments of the present disclosure, the antibody or antigen binding fragment may further include a light chain variable region CDR1 selected from at least one of amino acid sequences as set forth in SEQ ID NO: 4, SEQ ID NO: 10, SEQ ID NO: 16, and SEQ ID NO: 22, and amino acid sequences in conservative modification form thereof; a light chain variable region CDR2 selected from at least one of amino acid sequences as set forth in SEQ ID NO: 5, SEQ ID NO: 11, SEQ ID NO: 17, and SEQ ID NO: 23, and amino acid sequences in conservative modification form thereof; and a light chain variable region CDR3 selected from at least one of amino acid sequences as set forth in SEQ ID NO: 6, SEQ ID NO: 12, SEQ ID NO: 18, and SEQ ID NO: 24, and amino acid sequences in conservative modification form thereof.

According to some specific embodiments of the present disclosure, the antibody or antigen binding fragment includes: 1) a heavy chain variable region CDR1 as set forth in SEQ ID NO: 1, a heavy chain variable region CDR2 as set forth in SEQ ID NO: 2, a heavy chain variable region CDR3 as set forth in SEQ ID NO: 3, a light chain variable region CDR1 as set forth in SEQ ID NO: 4, a light chain variable region CDR2 as set forth in SEQ ID NO: 5, and a light chain variable region CDR3 as set forth in SEQ ID NO: 6; or 2) a heavy chain variable region CDR1 as set forth in SEQ ID NO: 7, a heavy chain variable region CDR2 as set forth in SEQ ID NO: 8, a heavy chain variable region CDR3 as set forth in SEQ ID NO: 9, a light chain variable region CDR1 as set forth in SEQ ID NO: 10, a light chain variable region CDR2 as set forth in SEQ ID NO: 11, and a light chain variable region CDR3 as set forth in SEQ ID NO: 12; or 3) a heavy chain variable region CDR1 as set forth in SEQ ID NO: 13, a heavy chain variable region CDR2 as set forth in SEQ ID NO: 14, a heavy chain variable region CDR3 as set forth in SEQ ID NO: 15, a light chain variable region CDR1 as set forth in SEQ ID NO: 16, a light chain variable region CDR2 as set forth in SEQ ID NO: 17, and a light chain variable region CDR3 as set forth in SEQ ID NO: 18; or 4) a heavy chain variable region CDR1 as set forth in SEQ ID NO: 19, a heavy chain variable region CDR2 as set forth in SEQ ID NO: 20, a heavy chain variable region CDR3 as set forth in SEQ ID NO: 21, a light chain variable region CDR1 as set forth in SEQ ID NO: 22, a light chain variable region CDR2 as set forth in SEQ ID NO: 23, and a light chain variable region CDR3 as set forth in SEQ ID NO: 24.

According to some specific embodiments of the present disclosure, the antibody or antigen binding fragment includes: i) a heavy chain variable region having an amino acid sequence with at least 80% homology to at least one of amino acid sequences as set forth in SEQ ID NO. 25, SEQ ID NO. 27, SEQ ID NO. 29, and SEQ ID NO. 31 and amino acid sequences in conservative modification form thereof; and/or ii) a light chain variable region having an amino acid sequence with at least 80% homology to at least one of amino acid sequences as set forth in SEQ ID NO. 26, SEQ ID NO. 28, SEQ ID NO. 30, and SEQ ID NO. 32 and amino acid sequences in conservative modification form thereof.

According to some specific embodiments of the present disclosure, the heavy chain variable region has an amino acid sequence with at least 90%, at least 95%, at least 96%, at least 97%, at least 98%, at least 99%, or 100% homology to the heavy chain variable region selected from i); and the light chain variable region has an amino acid sequence with at least 90%, at least 95%, at least 96%, at least 97%, at least 98%, at least 99%, or 100% homology to the light chain variable region selected from ii).

According to some specific embodiments of the present disclosure, the antibody or antigen binding fragment includes: 1) a heavy chain variable region as set forth in SEQ ID NO: 25 and a light chain variable region as set forth in SEQ ID NO: 26; or 2) a heavy chain variable region as set forth in SEQ ID NO: 27 and a light chain variable region as set forth in SEQ ID NO: 28; or 3) a heavy chain variable region as set forth in SEQ ID NO: 29 and a light chain variable region as set forth in SEQ ID NO: 30; or 4) a heavy chain variable region as set forth in SEQ ID NO: 31 and a light chain variable region as set forth in SEQ ID NO: 32.

According to some specific embodiments of the present disclosure, the antibody includes at least one of a heavy chain constant region and a light chain constant region, and at least a portion of the at least one of the heavy chain constant region and the light chain constant region is derived from at least one of a humanized antibody, a primate-derived antibody, or a mutant thereof.

According to some specific embodiments of the present disclosure, the light chain constant region and the heavy chain constant region of the antibody are both derived from a humanized IgG antibody or a mutant thereof.

According to some specific embodiments of the present disclosure, the light chain constant region and the heavy chain constant region of the antibody are both derived from a humanized IgG1 antibody, a humanized IgG4 antibody, or a mutant thereof.

According to some specific embodiments of the present disclosure, the antibody includes a heavy chain constant region having an amino acid sequence as set forth in SEQ ID NO: 41, SEQ ID NO: 42, or SEQ ID NO: 43; and/or a light chain constant region having an amino acid sequence as set forth in SEQ ID NO: 44.

According to some specific embodiments of the present disclosure, the antibody or antigen binding fragment includes a heavy chain having an amino acid sequence as set forth in SEQ ID NO:33, SEQ ID NO: 35, SEQ ID NO: 37, or SEQ ID NO: 39; and a light chain having an amino acid sequence as set forth in SEQ ID NO:34, SEQ ID NO: 36, SEQ ID NO: 38, or SEQ ID NO: 40.

According to some specific embodiments of the present disclosure, the antibody is a monoclonal antibody, a murine antibody, a chimeric antibody, a humanized antibody, an engineered human antibody, a humanized antibody, Fv, a single-chain antibody (scFv), Fab, Fab', Fab'-SH, or F(ab')₂.

According to some embodiments of the present disclosure, the antibody or antigen binding fragment is capable of binding to an amino acid sequence as set forth in SEQ ID NO: 73 and/or SEQ ID NO: 74.

Nucleic acids encoding the heavy chain and/or light chain of the antibodies of the present disclosure are within the scope of the present disclosure. Based on the amino acid sequences of the heavy chain and/or light chain, those skilled in the art can easily obtain the corresponding nucleic acid sequences.

Therefore, in another aspect of the present disclosure, the present disclosure provides a nucleic acid molecule encoding the antibody or antigen binding fragment according to the first aspect. According to some specific embodiments of the present disclosure, the antibody or antigen binding fragment encoded by the nucleic acid molecule has stronger specificity, longer half-life, and stronger efficacy, can bind to human or monkey CD155 protein, can block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

According to some specific embodiments of the present disclosure, the above nucleic acid molecule may further include at least one of the following additional technical features.

According to some specific embodiments of the present disclosure, the nucleic acid molecule is DNA.

It should be noted that, for the nucleic acid mentioned in the specification and the claims of the present disclosure, those skilled in the art should understand that either one or both strands of the complementary double strands are actually included. For convenience, although in most cases only one strand is provided in the specification and the claims, the complementary strand is also actually disclosed. In addition, the nucleic acid sequence in the present disclosure includes both a DNA form and an RNA form, and thus the disclosure of one form implies that the other is also disclosed.

In yet another aspect of the present disclosure, the present disclosure provides an expression vector. The expression vector carries the above nucleic acid molecule. The expression vector may include optional control sequences operably linked to the nucleic acid molecule. The control sequence refers to one or more control sequences that can direct the expression of the nucleic acid molecule in the host. The expression vector according to the embodiments of the present disclosure can efficiently express the antibody or antigen binding fragment in a suitable host cell. The antibody or antigen binding fragment has stronger specificity, longer half-life, and stronger efficacy, can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety. When the above nucleic acid molecule is linked to the vector, the nucleic acid molecule can be directly or indirectly linked to the control elements on the vector, as long as these control elements can regulate the translation, expression, or the like of the nucleic acid molecule. Certainly, these control elements may be directly derived from the vector itself or be exogenous, i.e., not derived from the vector itself. Certainly, the nucleic acid molecule needs to be operably linked to the control element. The "operably linked" herein means an exogenous gene is linked to a vector such that the control elements in the vector, such as a transcriptional control sequence and a translational control sequence, can exert their intended function of regulating the transcription and translation of the exogenous gene. Certainly, the nucleic acid molecule encoding the antibody or antigen-binding fragment can be independently inserted into different vectors, and is commonly inserted into the same vector. For example, common vectors may be plasmids, bacteriophages, etc., such as Plasmid-X.

In one aspect of the present disclosure, the present disclosure provides a method for preparing the above antibody or antigen binding fragment. The method includes: introducing the above expression vector into cells; and culturing the cells under conditions suitable for expression and secretion of protein, to obtain the antibody or antigen binding fragment. The method according to some specific embodiments of the present disclosure can effectively obtain the antibody or antigen binding fragment, which has stronger specificity, longer half-life, and stronger efficacy, can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells, having low toxicity and side effects and higher safety.

According to some specific embodiments of the present disclosure, the method for preparing the above antibody or antigen binding fragment may also include at least one of the following additional technical features.

According to some specific embodiments of the present disclosure, the cells are not particularly limited, and either prokaryotic cells or eukaryotic cells can be used. When the cells are eukaryotic cells, such as mammalian cells, the expression efficiency of the recombinant antibody is higher.

According to some embodiments of the present disclosure, the cell is a eukaryotic cell.

According to some specific embodiments of the present disclosure, the eukaryotic cell is a mammalian cell. According to some specific embodiments of the present disclosure, when the cells are eukaryotic cells, such as mammalian cells, the expression efficiency of the recombinant antibody is higher.

According to some embodiments of the present disclosure, the eukaryotic cells do not include animal germ cells, fertilized eggs, or embryonic stem cells.

In another aspect of the present disclosure, the present disclosure provides a recombinant cell. The recombinant cell carries the above nucleic acid or expression vector; or can express the above antibody or antigen binding fragment. The recombinant cell is obtained by transfecting or transforming the expression vector. According to some specific embodiments of the present disclosure, the recombinant cells can efficiently and massively express the above antibody or antigen binding fragment under suitable conditions. The antibody or antigen binding fragment has stronger specificity, longer half-life, and stronger efficacy, can bind to human or monkey CD155 protein, block the binding of CD155 to its receptors TIGIT, CD96, CD226, etc., and effectively treat or prevent a CD155-mediated disease at a lower dosage without inducing apoptosis of CD155-positive cells

It should be noted that, the recombinant cell of the present disclosure is not particularly limited and may be a prokaryotic cell, a eukaryotic cell, or a bacteriophage. The prokaryotic cell may be *Escherichia coli, Bacillus subtilis, Streptomyces, Proteus mirabilis,* or like. The eukaryotic cell includes fungi such as *Pichia pastoris, Saccharomyces cerevisiae, Schizosaccharomyces pombe,* and *Trichoderma,* insect cells such as *Spodoptera frugiperda,* plant cells such as tobacco, and mammalian cells such as a BHK cell, a CHO cell, a COS cell, a myeloma cell, etc. In some embodiments, the recombinant cell of the present disclosure is preferably the mammalian cell, including a BHK cell, a CHO cell, an NSO cell, or a COS cell, and does not include an animal germ cell, a fertilized egg, or an embryonic stem cell.

It should be noted that "suitable conditions" referred to in the specification of the present disclosure refer to conditions suitable for the expression of the antibody or antigen-binding fragment of the present disclosure. It is conceivable for those skilled in the art that the conditions suitable for the expression of the antibody or antigen-binding fragment include, but are not limited to, an appropriate transformation or transfection method, an appropriate transformation or transfection condition, a healthy host cell state, a suitable host cell density, a suitable cell culture environment, and a suitable cell culture duration. The "suitable conditions" are not particularly limited. Those skilled in the art can optimize the most suitable conditions for expression of the antibody or antigen-binding fragment based on specific laboratory environments.

In yet another aspect of the present disclosure, the present disclosure provides an immunoconjugate, which includes the above antibody or antigen binding fragment and a therapeutic agent. As mentioned above, the antibody or antigen binding fragment according to the embodiments of the present disclosure can effectively bind to CD155 protein and block the binding of CD155 protein to its receptor. Therefore, the immunoconjugate containing the antibody or antigen binding fragment can also bind to human or monkey CD155 protein. The immunoconjugate has good effects in preventing and/or treating a CD155-mediated disease, without inducing apoptosis of CD155-positive cells, having low toxicity and side effects and higher safety.

In one aspect of the present disclosure, the present disclosure provides a composition, which includes the above antibody, nucleic acid molecule, expression vector, or recombinant cell. As mentioned above, the antibody or antigen binding fragment according to some specific embodiments of the present disclosure can effectively bind to CD155 protein and block the binding of CD155 protein to its receptor without inducing apoptosis of CD155-positive cells. Therefore, the composition containing the above substances can also effectively block the binding of CD155 protein to its receptor, has a good effect in preventing and/or treating a CD155-mediated disease, without inducing apoptosis of CD155-positive cells. The type of the composition is not particularly limited and may be a food composition or a pharmaceutical composition.

The compositions of the present disclosure may also be administered in combination with each other or with one or more other therapeutic compounds, for example, in combination with a chemotherapeutic agent. Therefore, the composition may further contain a chemotherapeutic agent. The antibody or antigen-binding fragment, or the immunoconjugate of the present disclosure may also be combined with a second therapeutic agent. Examples of the second therapeutic agent include, but are not limited to, other agents that inhibit the activity of CD155 (including other antibodies or antigen-binding fragments, peptide inhibitors, small molecule antagonists, etc.) and/or agents that interfere with upstream or downstream signal transduction of CD155.

It should be noted that, the composition includes combinations separated in time and/or space, as long as they are capable of acting together to achieve the objectives of the present disclosure. For example, components contained in the composition may be administered to a subject as a whole or separately. When the components contained in the composition are administered separately to the subject, the respective components may be administered to the subject simultaneously or sequentially.

In another aspect of the present disclosure, the present disclosure provides a use of the above antibody or antigen binding fragment, nucleic acid molecule, expression vector, recombinant cell, or composition in the manufacture of a medicament for preventing and/or treating a CD155-related disease. As mentioned above, the antibody or antigen binding fragment according to some specific embodiments of the present disclosure can effectively bind to CD155 protein and block the binding of CD155 protein to its receptor without inducing apoptosis of CD155-positive cells. Therefore, the medicament containing the above substances can also effectively block the binding of CD155 protein to its receptor, has a good effect in preventing and/or treating a CD155-mediated disease without inducing apoptosis of CD155-positive cells.

According to some specific embodiments of the present disclosure, the above-mentioned use in the manufacture of the medicament may also include at least one of the following additional technical features.

According to some specific embodiments of the present disclosure, the CD155-related disease is cancer, autoimmune disease, transplant rejection, or infectious disease.

According to some specific embodiments of the present disclosure, the cancer includes at least one of melanoma, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, and head-and-neck cancer.

According to some specific embodiments of the present disclosure, the infectious disease includes but is not limited to HIV virus infection and/or hepatitis B virus infection.

In another aspect of the present disclosure, the present disclosure provides a medicament, which includes the above antibody, nucleic acid molecule, expression vector, recombinant cell, or composition. As mentioned above, the antibody or antigen binding fragment according to some specific embodiments of the present disclosure can effectively bind to CD155 protein and block the binding of CD155 protein to its receptor without inducing apoptosis of CD155-positive cells. Therefore, the medicament containing an effective amount of antibody active ingredients or a series of substances thereof can also effectively block the binding of CD155 protein to its receptor. The antibody or antigen binding fragment has a good effect in preventing and/or treating a CD155-mediated disease without inducing apoptosis of CD155-positive cells.

According to some specific embodiments of the present disclosure, the above-mentioned medicament may further include at least one of the following additional technical features.

According to some specific embodiments of the present disclosure, the medicament is used to treat or prevent a CD155-related disease.

According to some specific embodiments of the present disclosure, the CD155-related disease is cancer, autoimmune disease, transplant rejection, or infectious disease.

According to some specific embodiments of the present disclosure, the cancer includes at least one of melanoma, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, and head-and-neck cancer.

According to some specific embodiments of the present disclosure, the infectious disease includes but is not limited to HIV virus infection and/or hepatitis B virus infection.

According to some specific embodiments of the present disclosure, a pharmaceutically acceptable carrier may also be included.

As used herein, the term "effective amount" or "effective dose" refers to an amount that produces a function or activity in humans and/or animals and is acceptable to humans and/or animals.

As used herein, a "pharmaceutically acceptable" component is a substance that is suitable for use in humans and/or mammals without undue adverse side effects (e.g., toxicity, irritation, and allergic reactions), i.e., having a reasonable benefit/risk ratio. The term "pharmaceutically acceptable carrier" refers to a carrier for administration of the therapeutic agent and includes various excipients and diluents.

The medicament of the present disclosure contains a safe and effective amount of the active component of the present disclosure and the pharmaceutically acceptable carrier. Such carriers include, but not limited to, saline, buffer, glucose, water, glycerol, ethanol, and a combination thereof. Generally, a pharmaceutical preparation should be matched with the administration modes, which can be oral administration, nasal administration, intradermal administration, subcutaneous administration, intramuscular administration, intravenous administration, or intraperitoneal administration. A dosage form of the medicament of the present disclosure can be an injection, an oral preparation (tablets, capsules, and oral liquids), a transdermal agent, and a sustained-release agent. For example, the medicament is prepared by conventional methods using normal saline or an aqueous solution containing glucose and other adjuvants. The medicament is preferably manufactured under sterile conditions.

The effective amount of the active ingredient of the present disclosure can vary with the administration mode, the severity of the disease to be treated, etc. The selection of the preferred effective amount can be determined by those of ordinary skill in the art based on various factors (e.g., through clinical trials). Such factors include, but are not limited to: pharmacokinetic parameters of the active component such as bioavailability, metabolism, half-life period, etc.; and the severity of the disease to be treated in a patient, a weight of the patient, an immune status of the patient, a route of administration, etc. For example, depending on urgency of the treatment situation, the doses can be administered several times a day separately, or the dose can be proportionally reduced.

Of course, the anti-CD155 antibody herein may also be formulated into a kit or part of other diagnostic reagent as needed.

In one aspect of the present disclosure, the present disclosure provides a use of the above antibody or antigen binding fragment in the manufacture of a kit for detecting CD155. As mentioned above, the antibody or antigen binding fragment according to some specific embodiments of the present disclosure can effectively bind to human or monkey CD155 protein and block the binding of CD155 protein to its receptor. Therefore, the antibody or antigen binding fragment can be used to manufacture a kit for detecting CD155 protein, which can effectively perform qualitative or quantitative detection of CD155 protein.

In another aspect of the present disclosure, the present disclosure provides a kit for detecting CD155, which contains the above antibody or antigen binding fragment. As mentioned above, the antibody or antigen binding fragment according to some specific embodiments of the present disclosure can effectively bind to human or monkey CD155 protein and block the binding of the CD155 protein to its receptor. Therefore, the kit containing the antibody or antigen binding fragment can effectively perform qualitative or quantitative detection of CD155 protein. The kit according to the present disclosure can be used, for example, for immunoblotting, immunoprecipitation, and other detections that utilize the specific binding properties of CD155 antigen and antibody. These kits may contain any one or more of: an antagonist, an anti-CD155 antibody or a pharmaceutical reference material; a protein purification column; an immunoglobulin affinity purification buffer; a cell assay diluent; an instruction or literature, etc. The anti-CD155 antibody can be used for different types of diagnostic tests, for example, an in vitro or in vivo detection of various diseases or a presence of a medicament, a toxin, or other proteins. For example, the anti-CD155 antibody can be used to test related diseases through detecting the serum or blood of a subject. Such related diseases may include CD155-related diseases, such as cancer, autoimmune disease, transplant rejection, or infectious disease, wherein the cancer comprises at least one of melanoma, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, and head-and-neck cancer. Of course, the antibody provided herein can also be used for radioimmunoassay and radioimmunotherapy of the above diseases.

According to some specific embodiments of the present disclosure, the kit may further include a reagent conventionally used for detecting CD155, such as coating solution and the like.

The present disclosure also relates to a method for preventing and/or treating a CD155-related disease. The method includes: administering an effective amount of at least one of the antibody or antigen binding fragment, the nucleic acid molecule, the expression vector, the recombinant cell, the immunoconjugate, or the composition of the present disclosure to a subject.

The "patient" or "subject" involved in the present disclosure generally refers to a mammal, such as a primate and/or a rodent, in particular a human or a mouse.

The detailed descriptions of the sequences involved in the present disclosure are shown in Table 1.

**[Table 1]: Description table of amino acid sequences**

| SEQ ID NO: | Sequence | Description | SEQ ID NO: | |
|---|---|---|---|---|
| 1 | GYTFTSSY | Heavy chain variable region CDR1 of affinity-matured humanized antibody V20 (tgi1005 V20-hIgG1/4 /1LALA HCDR1) | 82 | GGATACACCTTTACCTCATC ATAC |
| 2 | IYAGTGDT | Heavy chain variable region CDR2 of affinity-matured humanized antibody V20 (tgi1005 V20-hIgG1/4/1LALA HCDR2) | 83 | ATCTACGCTGGAACCGGTGA CACC |
| 3 | ARHGYTGRGWYF AV | Heavy chain variable region CDR3 of affinity-matured humanized antibody V20 (tgi1005 V20-hIgG1/4/1LALA HCDR3) | 84 | GCTCGTCACGGCTATACCGG AAGAGGCTGGTATTTGCTG TT |
| 4 | TGAVTTSN | Light chain variable region CDR1 of affinity-matured humanized antibody V20 (tgi1005 V20-hIgG1I4/1LALA LCDR1) | 85 | ACAGGCGCCGTAACAACTTC CAAT |
| 5 | HTN | Light chain variable region CDR2 of affinity-matured humanized antibody V20 (tgi1005 V20-hIgG1I4/1LALA LCDR2) | 86 | CACACTAAC |
| 6 | ALWYSNKFV | Light chain variable region CDR3 of affinity-matured humanized antibody V20 (tgi1005 V20-hIgG1I4/1LALA LCDR3) | 87 | GCCCTCTGGTACTCAAATAA GTTCGTC |
| 7 | GYTFTSSY | Heavy chain variable region CDR1 of affinity-matured humanized antibody V21 (tgi1005 V21-hIgG1/4 HCDR1) | 88 | GGATACACCTTTACCTCATC ATAC |
| 8 | IYAGTGDT | Heavy chain variable region CDR2 of affinity-matured humanized antibody V21 (tgi1005 V21-hIgG1/4 HCDR2) | 89 | ATCTACGCTGGAACCGGTGA CACC |
| 9 | ARHGYTGRGWYF AV | Heavy chain variable region CDR3 of affinity-matured humanized antibody V21 (tgi1005 V21-hIgG1/4 HCDR3) | 90 | GCTCGTCACGGCTATACCGG AAGAGGCTGGTATTTGCTG TT |
| 10 | TGAVTTSN | Light chain variable region CDR1 of affinity-matured humanized antibody V21 (tgi1005 V21-hIgG1/4 LCDR1) | 91 | ACAGGCGCTGTGACTACCTC AAAC |
| 11 | GTN | Light chain variable region CDR2 of affinity-matured humanized antibody V21 (tgi1005 V21-hIgG1/4 LCDR2) | 92 | GGGACTAAC |
| 12 | ILWYSNHFV | Light chain variable region CDR3 of affinity-matured humanized antibody V21 (tgi1005 V21-hIgG1/4 LCDR3) | 93 | ATCCTCTGGTATAGCAACCAT TTCGTT |
| 13 | GYTFTSSY | Heavy chain variable region CDR1 of affinity-matured humanized antibody V28 (tgil005 V28-hIgG1/4/1LALA HCDR1) | 94 | GGATACACCTTTACAAGCAG TTAC |
| 14 | IYAGTGDT | Heavy chain variable region CDR2 of affinity-matured humanized antibody V28 (tgil005 V28-hIgG1/4/1LALA HCDR2) | 95 | ATCTATGCCGGAACAGGGGA CACA |
| 15 | ARHGLTGRHWYF AV | Heavy chain variable region CDR3 of affinity-matured humanized antibody V28 (tgil005 V28-hIgG1/4/1LALA HCDR3) | 96 | GCCAGACACGGACTGACAG GTCGTCACTGGTATTTCGCC GTG |
| 16 | TGAVTTSN | Light chain variable region CDR1 of affinity-matured humanized antibody V28 (tgil005 V28-hIgG1/4/1LALA LCDR1) | 97 | ACAGGTGCCGTGACCACAA GCAAT |
| 17 | GTN | Light chain variable region CDR2 of affinity-matured humanized antibody V28 (tgil005 V28-hIgG1/4/1LALA LCDR2) | 98 | GGTACAAAT |
| 18 | ILWYSNKFV | Light chain variable region CDR3 of affinity-matured humanized antibody V28 (tgil005 V28-hIgG1/4/1LALA LCDR3) | 99 | ATCCTGTGGTATTCCAACAA GTTCGTC |
| 19 | GYTFTSSY | Heavy chain variable region CDR1 of affinity-matured humanized antibody V40 (tgil005 V40-hIgG1/4 HCDR1) | 100 | GGATACACCTTTACCAGTAG CTAC |
| 20 | IYAGTGDT | Heavy chain variable region CDR2 of affinity-matured humanized antibody V40 (tgil005 V40-hIgG1/4 HCDR2) | 101 | ATCTATGCTGGCACTGGTGA CACC |
| 21 | ARHGMTGRGWY FAV | Heavy chain variable region CDR3 of affinity-matured humanized antibody V40 (tgil005 V40-hIgG1/4 HCDR3) | 102 | GCACGCCACGGTATGACTGG CAGGGGGTGGTATTTTGCTG TG |
| 22 | TGAVTTSN | Light chain variable region CDR1 of affinity-matured humanized antibody V40 (tgil005 V40-hIgG1/4 LCDR1) | 103 | ACAGGTGCCGTGACCACCTC CAAT |
| 23 | HTN | Light chain variable region CDR2 of affinity-matured humanized antibody V40 (tgil005 V40-hIgG1/4 LCDR2) | 104 | CACACTAAC |
| 24 | ILWYSNKFV | Light chain variable region CDR3 of affinity-matured humanized antibody V40 (tgil005 V40-hIgG1/4 LCDR3) | 105 | ATCCTCTGGTATAGCAACAA GTTTGTG |
| 25 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGYTGRGWYFAV WGQGTTVTVSS | Heavy chain variable region of affinity-matured humanized antibody V20 (tgi1005 V20-hIgG1/4 VH) | 106 | CAGGTGCAACTGGTGCAGA GTGGGGCAGAAGTAAAAA ACCAGGGGCTTCTGTTAAGG TGAGCTGTAAGGCAAGTGG ATACACCTTTACCTCATCATA CATCAGCTGGGTACGGCAAG CTCCTGGACAAGGTCTGGA ATGGATTGCTTGGATCTACG CTGGAACCGGTGACACCAAT TACAACCAGAAGTTTACTGG |
| | | | | AAGGGTGACCATGACTACCG ACACATCAACCTCCACTGCT TATATGGAGCTGAGAAGCCT GCGAAGCGACGACACTGCT GTTTACTATTGCGCTCGTCAC GGCTATACCGGAAGAGGCTG GTATTTTGCTGTTTGGGGTC AGGGTACAACTGTGACAGT GTCAAGC |
| 26 | QAVVTQEPSLTVS PGGTVTLTCRSST GAVTTSNLANWV QQKPGQAFRGLIG HTNNRAPGVPAR FSGSLLGDKAALT LSGAQPEDEAEY YCALWYSNKFVF GGGTKLTVL | Light chain variable region of affinity-matured humanized antibody V20 (tgil005 V20-hIgG1/4 VL) | 107 | CAGGCCGTTGTTACCCAAGA ACCTTCCCTCACCGTTTCCC CAGGCGGCACAGTGACCTT GACTTGTCGCTCTAGCACAG GCGCCGTAACAACTTCCAAT CTGGCCAATTGGGTGCAGCA GAAGCCTGGACAGGCATTTA GGGGCCTGATTGGACACACT AACAACAGAGCCCCCGGAG TACCTGCTAGGTTCAGTGGC AGTCTGTTGGGAGACAAAG CAGCTCTTACACTGTCAGGC GCCCAGCCAGAGGATGAGG CAGAGTACTACTGTGCCCTC TGGTACTCAAATAAGTTCGT CTTCGGAGGGGGACTAAG CTGACTGTTCTT |
| 27 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGYTGRGWYFAV WGQGTTVTVSS | Heavy chain variable region of affinity-matured humanized antibody V21 (tgi1005 V21-hIgG1/4 VH) | 108 | CAGGTGCAACTGGTGCAGA GTGGGGCAGAAGTAAAAA ACCAGGGGCTTCTGTTAAGG TGAGCTGTAAGGCAAGTGG ATACACCTTTACCTCATCATA CATCAGCTGGGTACGGCAAG CTCCTGGACAAGGTCTGGA ATGGATTGCTTGGATCTACG CTGGAACCGGTGACACCAAT TACAACCAGAAGTTTACTGG AAGGGTGACCATGACTACCG ACACATCAACCTCCACTGCT TATATGGAGCTGAGAAGCCT GCGAAGCGACGACACTGCT GTTTACTATTGCGCTCGTCAC GGCTATACCGGAAGAGGCTG GTATTTTGCTGTTTGGGGTC AGGGTACAACTGTGACAGT GTCAAGC |
| 28 | QAVVTQEPSLTVS PGGTVTLTCRSST GAVTTSNYANWV QQKPGQAFRGLIG GTNNRAPGVPAR FSGSLLGDKAALT LSGAQPEDEAEY YCILWYSNHFVFG GGTKLTVL | Light chain variable region of affinity-matured humanized antibody V21 (tgi1005 V21-hIgG1/4 VL) | 109 | CAAGCCGTTGTGACCCAAG AGCCCAGCCTGACTGTAAGC CCCGGCGGCACAGTCACAC TGACTTGCCGCTCAAGTACA GGCGCTGTGACTACCTCAAA CTATGCCAACTGGGTGCAGC AGAAACCAGGCCAAGCTTT TAGGGGTCTGATAGGCGGGA CTAACAACAGAGCACCTGG GGTGCCTGCCAGGTTTAGTG |
| | | | | GTTCCCTGCTTGGCGATAAG GCTGCTTTGACTTTGTCTGG TGCTCAACCCGAGGACGAG GCTGAGTACTACTGTATCCT CTGGTATAGCAACCATTTCG TTTTCGGCGGAGGCACCAA GTTGACTGTGCTG |
| 29 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGLTGRHWYFAV WGQGTTVTVSS | Heavy chain variable region of affinity-matured humanized antibody V28 (tgi1005 V28-hIgG1/4 VH) | 110 | CAGGTCCAGCTCGTTCAAAG TGGTGCAGAAGTCAAGAAG CCCGGCGCTAGTGTCAAAGT TTCCTGCAAGGCCAGCGGAT ACACCTTTACAAGCAGTTAC ATTTCTTGGGTGAGACAGGC ACCTGGGCAGGGTCTCGAG TGGATTGCTTGGATCTATGCC GGAACAGGGGACACAAACT ACAACCAGAAGTTCACCGG TCGGGTGACCATGACTACCG ATACCTCTACCAGTACAGCA TACATGGAGCTTCGTTCACT GCGAAGCGACGATACCGCT GTATATTACTGTGCCAGACA CGGACTGACAGGTCGTCACT GGTATTTCGCCGTGTGGGGG CAGGGTACAACTGTGACTGT GTCCTCC |
| 30 | QAVVTQEPSLTVS PGGTVTLTCRSST GAVTTSNIANWV QQKPGQAFRGLIG GTNNRGPGVPAR FSGSLLGDKAALT LSGAQPEDEAEY YCILWYSNKFVFG GGTKLTVL | Light chain variable region of affinity-matured humanized antibody V28 (tgil005 V28-hIgG1/4 VL) | 111 | CAAGCCGTTGTTACCCAGGA ACCTTCATTGACCGTTAGCC CAGGCGGGACCGTGACCCT TACCTGTCGGTCATCCACAG GTGCCGTGACCACAAGCAAT ATTGCTAATTGGGTCCAACA GAAGCCTGGCCAAGCTTTCA GGGGGCTGATCGGGGGTAC AAATAACAGAGGTCCTGGG GTTCCTGCCAGATTTTCCGG ATCTTTGCTGGGAGACAAAG CCGCCTTGACTCTCAGTGGC GCCCAGCCAGAAGACGAGG CCGAGTATTACTGCATCCTGT GGTATTCCAACAAGTTCGTC TTTGGGGGCGGAACCAAAC TTACAGTTTTG |
| 31 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGMTGRGWYFAV WGQGTTVTVSS | Heavy chain variable region of affinity-matured humanized antibody V40 (tgi1005 V40-hIgG1/4 VH) | 112 | CAGGTCCAACTCGTACAGTC AGGTGCAGAAGTCAAAAA CCTGGGGCTTCCGTGAAAGT GAGCTGCAAGGCTAGTGGAT ACACCTTTACCAGTAGCTAC ATCTCATGGGTGAGACAGGC TCCAGGACAGGGACTGGAA TGGATTGCTTGGATCTATGCT GGCACTGGTGACACCAATTA CAACCAAAAGTTCACCGGC AGAGTGACCATGACAACAG ATACCTCTACTTCCACCGCTT ACATGGAGTTGAGGTCCCTT AGGAGCGATGATACAGCTGT |
| | | | | GTACTACTGTGCACGCCACG GTATGACTGGCAGGGGGTG GTATTTTGCTGTGTGGGGAC AGGGAACTACAGTTACCGTG TCTTCA |
| 32 | QAVVTQEPSLTVS PGGTVTLTCRSST GAVTTSNLANWV QQKPGQAFRGLIG HTNNRAPGVPAR FSGSLLGDKAALT LSGAQPEDEAEY YCILWYSNKFVFG GGTKLTVL | Light chain variable region of affinity-matured humanized antibody V40 (tgil005 V40-hIgG1/4 VL) | 113 | CAAGCCGTCGTGACACAAG AGCCATCCCTTACAGTCTCA CCCGGCGGTACCGTTACCCT GACTTGCCGATCCTCCACAG GTGCCGTGACCACCTCCAAT CTCGCTAATTGGGTGCAGCA GAAGCCAGGGCAGGCTTTT CGTGGACTGATCGGGCACAC TAACAATAGAGCACCAGGTG TGCCCGCCCGGTTTTCAGGT TCCTTGTTGGGCGACAAGGC TGCTCTGACCTTGAGTGGAG CTCAACCTGAGGACGAAGC AGAGTACTACTGCATCCTCT GGTATAGCAACAAGTTTGTG TTTGGGGGCGGGACTAAGTT GACCGTGCTG |
| 33 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGYTGRGWYFAV WGQGTTVTVSSA STKGPSVFPLAPS SKSTSGGTAALGC LVKDYFPEPVTVS WNSGALTSGVHT FPAVLQSSGLYSLS SVVTVPSSSLGTQ TYICNVNHKPSNT KVDKKVEPKSCD KTHTCPPCPAPEL LGGPSVFLFPPKP KDTLMISRTPEVT CVVVDVSHEDPE VKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH | Heavy chain of affinity-matured humanized antibody V20 (tgil005 V20-hIgG1 H) | 114 | CAGGTGCAACTGGTGCAGA GTGGGGCAGAAGTAAAAA ACCAGGGGCTTCTGTTAAGG TGAGCTGTAAGGCAAGTGG ATACACCTTTACCTCATCATA CATCAGCTGGGTACGGCAAG CTCCTGGACAAGGTCTGGA ATGGATTGCTTGGATCTACG CTGGAACCGGTGACACCAAT TACAACCAGAAGTTTACTGG AAGGGTGACCATGACTACCG ACACATCAACCTCCACTGCT TATATGGAGCTGAGAAGCCT GCGAAGCGACGACACTGCT GTTTACTATTGCGCTCGTCAC GGCTATACCGGAAGAGGCTG GTATTTTGCTGTTTGGGGTC AGGGTACAACTGTGACAGT GTCAAGC GCCTCCACTAAGGGACCTTC CGTATTTCCTCTTGCCCCTAG CAGTAAGTCAACTTCCGGAG GAACCGCAGCCCTGGGATG CCTTGTAAAGGATTATTTTCC CGAACCCGTTACCGTGTCAT GGAATAGCGGAGCTCTCACC TCTGGTGTGCACACTTTCCC TGCAGTGCTCCAGAGCAGC GGACTGTATAGTCTGTCTAG TGTCGTAACAGTGCCTAGCT CTTCACTGGGCACCCAGACA TACATCTGTAATGTGAACCA CAAACCATCCAATACCAAGG TGGACAAGAAGGTGGAGCC |
| | YTQKSLSLSPGK | | | CAAGAGTTGCGACAAGACA CATACTTGTCCACCTTGTCC CGCTCCTGAACTGCTCGGAG GCCCCTCCGTCTTCCTGTTC CCCCCTAAGCCTAAGGACAC CTTGATGATCTCCAGAACAC CCGAAGTCACCTGTGTGGTG GTGGATGTGTCTCATGAAGA CCCAGAAGTGAAGTTTAACT GGTACGTGGATGGGGTCGA AGTCCATAATGCTAAAACTA AGCCCCGAGAGGAGCAATAT AACTCCACCTACCGTGTCGT GTCTGTTTTGACAGTGCTGC ATCAGGATTGGCTTAACGGT AAGGAGTATAAATGCAAGGT GAGCAACAAAGCTCTGCCC GCACCCATCGAGAAGACAAT ATCTAAGGCAAAAGGGCAG CCACGGGAACCCCAGGTCT ATACTCTCCCACCCTCTAGG GAGGAGATGACTAAGAACC AGGTGTCTCTGACATGTCTC GTAAAAGGCTTCTACCCTTC TGACATTGCCGTTGAATGGG AATCCAACGGCCAGCCTGA GAACAACTACAAGACAACC CCCCCCGTCCTGGATAGTGA CGGCAGTTTCTTCTTGTACA GCAAGCTGACTGTGGATAAG TCCCGATGGCAGCAGGGTAA TGTATTCTCCTGCAGCGTCAT GCATGAGGCACTTCATAACC ACTATACACAGAAAAGCCTG TCACTGTCCCCCGGCAAA |
| 34 | QAVVTQEPSLTVS PGGTVTLTCRSST GAVTTSNLANWV QQKPGQAFRGLIG HTNNRAPGVPAR FSGSLLGDKAALT LSGAQPEDEAEY YCALWYSNKFVF GGGTKLTVLGQP KAAPSVTLFPPSS EELQANKATLVCL ISDFYPGAVTVAW KADSSPVKAGVE TTTPSKQSNNKYA ASSYLSLTPEQWK SHRSYSCQVTHEG STVEKTVAPTECS | Light chain of affinity-matured humanized antibody V20 (tgil005 V20-hIgG1 L) | 115 | CAGGCCGTTGTTACCCAAGA ACCTTCCCTCACCGTTTCCC CAGGCGGCACAGTGACCTT GACTTGTCGCTCTAGCACAG GCGCCGTAACAACTTCCAAT CTGGCCAATTGGGTGCAGCA GAAGCCTGGACAGGCATTTA GGGGCCTGATTGGACACACT AACAACAGAGCCCCCGGAG TACCTGCTAGGTTCAGTGGC AGTCTGTTGGGAGACAAAG CAGCTCTTACACTGTCAGGC GCCCAGCCAGAGGATGAGG CAGAGTACTACTGTGCCCTC TGGTACTCAAATAAGTTCGT CTTCGGAGGGGGACTAAG CTGACTGTTCTT |
| | | | | GGCCAACCTAAAGCCGCTCC CTCCGTGACCCTTTTCCCCC |
| | | | | CTAGCAGTGAAGAATTGCAG GCAAATAAGGCCACATTGGT GTGCTTGATCAGCGACTTTT ACCCCGGAGCAGTCACTGT GGCATGGAAAGCTGATTCCT CCCCTGTCAAAGCAGGTGTT GAGACTACCACCCCCTCTAA GCAGAGCAATAATAAGTACG CTGCCAGTAGCTACCTGTCT TTGACTCCCGAGCAGTGGA AATCTCACAGATCCTATTCCT GCCAAGTTACTCACGAGGG AAGTACAGTGGAGAAAACT GTGGCTCCTACAGAGTGTAG C |
| 35 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGYTGRGWYFAV WGQGTTVTVSSA STKGPSVFPLAPS SKSTSGGTAALGC LVKDYFPEPVTVS WNSGALTSGVHT FPAVLQSSGLYSLS SVVTVPSSSLGTQ TYICNVNHKPSNT KVDKKVEPKSCD KTHTCPPCPAPEL LGGPSVFLFPPKP KDTLMISRTPEVT CVVVDVSHEDPE VKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH YTQKSLSLSPGK | Heavy chain of affinity-matured humanized antibody V21 (tgi1005 V21-hIgG1 H) | 116 | CAGGTGCAACTGGTGCAGA GTGGGGCAGAAGTAAAAA ACCAGGGGCTTCTGTTAAGG TGAGCTGTAAGGCAAGTGG ATACACCTTTACCTCATCATA CATCAGCTGGGTACGGCAAG CTCCTGGACAAGGTCTGGA ATGGATTGCTTGGATCTACG CTGGAACCGGTGACACCAAT TACAACCAGAAGTTTACTGG AAGGGTGACCATGACTACCG ACACATCAACCTCCACTGCT TATATGGAGCTGAGAAGCCT GCGAAGCGACGACACTGCT GTTTACTATTGCGCTCGTCAC GGCTATACCGGAAGAGGCTG GTATTTTGCTGTTTGGGGTC AGGGTACAACTGTGACAGT GTCAAGCGCCTCCACTAAGG GACCTTCCGTATTTCCTCTTG CCCCTAGCAGTAAGTCAACT TCCGGAGGAACCGCAGCCC TGGGATGCCTTGTAAAGGAT TATTTTCCCGAACCCGTTACC GTGTCATGGAATAGCGGAGC TCTCACCTCTGGTGTGCACA CTTTCCCTGCAGTGCTCCAG AGCAGCGGACTGTATAGTCT GTCTAGTGTCGTAACAGTGC CTAGCTCTTCACTGGGCACC CAGACATACATCTGTAATGT GAACCACAAACCATCCAATA CCAAGGTGGACAAGAAGGT GGAGCCCAAGAGTTGCGAC AAGACACATACTTGTCCACC TTGTCCCGCTCCTGAACTGC TCGGAGGCCCCTCCGTCTTC CTGTTCCCCCCTAAGCCTAA GGACACCTTGATGATCTCCA GAACACCCGAAGTCACCTG TGTGGTGGTGGATGTGTCTC |
| | | | | ATGAAGACCCAGAAGTGAA GTTTAACTGGTACGTGGATG GGGTCGAAGTCCATAATGCT AAAACTAAGCCCCGAGAGG AGCAATATAACTCCACCTAC CGTGTCGTGTCTGTTTTGAC AGTGCTGCATCAGGATTGGC TTAACGGTAAGGAGTATAAA TGCAAGGTGAGCAACAAAG CTCTGCCCGCACCCATCGAG AAGACAATATCTAAGGCAAA AGGGCAGCCACGGGAACCC CAGGTCTATACTCTCCCACC CTCTAGGGAGGAGATGACTA AGAACCAGGTGTCTCTGAC ATGTCTCGTAAAAGGCTTCT ACCCTTCTGACATTGCCGTT GAATGGGAATCCAACGGCC AGCCTGAGAACAACTACAA GACAACCCCCCCGTCCTGG ATAGTGACGGCAGTTTCTTC TTGTACAGCAAGCTGACTGT GGATAAGTCCCGATGGCAGC AGGGTAATGTATTCTCCTGC AGCGTCATGCATGAGGCACT TCATAACCACTATACACAGA AAAGCCTGTCACTGTCCCCC GGCAAA |
| 36 | QAVVTQEPSLTVS PGGTVTLTCRSST GAVTTSNYANWV QQKPGQAFRGLIG GTNNRAPGVPAR FSGSLLGDKAALT LSGAQPEDEAEY YCILWYSNHFVFG GGTKLTVLGQPK AAPSVTLFPPSSEE LQANKATLVCLIS DFYPGAVTVAWK ADSSPVKAGVETT TPSKQSNNKYAAS SYLSLTPEQWKSH RSYSCQVTHEGST VEKTVAPTECS | Light chain of affinity-matured humanized antibody V21 (tgi1005 V21-hIgG1 L) | 117 | CAAGCCGTTGTGACCCAAG AGCCCAGCCTGACTGTAAGC CCCGGCGGCACAGTCACAC TGACTTGCCGCTCAAGTACA GGCGCTGTGACTACCTCAAA CTATGCCAACTGGGTGCAGC AGAAACCAGGCCAAGCTTT TAGGGGTCTGATAGGCGGGA CTAACAACAGAGCACCTGG GGTGCCTGCCAGGTTTAGTG GTTCCCTGCTTGGCGATAAG GCTGCTTTGACTTTGTCTGG TGCTCAACCCGAGGACGAG GCTGAGTACTACTGTATCCT CTGGTATAGCAACCATTTCG TTTTCGGCGGAGGCACCAA GTTGACTGTGCTGGGCCAAC CTAAAGCCGCTCCCTCCGTG ACCCTTTTCCCCCCTAGCAG TGAAGAATTGCAGGCAAATA AGGCCACATTGGTGTGCTTG ATCAGCGACTTTTACCCCGG AGCAGTCACTGTGGCATGGA AAGCTGATTCCTCCCCTGTC AAAGCAGGTGTTGAGACTA CCACCCCCTCTAAGCAGAGC AATAATAAGTACGCTGCCAG TAGCTACCTGTCTTTGACTC |
| | | | | CCGAGCAGTGGAAATCTCAC AGATCCTATTCCTGCCAAGT TACTCACGAGGGAAGTACA GTGGAGAAAACTGTGGCTC CTACAGAGTGTAGC |
| 37 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGLTGRHWYFAV WGQGTTVTVSSA STKGPSVFPLAPS SKSTSGGTAALGC LVKDYFPEPVTVS WNSGALTSGVHT FPAVLQSSGLYSLS SVVTVPSSSLGTQ TYICNVNHKPSNT KVDKKVEPKSCD KTHTCPPCPAPEL LGGPSVFLFPPKP KDTLMISRTPEVT CVVVDVSHEDPE VKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH YTQKSLSLSPGK | Heavy chain of affinity-matured humanized antibody V28 (tgil005 V28-hIgG1 H) | 118 | CAGGTCCAGCTCGTTCAAAG TGGTGCAGAAGTCAAGAAG CCCGGCGCTAGTGTCAAAGT TTCCTGCAAGGCCAGCGGAT ACACCTTTACAAGCAGTTAC ATTTCTTGGGTGAGACAGGC ACCTGGGCAGGGTCTCGAG TGGATTGCTTGGATCTATGCC GGAACAGGGGACACAAACT ACAACCAGAAGTTCACCGG TCGGGTGACCATGACTACCG ATACCTCTACCAGTACAGCA TACATGGAGCTTCGTTCACT GCGAAGCGACGATACCGCT GTATATTACTGTGCCAGACA CGGACTGACAGGTCGTCACT GGTATTTCGCCGTGTGGGGG CAGGGTACAACTGTGACTGT GTCCTCCGCCTCCACTAAGG GACCTTCCGTATTTCCTCTTG CCCCTAGCAGTAAGTCAACT TCCGGAGGAACCGCAGCCC TGGGATGCCTTGTAAAGGAT TATTTCCCGAACCCGTTACC GTGTCATGGAATAGCGGAGC TCTCACCTCTGGTGTGCACA CTTTCCCTGCAGTGCTCCAG AGCAGCGGACTGTATAGTCT GTCTAGTGTCGTAACAGTGC CTAGCTCTTCACTGGGCACC CAGACATACATCTGTAATGT GAACCACAAACCATCCAATA CCAAGGTGGACAAGAAGGT GGAGCCCAAGAGTTGCGAC AAGACACATACTTGTCCACC TTGTCCCGCTCCTGAACTGC TCGGAGGCCCCTCCGTCTTC CTGTTCCCCCCTAAGCCTAA GGACACCTTGATGATCTCCA GAACACCCGAAGTCACCTG TGTGGTGGTGGATGTGTCTC ATGAAGACCCAGAAGTGAA GTTTAACTGGTACGTGGATG GGGTCGAAGTCCATAATGCT AAAACTAAGCCCCGAGAGG AGCAATATAACTCCACCTAC CGTGTCGTGTCTGTTTTGAC AGTGCTGCATCAGGATTGGC TTAACGGTAAGGAGTATAAA TGCAAGGTGAGCAACAAAG CTCTGCCCGCACCCATCGAG AAGACAATATCTAAGGCAAA |
| | | | | AGGGCAGCCACGGAACCC CAGGTCTATACTCTCCCACC CTCTAGGGAGGAGATGACTA AGAACCAGGTGTCTCTGAC ATGTCTCGTAAAAGGCTTCT ACCCTTCTGACATTGCCGTT GAATGGGAATCCAACGGCC AGCCTGAGAACAACTACAA GACAACCCCCCCCGTCCTGG ATAGTGACGGCAGTTTCTTC TTGTACAGCAAGCTGACTGT GGATAAGTCCCGATGGCAGC AGGGTAATGTATTCTCCTGC AGCGTCATGCATGAGGCACT TCATAACCACTATACACAGA AAAGCCTGTCACTGTCCCCC GGCAAA |
| 38 | QAVVTQEPSLTVS PGGTVTLTCRSST GAVTTSNIANWV QQKPGQAFRGLIG GTNNRGPGVPAR FSGSLLGDKAALT LSGAQPEDEAEY YCILWYSNKFVFG GGTKLTVLGQPK AAPSVTLFPPSSEE LQANKATLVCLIS DFYPGAVTVAWK ADSSPVKAGVETT TPSKQSNNKYAAS SYLSLTPEQWKSH RSYSCQVTHEGST VEKTVAPTECS | Light chain of affinity-matured humanized antibody V28 (tgil005 V28-hIgG1 L) | 119 | CAAGCCGTTGTTACCCAGGA ACCTTCATTGACCGTTAGCC CAGGCGGGACCGTGACCCT TACCTGTCGGTCATCCACAG GTGCCGTGACCACAAGCAAT ATTGCTAATTGGGTCCAACA GAAGCCTGGCCAAGCTTTCA GGGGGCTGATCGGGGGTAC AAATAACAGAGGTCCTGGG GTTCCTGCCAGATTTTCCGG ATCTTTGCTGGGAGACAAAG CCGCCTTGACTCTCAGTGGC GCCCAGCCAGAAGACGAGG CCGAGTATTACTGCATCCTGT GGTATTCCAACAAGTTCGTC TTTGGGGGCGGAACCAAAC TTACAGTTTTGGGCCAACCT AAAGCCGCTCCCTCCGTGAC CCTTTTCCCCCCTAGCAGTG AAGAATTGCAGGCAAATAA GGCCACATTGGTGTGCTTGA TCAGCGACTTTTACCCCGGA GCAGTCACTGTGGCATGGAA AGCTGATTCCTCCCCTGTCA AAGCAGGTGTTGAGACTAC CACCCCCTCTAAGCAGAGCA ATAATAAGTACGCTGCCAGT AGCTACCTGTCTTTGACTCC CGAGCAGTGGAAATCTCAC AGATCCTATTCCTGCCAAGT TACTCACGAGGGAAGTACA GTGGAGAAAACTGTGGCTC CTACAGAGTGTAGC |
| 39 | QVQLVQSGAEVK KPGASVKVSCKA SGYTFTSSYISWV RQAPGQGLEWIA WIYAGTGDTNYN QKFTGRVTMTTD TSTSTAYMELRSL | Heavy chain of affinity-matured humanized antibody V40 (tgil005 V40-hIgGl H) | 120 | CAGGTCCAACTCGTACAGTC AGGTGCAGAAGTCAAAAA CCTGGGGCTTCCGTGAAAGT GAGCTGCAAGGCTAGTGGAT ACACCTTTACCAGTAGCTAC ATCTCATGGGTGAGACAGGC TCCAGGACAGGGACTGGAA |
| | RSDDTAVYYCAR HGMTGRGWYFAV WGQGTTVTVSSA STKGPSVFPLAPS SKSTSGGTAALGC LVKDYFPEPVTVS WNSGALTSGVHT FPAVLQSSGLYSLS SVVTVPSSSLGTQ TYICNVNHKPSNT KVDKKVEPKSCD KTHTCPPCPAPEL LGGPSVFLFPPKP KDTLMISRTPEVT CVVVDVSHEDPE VKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH YTQKSLSLSPGK | | | TGGATTGCTTGGATCTATGCT GGCACTGGTGACACCAATTA CAACCAAAAGTTCACCGGC AGAGTGACCATGACAACAG ATACCTCTACTTCCACCGCTT ACATGGAGTTGAGGTCCCTT AGGAGCGATGATACAGCTGT GTACTACTGTGCACGCCACG GTATGACTGGCAGGGGGTG GTATTTTGCTGTGTGGGGAC AGGGAACTACAGTTACCGTG TCTTCAGCCTCCACTAAGGG ACCTTCCGTATTTCCTCTTGC CCCTAGCAGTAAGTCAACTT CCGGAGGAACCGCAGCCCT GGGATGCCTTGTAAAGGATT ATTTTCCCGAACCCGTTACC GTGTCATGGAATAGCGGAGC TCTCACCTCTGGTGTGCACA CTTTCCCTGCAGTGCTCCAG AGCAGCGGACTGTATAGTCT GTCTAGTGTCGTAACAGTGC CTAGCTCTTCACTGGGCACC CAGACATACATCTGTAATGT GAACCACAAACCATCCAATA CCAAGGTGGACAAGAAGGT GGAGCCCAAGAGTTGCGAC AAGACACATACTTGTCCACC TTGTCCCGCTCCTGAACTGC TCGGAGGCCCCTCCGTCTTC CTGTTCCCCCCTAAGCCTAA GGACACCTTGATGATCTCCA GAACACCCGAAGTCACCTG TGTGGTGGTGGATGTGTCTC ATGAAGACCCAGAAGTGAA GTTTAACTGGTACGTGGATG GGGTCGAAGTCCATAATGCT AAAACTAAGCCCCGAGAGG AGCAATATAACTCCACCTAC CGTGTCGTGTCTGTTTTGAC AGTGCTGCATCAGGATTGGC TTAACGGTAAGGAGTATAAA TGCAAGGTGAGCAACAAAG CTCTGCCCGCACCCATCGAG AAGACAATATCTAAGGCAAA AGGGCAGCCACGGGAACCC CAGGTCTATACTCTCCCACC CTCTAGGGAGGAGATGACTA AGAACCAGGTGTCTCTGAC ATGTCTCGTAAAAGGCTTCT ACCCTTCTGACATTGCCGTT GAATGGGAATCCAACGGCC AGCCTGAGAACAACTACAA GACAACCCCCCCCGTCCTGG ATAGTGACGGCAGTTTCTTC TTGTACAGCAAGCTGACTGT GGATAAGTCCCGATGGCAGC |
| | | | | AGGGTAATGTATTCTCCTGC AGCGTCATGCATGAGGCACT TCATAACCACTATACACAGA AAAGCCTGTCACTGTCCCCC GGCAAA |
| 40 | QAVVTQEPSLTVS PGGTVTLTCRSST GAVTTSNLANWV QQKPGQAFRGLIG HTNNRAPGVPAR FSGSLLGDKAALT LSGAQPEDEAEY YCILWYSNKFVFG GGTKLTVL GQPKAAPSVTLFP PSSEELQANKATL VCLISDFYPGAVT VAWKADSSPVKA GVETTTPSKQSNN KYAASSYLSLTPE QWKSHRSYSCQV THEGSTVEKTVAP TECS | Light chain of affinity-matured humanized antibody V40 (tgil005 V40-hIgG1 L) | 121 | CAAGCCGTCGTGACACAAG AGCCATCCCTTACAGTCTCA CCCGGCGGTACCGTTACCCT GACTTGCCGATCCTCCACAG GTGCCGTGACCACCTCCAAT CTCGCTAATTGGGTGCAGCA GAAGCCAGGGCAGGCTTTT CGTGGACTGATCGGGCACAC TAACAATAGAGCACCAGGTG TGCCCGCCCGGTTTTCAGGT TCCTTGTTGGGCGACAAGGC TGCTCTGACCTTGAGTGGAG CTCAACCTGAGGACGAAGC AGAGTACTACTGCATCCTCT GGTATAGCAACAAGTTTGTG TTTGGGGGCGGGACTAAGTT GACCGTGCTGGGCCAACCTA AAGCCGCTCCCTCCGTGACC CTTTTCCCCCCTAGCAGTGA AGAATTGCAGGCAAATAAG GCCACATTGGTGTGCTTGAT CAGCGACTTTTACCCCGGAG CAGTCACTGTGGCATGGAAA GCTGATTCCTCCCCTGTCAA AGCAGGTGTTGAGACTACC ACCCCCTCTAAGCAGAGCA ATAATAAGTACGCTGCCAGT AGCTACCTGTCTTTGACTCC CGAGCAGTGGAAATCTCAC AGATCCTATTCCTGCCAAGT TACTCACGAGGGAAGTACA GTGGAGAAAACTGTGGCTC CTACAGAGTGTAGC |
| 41 | ASTKGPSVFPLAP SSKSTSGGTAALG CLVKDYFPEPVTV SWNSGALTSGVH TFPAVLQSSGLYSL SSVVTVPSSSLGT QTYICNVNHKPSN TKVDKKVEPKSC DKTHTCPPCPAPE LLGGPSVFLFPPK PKDTLMISRTPEV TCVVVDVSHEDP EVKFNWYVDGVE VHNAKTKPREEQ YNSTYRVVSVLTV LHQDWLNGKEYK CKVSNKALPAPIE KTISKAKGQPREP QVYTLPPSREEMT | Heavy chain constant region IgG1(WT) | 122 | GCCTCCACTAAGGACCTTC CGTATTTCCTCTTGCCCCTAG CAGTAAGTCAACTTCCGGAG GAACCGCAGCCCTGGGATG CCTTGTAAAGGATTATTTTCC CGAACCCGTTACCGTGTCAT GGAATAGCGGAGCTCTCACC TCTGGTGTGCACACTTTCCC TGCAGTGCTCCAGAGCAGC GGACTGTATAGTCTGTCTAG TGTCGTAACAGTGCCTAGCT CTTCACTGGGCACCCAGACA TACATCTGTAATGTGAACCA CAAACCATCCAATACCAAGG TGGACAAGAAGGTGGAGCC CAAGAGTTGCGACAAGACA CATACTTGTCCACCTTGTCC CGCTCCTGAACTGCTCGGAG GCCCCTCCGTCTTCCTGTTC |
| | KNQVSLTCLVKGF YPSDIAVEWESNG QPENNYKTTPPVL DSDGSFFLYSKLT VDKSRWQQGNVF SCSVMHEALHNH YTQKSLSLSPGK | | | CCCCCTAAGCCTAAGGACAC CTTGATGATCTCCAGAACAC CCGAAGTCACCTGTGTGGTG GTGGATGTGTCTCATGAAGA CCCAGAAGTGAAGTTTAACT GGTACGTGGATGGGGTCGA AGTCCATAATGCTAAAACTA AGCCCCGAGAGGAGCAATAT AACTCCACCTACCGTGTCGT GTCTGTTTTGACAGTGCTGC ATCAGGATTGGCTTAACGGT AAGGAGTATAAATGCAAGGT GAGCAACAAAGCTCTGCCC GCACCCATCGAGAAGACAAT ATCTAAGGCAAAAGGGCAG CCACGGGAACCCCAGGTCT ATACTCTCCCACCCTCTAGG GAGGAGATGACTAAGAACC AGGTGTCTCTGACATGTCTC GTAAAAGGCTTCTACCCTTC TGACATTGCCGTTGAATGGG AATCCAACGGCCAGCCTGA GAACAACTACAAGACAACC CCCCCCGTCCTGGATAGTGA CGGCAGTTTCTTCTTGTACA GCAAGCTGACTGTGGATAAG TCCCGATGGCAGCAGGGTAA TGTATTCTCCTGCAGCGTCAT GCATGAGGCACTTCATAACC ACTATACACAGAAAAGCCTG TCACTGTCCCCCGGCAAA |
| 42 | ASTKGPSVFPLAP SSKSTSGGTAALG CLVKDYFPEPVTV SWNSGALTSGVH TFPAVLQSSGLYSL SSVVTVPSSSLGT QTYICNVNHKPSN TKVDKKVEPKSC DKTHTCPPCPAPE AAGGPSVFLFPPK PKDTLMISRTPEV TCVVVDVSHEDP EVKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH YTQKSLSLSPGK | Heavy chain constant region hIgG1 (L234A and L235A variant) | 123 | GCCTCAACCAAAGGACCATC CGTGTTTCCCCTTGCCCCAT CCTCCAAGTCCACCAGTGGC GGGACTGCCGCTTTGGGGTG CCTCGTGAAGGATTATTTCC CTGAGCCTGTCACAGTCAGT TGGAATTCCGGGGCACTGAC CTCTGGCGTGCATACTTTCC CAGCTGTGCTGCAGAGCTCA GGGTTGTACAGCCTCTCATC TGTGGTAACAGTACCCAGCT CATCCCTTGGAACCCAGACC TACATCTGCAACGTTAACCA CAAGCCATCAAACACAAAG GTGGATAAGAAAGTTGAGC CCAAGTCTTGCGATAAGACA CACACTTGTCCTCCTTGTCC TGCACCTGAAGCTGCAGGA GGGCCTAGTGTCTTCCTGTT CCCACCAAAGCCCAAAGAT ACACTGATGATCAGCAGGAC CCCAGAGGTCACTTGTGTAG TGGTGGATGTCAGCCATGAA GATCCCGAGGTAAAATTCAA CTGGTACGTAGACGGAGTGG AGGTACACAATGCCAAGAC |
| | | | | AAAACCAAGAGAGGAACAG TACAACAGCACTTACCGCGT GGTCAGTGTGCTGACCGTGC TTCACCAGGATTGGCTTAAC GGCAAAGAGTACAAGTGTA AGGTTTCTAACAAGGCATTG CCAGCCCCCATCGAGAAGA CCATCAGTAAGGCTAAGGGC CAGCCTCGAGAGCCTCAGG TCTACACACTCCCCCCATCT CGAGAAGAGATGACCAAAA ATCAGGTCTCCCTGACTTGT CTCGTGAAGGGATTCTATCC CAGCGATATAGCCGTCGAAT GGGAATCCAATGGACAGCC AGAGAATAATTACAAGACCA CTCCACCAGTACTGGACTCC GATGGCAGTTTTTTCCTGTA CAGCAAACTGACTGTTGAC AAATCTCGATGGCAGCAGGG CAACGTGTTCTCATGCTCTG TCATGCATGAAGCTCTGCAT AATCATTACACCCAGAAGAG CCTCAGTCTGTCCCCCGGCA AG |
| 43 | ASTKGPSVFPLAP CSRSTSESTAALG CLVKDYFPEPVTV SWNSGALTSGVH TFPAVLQSSGLYSL SSVVTVPSSSLGT KTYTCNVDHKPS NTKVDKRVESKY GPPCPPCPAPEFLG GPSVFLFPPKPKD TLMISRTPEVTCV VVDVSQEDPEVQ FNWYVDGVEVH NAKTKPREEQFNS TYRVVSVLTVLHQ DWLNGKEYKCK VSNKGLPSSIEKTI SKAKGQPREPQV YTLPPSQEEMTKN QVSLTCLVKGFYP SDIAVEWESNGQP ENNYKTTPPVLDS DGSFFLYSRLTVD KSRWQEGNVFSC SVMHEALHNHYT QKSLSLSLGK | Heavy chain constant region IgG4 (WT) | 124 | GCTTCAACCAAAGGCCCTTC CGTATTCCCCCTCGCCCCAT GCAGCAGAAGCACCTCCGA AAGCACAGCTGCTTTGGGTT GCCTGGTCAAGGACTACTTC CCAGAGCCCGTAACCGTTTC TTGGAACTCCGGCGCCTTGA CATCTGGCGTGCACACCTTC CCTGCCGTCCTGCAAAGTAG CGGCCTGTACAGCTTGTCCT CTGTGGTTACCGTGCCATCC TCTAGCCTCGGCACAAAGAC CTACACTTGCAATGTGGACC ACAAACCCAGCAACACAAA GGTTGACAAGAGGGTGGAG AGCAAATATGGCCCACCTTG CCCACCATGCCCAGCTCCAG AGTTTCTGGGGGGACCTTCT GTATTTCTGTTCCCACCAAA GCCTAAGGACACCCTGATGA TTTCCAGGACCCCCGAGGTG ACATGCGTGGTTGTGGATGT GTCCCAGGAAGATCCTGAA GTGCAATTTAACTGGTACGT CGACGGGGTAGAGGTCCAC AATGCTAAGACAAAGCCAC GGGAGGAGCAGTTTAACAG TACATATCGCGTTGTGTCCGT GTTGACTGTGCTGCATCAGG ATTGGCTCAATGGGAAGGAG TATAAGTGCAAGGTCTCTAA CAAAGGTCTGCCCTCCTCTA |
| | | | | TCGAGAAAACCATTAGCAA GGCAAAGGGTCAGCCTAGG GAACCCCAGGTTTATACCCT GCCTCCCTCTCAGGAAGAG ATGACCAAGAACCAGGTGA GCCTTACTTGCCTGGTAAAA GGCTTCTATCCTTCAGACATA GCTGTGGAGTGGGAATCCA ACGGTCAACCTGAGAACAA CTACAAAACCACTCCTCCAG TGCTGGATTCTGATGGATCC TTTTTCCTGTACAGCCGGCT GACAGTGGATAAATCAAGGT GGCAGGAAGGGAACGTGTT CTCATGTAGCGTGATGCATG AAGCACTGCACAACCACTAT ACCCAGAAGAGTCTTTCTCT GTCACTTGGCAAA |
| 44 | GQPKAAPSVTLFP PSSEELQANKATL VCLISDFYPGAVT VAWKADSSPVKA GVETTTPSKQSNN KYAASSYLSLTPE QWKSHRSYSCQV THEGSTVEKTVAP TECS | Light chain constant region lambda | 125 | GGCCAACCTAAAGCCGCTCC CTCCGTGACCCTTTTCCCCC CTAGCAGTGAAGAATTGCAG GCAAATAAGGCCACATTGGT GTGCTTGATCAGCGACTTTT ACCCCGGAGCAGTCACTGT GGCATGGAAAGCTGATTCCT CCCCTGTCAAAGCAGGTGTT GAGACTACCACCCCCTCTAA GCAGAGCAATAATAAGTACG CTGCCAGTAGCTACCTGTCT TTGACTCCCGAGCAGTGGA AATCTCACAGATCCTATTCCT GCCAAGTTACTCACGAGGG AAGTACAGTGGAGAAAACT GTGGCTCCTACAGAGTGTAG C |
| 45 | ATGGCTAGAG CTATGGCAGC TGCTTGGCCA CTGTTGTTGG TTGCATTGCT GGTGCTCTCT TGGCCACCTC CAGGAACAGG AGATGTGGTG GTCCAGGCAC CTACCCAGGT CCCTGGTTTC CTCGGGGACT CCGTGACCCT CCCATGTTAT CTGCAGGTGC CCAACATGGA GGTCACACAC GTTTCACAGC TTACCTGGGC CCGTCACGGG GAAAGTGGCT | Nucleotide sequence encoding human CD155 protein | | |
| | CTATGGCTGT GTTTCACCAG ACTCAAGGTC CTTCCTATAG CGAGTCAAAG CGACTCGAGT TTGTCGCCGC CAGGCTGGGA GCAGAATTGA GGAACGCTTC TCTGCGGATG TTTGGCCTGC GTGTGGAAGA CGAGGGAAAT TACACCTGCC TCTTTGTCAC TTTCCCTCAG GGCTCCCGTT CCGTGGACAT TTGGCTGAGG GTGCTGGCTA AACCCCAGAA CACTGCAGAA GTGCAGAAGG TGCAGCTTAC CGGCGAACCT GTGCCCATGG CTCGGTGTGT GTCTACCGGT GGAAGACCAC CTGCTCAGAT CACATGGCAT AGTGACCTGG GGGGGATGCC AAACACCTCA CAGGTCCCTG GCTTCTTGTC CGGTACAGTC ACTGTGACCT CACTTTGGAT CCTGGTGCCA TCCAGTCAGG TGGATGGCAA GAATGTGACA TGCAAGGTGG AACATGAGTC ATTTGAGAAA CCCCAACTGC TGACTGTGAA TCTGACCGTC TACTACCCAC CCGAAGTGTC TATCTCAGGC TACGATAATA ACTGGTATTT GGGTCAAAAT GAGGCAACAC | | | |
| | TTACTTGCGA TGCCCGTTCC AACCCTGAAC CTACCGGATA TAATTGGAGC ACCACCATGG GTCCACTCCC TCCATTTGCC GTGGCTCAAG GGGCCCAGTT GCTCATTAGG CCCGTCGATA AGCCAATTAA CACAACCCTT ATTTGCAATG TAACTAACGC CTTGGGGGCC AGACAGGCAG AACTGACCGT CCAGGTGAAG GAAGGACCAC CCTCAGAGCA TAGCGGAATC AGCAGGAACG CAATCATCTT TTTGGTGCTT GGGATTCTCG TGTTTCTGAT TCTTTTGGGC ATCGGCATTT ACTTTTATTG GTCCAAGTGC TCCCGTGAAG TGCTGTGGCA CTGCCACCTG TGCCCTTCAA GTACAGAGCA CGCATCTGCC AGCGCTAATG GGCACGTGAG TTATTCCGCT GTATCTAGAG AAAACAGTTC AAGCCAGGAT CCTCAGACCG AGGGAACCCG C | | | |
| 46 | ATGGCAAGAA CAATGGCCGC TGCCTGGCCT CCATTGCTTT TGACTCTGCT CGAATTGTCT TGGCCCCCCC CAGGAACCGG GGATATCATC GTCCAGGCCC CTACACAAGT | Nucleotide sequence encoding Rhesus CD155 protein | | |
| | CCCTGGCTTC CTGGGCGACT CAGTGACCCT GCCATGCTAT CTTCAGGTCC CTGGGATGGA GGAGACACAC GTGAGCCAGC TTACATGGTC AAGGCACGGT GAATCTGGCT CCATGGCAGT ATTCCATCAG ACACAGGGCC CCAATTACTC AGAACCCAAA AGGTTGGAAT TCGTCGCTGC CCGACTGGGT ACAGAACTTC GTGACGCCAG TCTGAGAATG TTCGGCCTGA GGGTCGAGGA CGAAGGCAAC TACACTTGTC TGTTTGTGAC CTTTCCACAG GGTTCTAGGT CTGTAGACAT CTGGTTGCGA GTCTTGGCAA AGCCACAGAA TACCGCCGAG GTGCAGAAGG TGCAGTTGAC AGGGAAGCCA GTCCCCGTGG CACGATGCGT ATCTACTGGT GGGCGGCCTC CTGCACATAT CACATGGCAC TCTGATCTGG GAGGGATGCC TAACACCTCT CAAGCCCCCG GCTTCTTGAG CGGGACCGTT ACAGTGACAT CACTGTGGAT CTTGGTGCCA TCATCCCAGG TTGACGGCAA GAGCGTGACC TGCAAAGTAG AGCACGAGTC | | | |
| | CTTTGAGAAG CCACAGCTGC TCACTGTCAA TTTGACTGTG TATTACCCTC CCGAGGTGTC CATCAGCGGG TATGACAACA ACTGGTATCT GAGCCAGAAT GAAGCCACCC TGACTTGCGA TGCACGATCT AATCCCGAGC CAACTGGTTA TAACTGGTCT ACTACAATGG GTCCATTGCC CCCATTTGCA GTCGCTCAGG GAGCCCAACT CTTGATACGG CCAGTGGATA AACCTATCAA TACTACCTTC ATCTGTAATG TTACTAACGC TCTGGGCGCT AGACAGGCAG AGCTCACCGT CCAGGTGAAG GAAGGCCCAC CCAGTGAGCC CTCAGGTATG AGCTCTAACA TCATTATTTT CCTCATACTG GGCATTGTGA TCCTCCTGAC TCTCCTTGGT ATCGGCGTGT ACTTTTACCG ATCTCGCTGC AGCCGGGAGT TTCTGTGGTG TCACCACTTG TCACCCAGTT CTGAGGAGCA TGCTTCTGCT AGCGCCAACG GCTATATCAG TTACAGCGAT GTGAGTAGGG AGGCATCAAG CAGCCAGGAT CCACAGACCG AGGGCACCAG A | | | |
| 47 | GFTFSSSY | Heavy chain variable region CDR1 of murine monoclonal antibody (mutgi1005 HCDR1) | 126 | GGGTTTACCTTCTCTTCTTCT TAC |
| 48 | IYAGTGDT | Heavy chain variable region CDR2 of murine monoclonal antibody (mutgi1005 HCDR2) | 127 | ATCTATGCTGGGACCGGAGA TACA |
| 49 | VRHGSTGRGWYF AV | Heavy chain variable region CDR3 of murine monoclonal antibody (mutgi1005 HCDR3) | 128 | GTGCGGCATGGAAGCACTG GTAGAGGTTGGTATTTCGCT GTC |
| 50 | TGAVTTSN | Light chain variable region CDR1 of murine monoclonal antibody (mutgi1005 LCDR1) | 129 | ACCGGTGCTGTAACAACCTC CAAT |
| 51 | GTN | Light chain variable region CDR2 of murine monoclonal antibody (mutgi1005 LCDR2) | 130 | GGGACTAAT |
| 52 | ALWYSNHFV | Light chain variable region CDR3 of murine monoclonal antibody (mutgi1005 LCDR3) | 131 | GCCCTGTGGTATTCTAACCA CTTCGTG |
| 53 | QVQMQQSGAELV KPGASVKLSCKTS GFTFSSSYISWLK QKPGQSLEWIAWI YAGTGDTNYNQK FTGKAQVTVDTSS STAYMQFSSLTTE DSAIYYCVRHGST GRGWYFAVWGA GTTVTVSS | Heavy chain variable region of murine monoclonal antibody (mutgi1005 VH) | 132 | CAAGTGCAGATGCAGCAGA GTGGTGCCGAATTGGTAAAA CCAGGGGCTTCTGTTAAGTT GTCCTGCAAGACCTCTGGGT TTACCTTCTCTTCTTCTTACA TCTCCTGGCTCAAACAGAAG CCAGGGCAGTCACTTGAGT GGATTGCCTGGATCTATGCT GGGACCGGAGATACAAACT ACAACCAGAAATTCACAGG CAAGGCCCAGGTGACCGTC GACACTTCTTCATCAACCGC CTATATGCAGTTCAGTTCCCT CACCACTGAGGATAGTGCCA TCTACTACTGCGTGCGGCAT GGAAGCACTGGTAGAGGTT GGTATTTCGCTGTCTGGGGT GCTGGTACAACTGTTACCGT GAGCTCC |
| 54 | QAVVTQESALTTS PGETVTLTCRSST GAVTTSNYANWV QEKPDHLFTGLIG GTNNRAPGVPAR FSGSLIGDKAALTI TGAQTEDEAIYFC ALWYSNHFVFGG GTKLTVL | Light chain variable region of murine monoclonal antibody (mutgi1005 VL) | 133 | CAAGCCGTTGTGACCCAAG AAAGTGCACTGACAACTTCC CCTGGAGAAACAGTTACACT GACTTGTAGGAGTAGCACCG GTGCTGTAACAACCTCCAAT TACGCCAACTGGGTGCAAG AGAAACCCGACCATCTGTTT ACAGGACTCATCGGCGGGA CTAATAATCGCGCTCCTGGA GTACCAGCTAGATTTTCCGG GTCATTGATTGGCGATAAGG CCGCCCTGACAATTACTGGG GCCCAAACTGAGGACGAAG CAATCTACTTCTGCGCCCTG |
| | | | | TGGTATTCTAACCACTTCGT GTTTGGAGGGGGCACTAAG TTGACCGTGCTC |
| 55 | QVQMQQSGAELV KPGASVKLSCKTS GFTFSSSYISWLK QKPGQSLEWIAWI YAGTGDTNYNQK FTGKAQVTVDTSS STAYMQFSSLTTE DSAIYYCVRHGST GRGWYFAVWGA GTTVTVSSAKTTA PSVYPLAPVCGDT TGSSVTLGCLVKG YFPEPVTLTWNSG SLSSGVHTFPAVL QSDLYTLSSSVTV TSSTWPSQSITCN VAHPASSTKVDK KIEPRGPTIKPCPP CKCPAPNLLGGPS VFIFPPKIKDVLMI SLSPIVTCVVVDV SEDDPDVQISWFV NNVEVHTAQTQT HREDYNSTLRVVS ALPIQHQDWMSG KEFKCKVNNKDL PAPIERTISKPKGS VRAPQVYVLPPPE EEMTKKQVTLTC MVTDFMPEDIYV EWTNNGKTELNY KNTEPVLDSDGSY FMYSKLRVEKKN WVERNSYSCSVV HEGLHNHHTTKS FSRTPGK | Heavy chain of murine monoclonal antibody (mutgi1005 H) | 134 | CAAGTGCAGATGCAGCAGA GTGGTGCCGAATTGGTAAAA CCAGGGGCTTCTGTTAAGTT GTCCTGCAAGACCTCTGGGT TTACCTTCTCTTCTTCTTACA TCTCCTGGCTCAAACAGAAG CCAGGGCAGTCACTTGAGT GGATTGCCTGGATCTATGCT GGGACCGGAGATACAAACT ACAACCAGAAATTCACAGG CAAGGCCCAGGTGACCGTC GACACTTCTTCATCAACCGC CTATATGCAGTTCAGTTCCCT CACCACTGAGGATAGTGCCA TCTACTACTGCGTGCGGCAT GGAAGCACTGGTAGAGGTT GGTATTTCGCTGTCTGGGGT GCTGGTACAACTGTTAGCCA AGACTACAGCCCCTTCAGTA TATCCCCTCGCCCCAGTTTG CGGGGATACTACCGGGTCCA GTGTCACACTTGGATGTCTG GTGAAGGGCTATTTTCCAGA GCCTGTGACACTGACATGGA ATAGCGGGAGTTTGAGTAGT GGCGTCCACACTTTCCCAGC TGTGCTGCAGTCTGATCTGT ACACTTTGTCCTCATCTGTG ACTGTGACCTCCTCTACTTG GCCATCACAGTCCATCACCT GCAATGTGGCCCACCCAGCC TCATCAACCAAAGTTGATAA GAAGATCGAGCCAAGAGGA CCAACCATAAAACCTTGTCC CCCCTGTAAATGTCCAGCAC CCAATCTGCTCGGTGGGCCT AGCGTCTTCATCTTCCCTCC CAAGATCAAGGACGTTCTCA TGATCTCACTGAGTCCCATT GTGACATGCGTCGTAGTAGA TGTGTCCGAGGATGACCCTG ATGTCCAGATCTCCTGGTTC GTTAATAATGTAGAGGTGCA CACCGCTCAAACACAGACT CATCGGGAGGATTATAATTCC ACCTTGCGGGTTGTGAGCGC CCTGCCAATACAGCATCAGG ATTGGATGTCTGGGAAAGAG TTCAAGTGCAAGGTGAACA ATAAGGACCTGCCCGCTCCC ATAGAGCGAACTATTTCTAA GCCAAAGGGAAGCGTGCGC GCACCTCAAGTCTACGTGCT CCCTCCACCTGAAGAGGAA |
| | | | | ATGACCAAGAAACAGGTTA CCCTGACATGTATGGTGACC GACTTTATGCCTGAGGATATT TACGTGGAGTGGACTAACAA CGGGAAGACCGAGCTGAAC TACAAGAATACAGAACCAGT GCTCGACAGTGATGGCAGCT ACTTTATGTACAGCAAGCTG CGGGTAGAGAAGAAGAACT GGGTGGAGCGCAACAGTTA CAGCTGTAGTGTGGTTCACG AGGGCCTGCACAACCACCA CACCACAAAAAGCTTCAGC CGCACTCCCGGAAAACCGT GAGCTCC |
| 56 | QAVVTQESALTTS PGETVTLTCRSST GAVTTSNYANWV QEKPDHLFTGLIG GTNNRAPGVPAR FSGSLIGDKAALTI TGAQTEDEAIYFC ALWYSNHFVFGG GTKLTVLQPKSSP SVTLFPPSSEELET NKATLVCTITDFY PGVVTVDWKVD GTPVTQGMETTQ PSKQSNNKYMAS SYLTLTARAWERH SSYSCQVTHEGHT VEKSLSRADCS | Light chain of murine monoclonal antibody (mutgi1005 L) | 135 | CAAGCCGTTGTGACCCAAG AAAGTGCACTGACAACTTCC CCTGGAGAAACAGTTACACT GACTTGTAGGAGTAGCACCG GTGCTGTAACAACCTCCAAT TACGCCAACTGGGTGCAAG AGAAACCCGACCATCTGTTT ACAGGACTCATCGGCGGGA CTAATAATCGCGCTCCTGGA GTACCAGCTAGATTTTCCGG GTCATTGATTGGCGATAAGG CCGCCCTGACAATTACTGGG GCCCAAACTGAGGACGAAG CAATCTACTTCTGCGCCCTG TGGTATTCTAACCACTTCGT GTTTGGAGGGGGCACTAAG TTGACCGTGCTCCAACCCAA ATCCTCTCCCAGCGTTACCC TTTTTCCCCCATCTAGTGAA GAACTGGAAACTAACAAAG CAACCCTCGTGTGCACCATC ACCGACTTTTACCCAGGCGT CGTCACAGTTGATTGGAAGG TGGATGGAACTCCCGTAACA CAGGGGATGGAGACTACCC AGCCTAGCAAACAGAGCAA CAACAAGTACATGGCCAGCT CTTATCTGACACTGACCGCT AGAGCATGGGAGCGACATA GCTCCTACAGTTGCCAGGTG ACACATGAAGGGCACACCG TTGAGAAGAGTCTGTCTAGG GCCGATTGCTCT |
| 57 | GYTFTSSY | Heavy chain variable region CDR1 of humanized antibody V0 (tgil005 V0-hIgG1/4 HCDR1) | 136 | GGGTATACTTTCACCAGCTC TTAT |
| 58 | IYAGTGDT | Heavy chain variable region CDR2 of humanized antibody V0 (tgi1005 V0-hIgG1/4 HCDR2) | 137 | ATCTACGCTGGCACTGGTGA CACT |
| 59 | ARHGSTGRGWYF AV | Heavy chain variable region CDR3 of humanized antibody V0 (tgil005 V0-hIgG1/4 HCDR3) | 138 | GCTCGACACGGTTCTACCGG TCGAGGTTGGTACTTTGCTG TG |
| 60 | TGAVTTSN | Light chain variable region CDR1 of humanized antibody V0 (tgi1005 V0-hIgG1/4 LCDR1) | 139 | ACAGGTGCCGTCACCACCA GCAAC |
| 61 | GTN | Light chain variable region CDR2 of humanized antibody V0 (tgi1005 V0-hIgG1/4 LCDR2) | 140 | GGCACCAAC |
| 62 | ALWYSNHFV | Light chain variable region CDR3 of humanized antibody V0 (tgi1005 V0-hIgG1/4 LCDR3) | 141 | GCACTGTGGTACAGCAATCA TTTCGTG |
| 63 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGSTGRGWYFAV WGQGTTVTVSS | Heavy chain variable region of humanized antibody V0 (tgi1005 V0-hIgG1/4 VH) | 142 | CAAGTGCAGTTGGTCCAATC AGGAGCTGAGGTCAAAAA CCAGGAGCTTCTGTGAAGGT CAGCTGTAAGGCTTCAGGGT ATACTTTCACCAGCTCTTATA TCTCTTGGGTAAGGCAAGCC CCTGGACAGGGACTGGAGT GGATCGCATGGATCTACGCT GGCACTGGTGACACTAACTA CAACCAGAAATTCACCGGG AGAGTCACCATGACAACCG ACACCTCAACAAGCACCGC ATATATGGAACTCCGTTCCTT GCGCAGCGACGATACCGCC GTGTATTATTGTGCTCGACAC GGTTCTACCGGTCGAGGTTG GTACTTTGCTGTGTGGGGTC AGGGAACCACAGTTACTGT GTCATCC |
| 64 | QAVVTQEPSLTVS PGGTVTLTCRSST GAVTTSNYANWV QQKPGQAFRGLIG GTNNRAPGVPAR FSGSLLGDKAALT LSGAQPEDEAEY YCALWYSNHFVF GGGTKLTVL | Light chain variable region of humanized antibody V0 (tgi1005 V0-hIgG1/4 VL) | 143 | CAGGCCGTAGTGACCCAAG AACCATCTCTCACCGTGTCC CCCGGCGGGACCGTAACAC TTACTTGCCGCAGTTCAACA GGTGCCGTCACCACCAGCA ACTACGCAAACTGGGTACAG CAGAAACCAGGTCAGGCTT TCCGAGGGCTTATTGGAGGC ACCAACAACAGGGCACCAG GTGTGCCAGCCCGCTTCTCT GGATCCCTTTTGGGCGATAA GGCCGCTCTGACACTGTCTG GTGCTCAGCCTGAGGATGAG GCTGAGTACTACTGCGCACT GTGGTACAGCAATCATTTCG TGTTTGGCGGCGGCACTAAA CTTACAGTGCTG |
| 65 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGSTGRGWYFAV WGQGTTVTVSSA STKGPSVFPLAPS SKSTSGGTAALGC LVKDYFPEPVTVS WNSGALTSGVHT FPAVLQSSGLYSLS SVVTVPSSSLGTQ TYICNVNHKPSNT KVDKKVEPKSCD KTHTCPPCPAPEL LGGPSVFLFPPKP KDTLMISRTPEVT CVVVDVSHEDPE VKFNWYVDGVE VHNAKTKPREEQ YNSTYRVVSVLTV LHQDWLNGKEYK CKVSNKALPAPIE KTISKAKGQPREP QVYTLPPSREEMT KNQVSLTCLVKGF YPSDIAVEWESNG QPENNYKTTPPVL DSDGSFFLYSKLT VDKSRWQQGNVF SCSVMHEALHNH YTQKSLSLSPGK | Heavy chain of humanized antibody V0 (tgil005 V0-hIgG1 H) | 144 | CAAGTGCAGTTGGTCCAATC AGGAGCTGAGGTCAAAAAA CCAGGAGCTTCTGTGAAGGT CAGCTGTAAGGCTTCAGGGT ATACTTTCACCAGCTCTTATA TCTCTTGGGTAAGGCAAGCC CCTGGACAGGGACTGGAGT GGATCGCATGGATCTACGCT GGCACTGGTGACACTAACTA CAACCAGAAATTCACCGGG AGAGTCACCATGACAACCG ACACCTCAACAAGCACCGC ATATATGGAACTCCGTTCCTT GCGCAGCGACGATACCGCC GTGTATTATTGTGCTCGACAC GGTTCTACCGGTCGAGGTTG GTACTTTGCTGTGTGGGGTC AGGGAACCACAGTTACTGT GTCATCCGCCTCCACTAAGG GACCTTCCGTATTTCCTCTTG CCCCTAGCAGTAAGTCAACT TCCGGAGGAACCGCAGCCC TGGGATGCCTTGTAAAGGAT TATTTTCCCGAACCCGTTACC GTGTCATGGAATAGCGGAGC TCTCACCTCTGGTGTGCACA CTTTCCCTGCAGTGCTCCAG AGCAGCGGACTGTATAGTCT GTCTAGTGTCGTAACAGTGC CTAGCTCTTCACTGGGCACC CAGACATACATCTGTAATGT GAACCACAAACCATCCAATA CCAAGGTGGACAAGAAGGT GGAGCCCAAGAGTTGCGAC AAGACACATACTTGTCCACC TTGTCCCGCTCCTGAACTGC TCGGAGGCCCCTCCGTCTTC CTGTTCCCCCCTAAGCCTAA GGACACCTTGATGATCTCCA GAACACCCGAAGTCACCTG TGTGGTGGTGGATGTGTCTC ATGAAGACCCAGAAGTGAA GTTTAACTGGTACGTGGATG GGGTCGAAGTCCATAATGCT AAAACTAAGCCCCGAGAGG AGCAATATAACTCCACCTAC CGTGTCGTGTCTGTTTTGAC AGTGCTGCATCAGGATTGGC TTAACGGTAAGGAGTATAAA TGCAAGGTGAGCAACAAAG CTCTGCCCGCACCCATCGAG AAGACAATATCTAAGGCAAA AGGGCAGCCACGGGAACCC CAGGTCTATACTCTCCCACC CTCTAGGGAGGAGATGACTA AGAACCAGGTGTCTCTGAC ATGTCTCGTAAAAGGCTTCT |
| | | | | ACCCTTCTGACATTGCCGTT GAATGGGAATCCAACGGCC AGCCTGAGAACAACTACAA GACAACCCCCCCCGTCCTGG ATAGTGACGGCAGTTTCTTC TTGTACAGCAAGCTGACTGT GGATAAGTCCCGATGGCAGC AGGGTAATGTATTCTCCTGC AGCGTCATGCATGAGGCACT TCATAACCACTATACACAGA AAAGCCTGTCACTGTCCCCC GGCAAA |
| 66 | QAVVTQEPSLTVS PGGTVTLTCRSST GAVTTSNYANWV QQKPGQAFRGLIG GTNNRAPGVPAR FSGSLLGDKAALT LSGAQPEDEAEY YCALWYSNHFVF GGGTKLTVLGQP KAAPSVTLFPPSS EELQANKATLVCL ISDFYPGAVTVAW KADSSPVKAGVE TTTPSKQSNNKYA ASSYLSLTPEQWK SHRSYSCQVTHEG STVEKTVAPTECS | Light chain of humanized antibody V0 (tgil005 V0-hIgG1 L) | 145 | CAGGCCGTAGTGACCCAAG AACCATCTCTCACCGTGTCC CCCGGCGGGACCGTAACAC TTACTTGCCGCAGTTCAACA GGTGCCGTCACCACCAGCA ACTACGCAAACTGGGTACAG CAGAAACCAGGTCAGGCTT TCCGAGGGCTTATTGGAGGC ACCAACAACAGGGCACCAG GTGTGCCAGCCCGCTTCTCT GGATCCCTTTTGGGCGATAA GGCCGCTCTGACACTGTCTG GTGCTCAGCCTGAGGATGAG GCTGAGTACTACTGCGCACT GTGGTACAGCAATCATTTCG TGTTTGGCGGCGGCACTAAA CTTACAGTGCTGGGCCAACC TAAAGCCGCTCCCTCCGTGA CCCTTTTCCCCCCTAGCAGT GAAGAATTGCAGGCAAATA AGGCCACATTGGTGTGCTTG ATCAGCGACTTTTACCCCGG AGCAGTCACTGTGGCATGGA AAGCTGATTCCTCCCCTGTC AAAGCAGGTGTTGAGACTA CCACCCCCTCTAAGCAGAGC AATAATAAGTACGCTGCCAG TAGCTACCTGTCTTTGACTC CCGAGCAGTGGAAATCTCAC AGATCCTATTCCTGCCAAGT TACTCACGAGGGAAGTACA GTGGAGAAAACTGTGGCTC CTACAGAGTGTAGC |
| 67 | QVQLVQSGAEVK KPGASVKVSCKA SGYTFTSSYISWV RQAPGQGLEWIA WIYAGTGDTNYN QKFTGRVTMTTD TSTSTAYMELRSL RSDDTAVYYCAR HGSTGRGWYFAV WGQGTTVTVSSA STKGPSVFPLAPC SRSTSESTAALGC | Heavy chain of humanized antibody V0 (tgil005 V0-hIgG4 H) | 146 | CAAGTGCAGTTGGTCCAATC AGGAGCTGAGGTCAAAAA CCAGGAGCTTCTGTGAAGGT CAGCTGTAAGGCTTCAGGGT ATACTTTCACCAGCTCTTATA TCTCTTGGGTAAGGCAAGCC CCTGGACAGGGACTGGAGT GGATCGCATGGATCTACGCT GGCACTGGTGACACTAACTA CAACCAGAAATTCACCGGG AGAGTCACCATGACAACCG ACACCTCAACAAGCACCGC |
| | LVKDYFPEPVTVS WNSGALTSGVHT FPAVLQSSGLYSLS SVVTVPSSSLGTK TYTCNVDHKPSN TKVDKRVESKYG PPCPPCPAPEFLGG PSVFLFPPKPKDT LMISRTPEVTCVV VDVSQEDPEVQF NWYVDGVEVHN AKTKPREEQFNST YRVVSVLTVLHQ DWLNGKEYKCK VSNKGLPSSIEKTI SKAKGQPREPQV YTLPPSQEEMTKN QVSLTCLVKGFYP SDIAVEWESNGQP ENNYKTTPPVLDS DGSFFLYSRLTVD KSRWQEGNVFSC SVMHEALHNHYT QKSLSLSLGK | | | ATATATGGAACTCCGTTCCTT GCGCAGCGACGATACCGCC GTGTATTATTGTGCTCGACAC GGTTCTACCGGTCGAGGTTG GTACTTTGCTGTGTGGGGTC AGGGAACCACAGTTACTGT GTCATCCGCTTCAACCAAAG GCCCTTCCGTATTCCCCCTC GCCCCATGCAGCAGAAGCA CCTCCGAAAGCACAGCTGCT TTGGGTTGCCTGGTCAAGGA CTACTTCCCAGAGCCCGTAA CCGTTTCTTGGAACTCCGGC GCCTTGACATCTGGCGTGCA CACCTTCCCTGCCGTCCTGC AAAGTAGCGGCCTGTACAGC TTGTCCTCTGTGGTTACCGT GCCATCCTCTAGCCTCGGCA CAAAGACCTACACTTGCAAT GTGGACCACAAACCCAGCA ACACAAAGGTTGACAAGAG GGTGGAGAGCAAATATGGCC CACCTTGCCCACCATGCCCA GCTCCAGAGTTTCTGGGGGG ACCTTCTGTATTTCTGTTCCC ACCAAAGCCTAAGGACACC CTGATGATTTCCAGGACCCC CGAGGTGACATGCGTGGTTG TGGATGTGTCCCAGGAAGAT CCTGAAGTGCAATTTAACTG GTACGTCGACGGGGTAGAG GTCCACAATGCTAAGACAAA GCCACGGGAGGAGCAGTTT AACAGTACATATCGCGTTGT GTCCGTGTTGACTGTGCTGC ATCAGGATTGGCTCAATGGG AAGGAGTATAAGTGCAAGGT CTCTAACAAAGGTCTGCCCT CCTCTATCGAGAAAACCATT AGCAAGGCAAAGGGTCAGC CTAGGGAACCCCAGGTTTAT ACCCTGCCTCCCTCTCAGGA AGAGATGACCAAGAACCAG GTGAGCCTTACTTGCCTGGT AAAAGGCTTCTATCCTTCAG ACATAGCTGTGGAGTGGGA ATCCAACGGTCAACCTGAGA ACAACTACAAAACCACTCCT CCAGTGCTGGATTCTGATGG ATCCTTTTTCCTGTACAGCC GGCTGACAGTGGATAAATCA AGGTGGCAGGAAGGGAACG TGTTCTCATGTAGCGTGATG CATGAAGCACTGCACAACC ACTATACCCAGAAGAGTCTT TCTCTGTCACTTGGCAAA |
| 68 | QAVVTQEPSLTVS | Light chain of humanized | 147 | CAGGCCGTAGTGACCCAAG |
| | PGGTVTLTCRSST GAVTTSNYANWV QQKPGQAFRGLIG GTNNRAPGVPAR FSGSLLGDKAALT LSGAQPEDEAEY YCALWYSNHFVF GGGTKLTVLGQP KAAPSVTLFPPSS EELQANKATLVCL ISDFYPGAVTVAW KADSSPVKAGVE TTTPSKQSNNKYA ASSYLSLTPEQWK SHRSYSCQVTHEG STVEKTVAPTECS | antibody V0 (tgi1005 V0-hIgG4 L) | | AACCATCTCTCACCGTGTCC CCCGGCGGGACCGTAACAC TTACTTGCCGCAGTTCAACA GGTGCCGTCACCACCAGCA ACTACGCAAACTGGGTACAG CAGAAACCAGGTCAGGCTT TCCGAGGGCTTATTGGAGGC ACCAACAACAGGGCACCAG GTGTGCCAGCCCGCTTCTCT GGATCCCTTTGGGCGATAA GGCCGCTCTGACACTGTCTG GTGCTCAGCCTGAGGATGAG GCTGAGTACTACTGCGCACT GTGGTACAGCAATCATTTCG TGTTTGGCGGCGGCACTAAA CTTACAGTGCTGGGCCAACC TAAAGCCGCTCCCTCCGTGA CCCTTTTCCCCCCTAGCAGT GAAGAATTGCAGGCAAATA AGGCCACATTGGTGTGCTTG ATCAGCGACTTTTACCCCGG AGCAGTCACTGTGGCATGGA AAGCTGATTCCTCCCCTGTC AAAGCAGGTGTTGAGACTA CCACCCCCTCTAAGCAGAGC AATAATAAGTACGCTGCCAG TAGCTACCTGTCTTTGACTC CCGAGCAGTGGAAATCTCAC AGATCCTATTCCTGCCAAGT TACTCACGAGGGAAGTACA GTGGAGAAAACTGTGGCTC CTACAGAGTGTAGC |
| 69 | QVQMQQSGAELV KPGASVKLSCKTS GFTFSSSYISWLK QKPGQSLEWIAWI YAGTGDTNYNQK FTGKAQVTVDTSS STAYMQFSSLTTE DSAIYYCVRHGST GRGWYFAVWGA GTTVTVSSASTKG PSVFPLAPSSKSTS GGTAALGCLVKD YFPEPVTVSWNSG ALTSGVHTFPAVL QSSGLYSLSSVVT VPSSSLGTQTYIC NVNHKPSNTKVD KKVEPKSCDKTH TCPPCPAPELLGG PSVFLFPPKPKDT LMISRTPEVTCVV VDVSHEDPEVKF NWYVDGVEVHN AKTKPREEQYNST YRVVSVLTVLHQ | Heavy chain of human-mouse chimeric antibody mutgi1005-hIgG1-H | 148 | CAAGTGCAGATGCAGCAGA GTGGTGCCGAATTGGTAAAA CCAGGGGCTTCTGTTAAGTT GTCCTGCAAGACCTCTGGGT TTACCTTCTCTTCTTCTTACA TCTCCTGGCTCAAACAGAAG CCAGGGCAGTCACTTGAGT GGATTGCCTGGATCTATGCT GGGACCGGAGATACAAACT ACAACCAGAAATTCACAGG CAAGGCCCAGGTGACCGTC GACACTTCTTCATCAACCGC CTATATGCAGTTCAGTTCCCT CACCACTGAGGATAGTGCCA TCTACTACTGCGTGCGGCAT GGAAGCACTGGTAGAGGTT GGTATTTCGCTGTCTGGGGT GCTGGTACAACTGTTACCGT GAGCTCCGCCTCCACTAAGG GACCTTCCGTATTTCCTCTTG CCCCTAGCAGTAAGTCAACT TCCGGAGGAACCGCAGCCC TGGGATGCCTTGTAAAGGAT TATTTTCCCGAACCCGTTACC GTGTCATGGAATAGCGGAGC |
| | DWLNGKEYKCK VSNKALPAPIEKTI SKAKGQPREPQV YTLPPSREEMTKN QVSLTCLVKGFYP SDIAVEWESNGQP ENNYKTTPPVLDS DGSFFLYSKLTVD KSRWQQGNVFSC SVMHEALHNHYT QKSLSLSPGK | | | TCTCACCTCTGGTGTGCACA CTTTCCCTGCAGTGCTCCAG AGCAGCGGACTGTATAGTCT GTCTAGTGTCGTAACAGTGC CTAGCTCTTCACTGGGCACC CAGACATACATCTGTAATGT GAACCACAAACCATCCAATA CCAAGGTGGACAAGAAGGT GGAGCCCAAGAGTTGCGAC AAGACACATACTTGTCCACC TTGTCCCGCTCCTGAACTGC TCGGAGGCCCCTCCGTCTTC CTGTTCCCCCCTAAGCCTAA GGACACCTTGATGATCTCCA GAACACCCGAAGTCACCTG TGTGGTGGTGGATGTGTCTC ATGAAGACCCAGAAGTGAA GTTTAACTGGTACGTGGATG GGGTCGAAGTCCATAATGCT AAAACTAAGCCCCGAGAGG AGCAATATAACTCCACCTAC CGTGTCGTGTCTGTTTTGAC AGTGCTGCATCAGGATTGGC TTAACGGTAAGGAGTATAAA TGCAAGGTGAGCAACAAAG CTCTGCCCGCACCCATCGAG AAGACAATATCTAAGGCAAA AGGGCAGCCACGGGAACCC CAGGTCTATACTCTCCCACC CTCTAGGGAGGAGATGACTA AGAACCAGGTGTCTCTGAC ATGTCTCGTAAAAGGCTTCT ACCCTTCTGACATTGCCGTT GAATGGGAATCCAACGGCC AGCCTGAGAACAACTACAA GACAACCCCCCCGTCCTGG ATAGTGACGGCAGTTTCTTC TTGTACAGCAAGCTGACTGT GGATAAGTCCCGATGGCAGC AGGGTAATGTATTCTCCTGC AGCGTCATGCATGAGGCACT TCATAACCACTATACACAGA AAAGCCTGTCACTGTCCCCC GGCAAA |
| 70 | QAVVTQESALTTS PGETVTLTCRSST GAVTTSNYANWV QEKPDHLFTGLIG GTNNRAPGVPAR FSGSLIGDKAALTI TGAQTEDEAIYFC ALWYSNHFVFGG GTKLTVLGQPKA APSVTLFPPSSEEL QANKATLVCLISD FYPGAVTVAWKA DSSPVKAGVETTT | Light chain of human-mouse chimeric antibody mutgi1005-hIgG1-L | 149 | CAAGCCGTTGTGACCCAAG AAAGTGCACTGACAACTTCC CCTGGAGAAACAGTTACACT GACTTGTAGGAGTAGCACCG GTGCTGTAACAACCTCCAAT TACGCCAACTGGGTGCAAG AGAAACCCGACCATCTGTTT ACAGGACTCATCGGCGGGA CTAATAATCGCGCTCCTGGA GTACCAGCTAGATTTTCCGG GTCATTGATTGGCGATAAGG CCGCCCTGACAATTACTGGG GCCCAAACTGAGGACGAAG |
| | PSKQSNNKYAASS YLSLTPEQWKSHR SYSCQVTHEGSTV EKTVAPTECS | | | CAATCTACTTCTGCGCCCTG TGGTATTCTAACCACTTCGT GTTTGGAGGGGGCACTAAG TTGACCGTGCTCGGCCAACC TAAAGCCGCTCCCTCCGTGA CCCTTTTCCCCCCTAGCAGT GAAGAATTGCAGGCAAATA AGGCCACATTGGTGTGCTTG ATCAGCGACTTTTACCCCGG AGCAGTCACTGTGGCATGGA AAGCTGATTCCTCCCCTGTC AAAGCAGGTGTTGAGACTA CCACCCCCTCTAAGCAGAGC AATAATAAGTACGCTGCCAG TAGCTACCTGTCTTTGACTC CCGAGCAGTGGAAATCTCAC AGATCCTATTCCTGCCAAGT TACTCACGAGGGAAGTACA GTGGAGAAAACTGTGGCTC CTACAGAGTGTAGC |
| 71 | QVQMQQSGAELV KPGASVKLSCKTS GFTFSSSYISWLK QKPGQSLEWIAWI YAGTGDTNYNQK FTGKAQVTVDTSS STAYMQFSSLTTE DSAIYYCVRHGST GRGWYFAVWGA GTTVTVSSASTKG PSVFPLAPCSRSTS ESTAALGCLVKDY FPEPVTVSWNSGA LTSGVHTFPAVLQ SSGLYSLSSVVTV PSSSLGTKTYTCN VDHKPSNTKVDK RVESKYGPPCPPC PAPEFLGGPSVFLF PPKPKDTLMISRT PEVTCVVVDVSQ EDPEVQFNWYVD GVEVHNAKTKPR EEQFNSTYRVVSV LTVLHQDWLNGK EYKCKVSNKGLP SSIEKTISKAKGQP REPQVYTLPPSQE EMTKNQVSLTCL VKGFYPSDIAVEW ESNGQPENNYKT TPPVLDSDGSFFL YSRLTVDKSRWQ EGNVFSCSVMHE ALHNHYTQKSLS LSLGK | Heavy chain of human-mouse chimeric antibody mutgi1005-hIgG4-H | 150 | CAAGTGCAGATGCAGCAGA GTGGTGCCGAATTGGTAAAA CCAGGGGCTTCTGTTAAGTT GTCCTGCAAGACCTCTGGGT TTACCTTCTCTTCTTCTTACA TCTCCTGGCTCAAACAGAAG CCAGGGCAGTCACTTGAGT GGATTGCCTGGATCTATGCT GGGACCGGAGATACAAACT ACAACCAGAAATTCACAGG CAAGGCCCAGGTGACCGTC GACACTTCTTCATCAACCGC CTATATGCAGTTCAGTTCCCT CACCACTGAGGATAGTGCCA TCTACTACTGCGTGCGGCAT GGAAGCACTGGTAGAGGTT GGTATTTCGCTGTCTGGGGT GCTGGTACAACTGTTACCGT GAGCTCCGCTTCAACCAAA GGCCCTTCCGTATTCCCCCT CGCCCCATGCAGCAGAAGC ACCTCCGAAAGCACAGCTG CTTTGGGTTGCCTGGTCAAG GACTACTTCCCAGAGCCCGT AACCGTTTCTTGGAACTCCG GCGCCTTGACATCTGGCGTG CACACCTTCCCTGCCGTCCT GCAAAGTAGCGGCCTGTACA GCTTGTCCTCTGTGGTTACC GTGCCATCCTCTAGCCTCGG CACAAAGACCTACACTTGCA ATGTGGACCACAAACCCAG CAACACAAAGGTTGACAAG AGGGTGGAGAGCAAATATG GCCCACCTTGCCCACCATGC CCAGCTCCAGAGTTTCTGGG GGGACCTTCTGTATTTCTGTT |
| | | | | CCCACCAAAGCCTAAGGAC ACCCTGATGATTTCCAGGAC CCCCGAGGTGACATGCGTGG TTGTGGATGTGTCCCAGGAA GATCCTGAAGTGCAATTTAA CTGGTACGTCGACGGGGTAG AGGTCCACAATGCTAAGACA AAGCCACGGGAGGAGCAGT TTAACAGTACATATCGCGTT GTGTCCGTGTTGACTGTGCT GCATCAGGATTGGCTCAATG GGAAGGAGTATAAGTGCAA GGTCTCTAACAAAGGTCTGC CCTCCTCTATCGAGAAAACC ATTAGCAAGGCAAAGGGTC AGCCTAGGGAACCCCAGGT TTATACCCTGCCTCCCTCTCA GGAAGAGATGACCAAGAAC CAGGTGAGCCTTACTTGCCT GGTAAAAGGCTTCTATCCTT CAGACATAGCTGTGGAGTGG GAATCCAACGGTCAACCTGA GAACAACTACAAAACCACT CCTCCAGTGCTGGATTCTGA TGGATCCTTTTTCCTGTACA GCCGGCTGACAGTGGATAA ATCAAGGTGGCAGGAAGGG AACGTGTTCTCATGTAGCGT GATGCATGAAGCACTGCACA ACCACTATACCCAGAAGAGT CTTTCTCTGTCACTTGGCAA A |
| 72 | QAVVTQESALTTS PGETVTLTCRSST GAVTTSNYANWV QEKPDHLFTGLIG GTNNRAPGVPAR FSGSLIGDKAALTI TGAQTEDEAIYFC ALWYSNHFVFGG GTKLTVLGQPKA APSVTLFPPSSEEL QANKATLVCLISD FYPGAVTVAWKA DSSPVKAGVETTT PSKQSNNKYAASS YLSLTPEQWKSHR SYSCQVTHEGSTV EKTVAPTECS | Light chain of human-mouse chimeric antibody mutgi1005-hIgG4-L | 151 | CAAGCCGTTGTGACCCAAG AAAGTGCACTGACAACTTCC CCTGGAGAAACAGTTACACT GACTTGTAGGAGTAGCACCG GTGCTGTAACAACCTCCAAT TACGCCAACTGGGTGCAAG AGAAACCCGACCATCTGTTT ACAGGACTCATCGGCGGGA CTAATAATCGCGCTCCTGGA GTACCAGCTAGATTTTCCGG GTCATTGATTGGCGATAAGG CCGCCCTGACAATTACTGGG GCCCAAACTGAGGACGAAG CAATCTACTTCTGCGCCCTG TGGTATTCTAACCACTTCGT GTTTGGAGGGGGCACTAAG TTGACCGTGCTCGGCCAACC TAAAGCCGCTCCCTCCGTGA CCCTTTTCCCCCCTAGCAGT GAAGAATTGCAGGCAAATA AGGCCACATTGGTGTGCTTG ATCAGCGACTTTTACCCCGG AGCAGTCACTGTGGCATGGA AAGCTGATTCCTCCCCTGTC AAAGCAGGTGTTGAGACTA |
| | | | | CCACCCCCTCTAAGCAGAGC AATAATAAGTACGCTGCCAG TAGCTACCTGTCTTTGACTC CCGAGCAGTGGAAATCTCAC AGATCCTATTCCTGCCAAGT TACTCACGAGGGAAGTACA GTGGAGAAAACTGTGGCTC CTACAGAGTGTAGC |
| 73 | MARAMAAAWPL LLVALLVLSWPPP GTGDVVVQAPTQ VPGFLGDSVTLPC YLQVPNMEVTHV SQLTWARHGESGS MAVFHQTQGPSY SESKRLEFVAARL GAELRNASLRMF GLRVEDEGNYTC LFVTFPQGSRSVD IWLRVLAKPQNTA EVQKVQLTGEPVP MARCVSTGGRPP AQITWHSDLGGM PNTSQVPGFLSGT VTVTSLWILVPSS QVDGKNVTCKVE HESFEKPQLLTVN LTVYYPPEVSISG YDNNWYLGQNE ATLTCDARSNPEP TGYNWSTTMGPL PPFAVAQGAQLLI RPVDKPINTTLICN VTNALGARQAEL TVQVKEGPPSEHS GISRNAIIFLVLGIL VFLILLGIGIYFYW SKCSREVLWHCH LCPSSTEHASASA NGHVSYSAVSRE NSSSQDPQTEGTR | Human CD155 protein (Human CD155) | | |
| 74 | MARTMAAAWPPL LLTLLELSWPPPG TGDIIVQAPTQVP GFLGDSVTLPCYL QVPGMEETHVSQ LTWSRHGESGSM AVFHQTQGPNYSE PKRLEFVAARLGT ELRDASLRMFGL RVEDEGNYTCLF VTFPQGSRSVDIW LRVLAKPQNTAE VQKVQLTGKPVP VARCVSTGGRPPA HITWHSDLGGMP NTSQAPGFLSGTV | Rhesus CD155 protein (Rhesus CD155) | | |
| | TVTSLWILVPSSQ VDGKSVTCKVEH ESFEKPQLLTVNL TVYYPPEVSISGY DNNWYLSQNEAT LTCDARSNPEPTG YNWSTTMGPLPP FAVAQGAQLLIRP VDKPINTTFICNV TNALGARQAELT VQVKEGPPSEPSG MSSNIIIFLILGIVIL LTLLGIGVYFYRS RCSREFLWCHHLS PSSEEHASASANG YISYSDVSREASS SQDPQTEGTR | | | |
| 75 | MARAMAAAWPL LLVALLVLSWPPP GTGDVVVQAPTQ VPGFLGDSVTLPC YLQVPNMEVTHV SQLTWARHGESGS MAVFHQTQGPSY SESKRLEFVAARL GAELRNASLRMF GLRVEDEGNYTC LFVTFPQGSRSVD IWLRVLAKPQNTA EVQKVQLTGEPVP MARCVSTGGRPP AQITWHSDLGGM PNTSQVPGFLSGT VTVTSLWILVPSS QVDGKNVTCKVE HESFEKPQLLTVN LTVYYPPEVSISG YDNNWYLGQNE ATLTCDARSNPEP TGYNWSTTMGPL PPFAVAQGAQLLI RPVDKPINTTLICN VTNALGARQAEL TVQVKEGPPSEHS GPKSCDKTHTCPP CPAPELLGGPSVF LFPPKPKDTLMIS RTPEVTCVVVDVS HEDPEVKFNWYV DGVEVHNAKTKP REEQYNSTYRVVS VLTVLHQDWLNG KEYKCKVSNKAL PAPIEKTISKAKGQ PREPQVYTLPPSR EEMTKNQVSLTC LVKGFYPSDIAVE | CD155-Fc | 152 | ATGGCCCGCGCTATGGCCGC TGCCTGGCCTCTCCTCCTCG TTGCCCTCCTGGTTTTGTCCT GGCCCCCACCAGGAACAGG GGACGTCGTGGTCCAAGCTC CCACTCAGGTACCTGGCTTT TTGGGCGATTCAGTGACCTT GCCCTGTTACCTCCAGGTGC CTAACATGGAGGTGACCCAC GTCTCCCAGCTTACCTGGGC AAGGCACGGCGAAAGCGGC AGTATGGCCGTGTTTCATCA AACCCAAGGCCCCAGCTATT CCGAGTCAAAAAGGTTGGA ATTTGTCGCTGCCCGACTTG GAGCAGAGCTTCGGAACGC CAGCCTGAGAATGTTCGGAC TGCGCGTCGAGGATGAGGG CAACTACACATGCTTGTTTG TGACTTTTCCACAGGGCTCT AGGTCCGTCGATATTTGGCT GAGAGTCTTGGCCAAGCCTC AGAACACTGCTGAAGTTCA AAAGGTCCAGCTCACTGGC GAACCCGTCCCAATGGCTAG ATGCGTGAGTACAGGCGGTA GGCCTCCTGCTCAGATCACC TGGCACAGCGATCTGGGTGG GATGCCTAACACATCCCAGG TGCCAGGATTCTTGTCTGGA ACCGTCACAGTGACCTCCCT GTGGATCCTGGTGCCTTCTA GCCAGGTGGATGGCAAAAA CGTTACCTGCAAGGTGGAGC ACGAGAGTTTCGAGAAGCC ACAGCTGCTGACAGTCAAC CTGACCGTCTATTATCCCCC GAGGTCAGCATCTCAGGTTA TGATAATAACTGGTATCTTGG CCAGAATGAAGCCACACTTA |
| | WESNGQPENNYK TTPPVLDSDGSFF LYSKLTVDKSRW QQGNVFSCSVMH EALHNHYTQKSL SLSPGK | | | CTTGCGATGCCAGAAGCAAC CCCGAGCCAACAGGATACA ACTGGTCTACTACCATGGGC CCACTGCCTCCATTCGCCGT GGCACAAGGCGCACAGCTG TTGATAAGACCCGTGGACAA GCCAATCAACACTACACTGA TCTGTAATGTGACCAACGCA CTGGGAGCTCGCCAGGCTG AGCTGACCGTCCAGGTAAA AGAGGGCCCACCCTCAGAA CATTCAGGACCTAAGTCTTG CGACAAGACCCACACTTGC CCCCCCTGTCCTGCTCCTGA GCTGTTGGGCGGTCCTTCCG TATTCCTCTTTCCCCCTAAGC CTAAGGATACCCTGATGATC TCTCGCACACCCGAGGTTAC CTGCGTGGTTGTCGACGTGA GTCATGAAGACCCCGAGGT GAAGTTTAATTGGTATGTGG ATGGAGTAGAGGTGCACAA CGCTAAAACCAAGCCCCGA GAAGAGCAGTATAACAGTAC TTACAGGGTCGTATCCGTCC TGACTGTACTGCATCAGGAT TGGCTGAATGGAAAGGAGT ATAAGTGCAAAGTGTCCAAT AAGGCCCTGCCCGCACCCAT CGAAAAAACCATTTCTAAGG CCAAAGGACAGCCAAGGGA GCCACAGGTGTACACTCTTC CTCCAAGTAGGGAGGAGAT GACCAAAAATCAGGTCTCCC TTACCTGTCTGGTGAAGGGC TTTTACCCTTCTGACATAGCA GTTGAGTGGGAAAGTAACG GACAACCAGAGAACAACTA CAAGACCACCCCACCTGTGC TGGACTCTGACGGAGCTTT TTCCTGTACTCAAAGCTGAC AGTTGACAAAAGTAGATGG CAGCAGGGGAATGTGTTTTC ATGCTCCGTGATGCACGAAG CCCTGCACAATCACTACACT CAGAAGTCACTGTCTCTGTC TCCTGGGAAG |
| 76 | METDTLLLWVLL LWVPGSTGEFMM TGTIETTGNISAEK GGSIILQCHLSSTT AQVTQVNWEQQ DQLLAICNADLG WHISPSFKDRVAP GPGLGLTLQSLTV NDTGEYFCIYHTY PDGTYTGRIFLEV | TIGIT-Fc | 153 | ATGGAAACAGACACCTTGCT TTTGTGGGTTCTCTTGTTGT GGGTGCCTGGTAGCACTGG GGAGTTTATGATGACCGGCA CAATAGAAACAACCGGCAAT ATTTCAGCTGAGAAAGGTGG GTCAATTATCCTGCAGTGCC ACCTTAGCAGTACAACAGCT CAAGTCACTCAGGTCAATTG GGAGCAACAGGATCAACTG |
| | LELEPKSCDKTHT CPPCPAPELLGGP SVFLFPPKPKDTL MISRTPEVTCVVV DVSHEDPEVKFN WYVDGVEVHNA KTKPREEQYNSTY RVVSVLTVLHQD WLNGKEYKCKVS NKALPAPIEKTISK AKGQPREPQVYT LPPSRDELTKNQV SLTCLVKGFYPSDI AVEWESNGQPEN NYKTTPPVLDSDG SFFLYSKLTVDKS RWQQGNVFSCSV MHEALHNHYTQK SLSLSPGK | | | CTCGCTATTTGTAACGCAGA CCTGGGCTGGCATATCAGCC CTAGTTTCAAGGACAGAGTA GCCCCAGGTCCAGGTTTGGG TCTGACACTGCAGAGTCTGA CCGTCAATGATACAGGCGAG TACTTTTGCATATATCATACC TATCCCGACGGAACTTACAC TGGCCGTATTTCTTGGAGG TTCTGGAACTTGAGCCCAAA AGCTGCGACAAGACCCACA CATGTCCTCCTTGTCCTGCC CCAGAACTGCTTGGTGGTCC AAGCGTCTTTCTGTTTCCAC CTAAGCCAAAGGATACTCTT ATGATTAGTAGAACCCCTGA GGTGACCTGTGTGGTCGTGG ATGTCAGTCACGAAGACCCA GAGGTGAAGTTCAACTGGT ATGTCGATGGGGTAGAGGTG CACAACGCTAAAACAAAAC CACGCGAAGAGCAGTACAA CAGCACATACCGAGTAGTAT CAGTTTTGACAGTGCTGCAC CAGGACTGGTTGAATGGCA AAGAGTATAAGTGCAAGGTC AGCAATAAGGCACTGCCCGC TCCCATCGAAAAGACCATAT CAAAAGCCAAAGGACAGCC TCGAGAGCCACAGGTTTACA CCCTGCCTCCTTCACGAGAT GAGCTGACCAAGAACCAAG TGTCCCTCACCTGCCTTGTG AAGGGCTTTTACCCATCTGA TATTGCTGTAGAGTGGGAAA GCAACGGGCAGCCTGAGAA CAACTACAAAACAACACCC CCAGTGCTCGATTCCGATGG TTCTTTTTTCTTGTATTCCAA GTTGACCGTGGACAAAAGC AGGTGGCAGCAGGGAAATG TGTTTAGCTGTTCCGTCATG CACGAGGCTCTCCATAACCA TTACACCCAGAAGTCCCTGT CCCTTAGCCCTGGTAAG |
| 77 | MEKKWKYCAVY YIIQIHFVKGVWE KTVNTEENVYATL GSDVNLTCQTQT VGFFVQMQWSKV TNKIDLIAVYHPQ YGFYCAYGRPCES LVTFTETPENGSK WTLHLRNMSCSV SGRYECmLVLYPE GIQTKIYNLLIQTH VTADEWNSNHTIE | CD96-Fc | 154 | ATGGAAAAGAAATGGAAAT ACTGCGCAGTTTATTATATCA TTCAGATCCACTTTGTGAAG GGTGTGTGGGAAAAGACTG TGAATACAGAAGAGAATGTG TACGCAACATTGGGCAGCGA CGTAAATCTGACTTGTCAGA CTCAGACTGTCGGTTTTTTC GTGCAGATGCAATGGAGTAA AGTAACAAACAAAATCGATC TCATTGCTGTGTATCACCCA CAGTATGGGTTTTACTGTGC |
| | IEINQTLEIPCFQN SSSKISSEFTYAWS VENSSTDSWVLLS KGIKEDNGTQETL ISQNHLISNSTLLK DRVKLGTDYRLH LSPVQIFDDGRKF SCHIRVGPNKILRS STPKSCDKTHTCP PCPAPELLGGPSV FLFPPKPKDTLMI SRTPEVTCVVVDV SHEDPEVKFNWY VDGVEVHNAKTK PREEQYNSTYRVV SVLTVLHQDWLN GKEYKCKVSNKA LPAPIEKTISKAKG QPREPQVYTLPPS REEMTKNQVSLT CLVKGFYPSDIAV EWESNGQPENNY KTTPPVLDSDGSF FLYSKLTVDKSRW QQGNVFSCSVMH EALHNHYTQKSL SLSPGK | | | TTATGGCCGGCCCTGCGAAA GCCTTGTTACCTTTACCGAA ACACCCGAAAATGGATCTAA ATGGACCTTGCACCTCCGTA ATATGTCCTGCAGTGTTTCA GGACGATACGAATGCATGCT GGTCTTGTACCCCGAAGGTA TTCAGACCAAAATCTACAAT CTCTTGATCCAAACTCATGT CACTGCTGACGAGTGGAATA GTAACCACACTATCGAGATC GAAATCAACCAGACCCTCG AGATTCCTTGTTTCCAGAAT AGCTCTTCTAAGATATCATCC GAGTTTACTTACGCCTGGTC AGTAGAGAATAGCTCAACAG ACAGTTGGGTGCTCTTGTCC AAGGGTATCAAGGAAGATAA CGGCACCCAGGAGACACTG ATCTCACAGAATCACCTGAT CAGCAACTCTACACTCCTGA AAGACCGTGTCAAGCTGGG CACCGACTACCGCTTGCATT TGAGCCCTGTCCAGATCTTC GACGATGGACGCAAGTTCA GCTGTCATATACGCGTTGGG CCTAACAAAATTCTTAGGAG CAGTACTCCTAAAAGCTGCG ATAAGACCCATACCTGTCCT CCATGCCCTGCACCAGAACT GTTGGGTGGTCCCAGTGTTT TCCTGTTCCCTCCAAAACCT AAGGATACCCTGATGATCTC AAGGACCCCAGAGGTAACT TGTGTCGTGGTGGATGTGAG TCATGAGGACCCAGAGGTC AAATTTAACTGGTATGTCGA CGGCGTGGAAGTTCACAAC GCTAAGACCAAGCCTAGAG AGGAACAGTACAACAGCAC CTACCGTGTTGTTTCTGTCCT TACTGTGCTCCATCAAGACT GGTTGAACGGTAAGGAATAC AAATGCAAGGTTAGCAACA AGGCTTTGCCAGCCCCTATC GAGAAAACCATTTCCAAAG CTAAAGGCCAACCTCGAGA GCCTCAGGTCTATACTCTGC CACCTTCTCGCGAAGAAATG ACCAAGAATCAGGTATCACT GACATGCCTCGTGAAGGGCT TCTATCCCTCAGACATCGCT GTAGAATGGGAGAGTAACG GCCAGCCAGAAAACAACTA TAAGACTACTCCCCCTGTGT TGGACAGCGACGGATCCTTC TTCTTGTACAGTAAGCTGAC |
| | | | | TGTGGACAAATCCCGATGGC AGCAGGGAAATGTTTTCAGC TGCAGTGTGATGCACGAGGC ACTTCATAACCACTATACTCA AAAGTCACTGTCACTGTCTC CAGGCAAA |
| 78 | MGWSCIILFLVAT ATGVHSEEVLWH TSVPFAENMSLEC VYPSMGILTQVE WFKIGTQQDSIAIF SPTHGMVIRKPYA ERVYFLNSTMAS NNMTLFFRNASE DDVGYYSCSLYT YPQGTWQKVIQV VQSDSFEAAVPSN SHIVSEPGKNVTL TCQPQMTWPVQA VRWEKIQPRQIDL LTYCNLVHGRNFT SKFPRQIVSNCSH GRWSVIVIPDVTV SDSGLYRCYLQAS AGENETFVMRLT VAEGKTDNQYTL FVAPKSCDKTHTC PPCPAPELLGGPS VFLFPPKPKDTLM ISRTPEVTCVVVD VSHEDPEVKFNW YVDGVEVHNAKT KPREEQYNSTYRV VSVLTVLHQDWL NGKEYKCKVSNK ALPAPIEKTISKAK GQPREPQVYTLPP SRDELTKNQVSLT CLVKGFYPSDIAV EWESNGQPENNY KTTPPVLDSDGSF FLYSKLTVDKSRW QQGNVFSCSVMH EALHNHYTQKSL SLSPGK | CD226-Fc | 155 | ATGGGCTGGTCTTGTATAATT CTTTTTCTCGTCGCTACTGCC ACTGGTGTTCACTCTGAGGA AGTGCTCTGGCATACATCCG TCCCATTCGCAGAAAATATG TCCCTGGAGTGCGTCTACCC CTCCATGGGGATTTTGACCC AGGTGGAGTGGTTCAAGATC GGTACCCAACAGGATTCTAT CGCCATCTTCAGCCCTACAC ATGGGATGGTGATCAGAAAA CCATACGCTGAGAGGGTTTA TTTTCTCAACTCCACTATGG CTTCTAACAATATGACCCTCT TCTTCAGGAATGCAAGCGAA GACGATGTGGGCTACTATTC TTGTTCCCTGTATACCTACCC ACAGGGCACTTGGCAGAAG GTCATACAGGTCGTTCAGAG TGACTCCTTTGAGGCCGCCG TGCCTAGTAATTCACACATA GTGTCTGAGCCTGGAAAAA ATGTGACCCTGACCTGTCAG CCACAGATGACCTGGCCTGT ACAGGCCGTGAGATGGGAG AAGATTCAACCCCGTCAAAT CGACTTGCTGACCTACTGCA ATCTGGTGCACGGCAGGAA CTTTACTTCCAAGTTTCCCA GGCAGATCGTTTCAAATTGC TCTCACGGGAGGTGGAGCG TAATCGTGATCCCAGATGTC ACAGTGAGTGATTCCGGTCT GTACCGATGCTACCTGCAGG CAAGTGCAGGAGAGAATGA GACCTTCGTGATGCGATTGA CTGTTGCAGAAGGCAAGAC CGATAACCAATACACCCTCT TTGTGGCTCCCAAGAGCTGT GATAAGACTCATACCTGTCC TCCTTGTCCCGCCCCAGAAC TCCTGGGTGGCCCTTCTGTG TTCCTTTTCCCACCTAAACC CAAGGACACACTTATGATCT CTAGGACCCCTGAGGTGAC ATGTGTAGTGGTGGATGTGA GTCATGAAGACCCAGAGGT CAAGTTTAACTGGTACGTAG ACGGTGTTGAGGTGCACAAT GCCAAAACTAAACCAAGGG AGGAACAGTACAACAGCAC |
| | | | | CTACCGGGTCGTGTCAGTTT TGACCGTACTTCACCAGGAC TGGCTGAATGGCAAAGAGT ATAAATGCAAAGTCAGCAAC AAAGCCCTGCCAGCCCCTAT CGAGAAAACTATTAGCAAGG CCAAAGGACAGCCCCGCGA GCCTCAAGTTTACACTCTGC CACCTTCTAGAGATGAGCTG ACTAAAAACCAGGTGTCTCT CACATGCTTGGTGAAAGGTT TCTATCCCAGCGACATAGCT GTGGAATGGGAGAGTAACG GGCAGCCCGAGAATAACTAC AAAACCACCCCCCCTGTCCT GGACTCAGACGGATCCTTCT TCCTGTACTCCAAGCTCACA GTGGATAAGAGTCGCTGGCA ACAGGGTAATGTTTTCAGCT GCTCCGTCATGCACGAGGCT CTGCATAATCACTACACACA GAAATCCCTGTCTTTGAGCC CCGGTAAA |
| 79 | QVQLVQSGAEVK KPGASVKVSCKA TGYTFSNYWIEW VRQAPGQGLEWI GEIFPGSGRINFNE KFKGRVTFTADTS ISTTYMELSRLRS DDTAVYYCARTKI YGNSFDYWGQGT LVTVSSASTKGPS VFPLAPSSKSTSG GTAALGCLVKDY FPEPVTVSWNSGA LTSGVHTFPAVLQ SSGLYSLSSVVTV PSSSLGTQTYICN VNHKPSNTKVDK KVEPKSCDKTHT CPPCPAPELLGGP SVFLFPPKPKDTL MISRTPEVTCVVV DVSHEDPEVKFN WYVDGVEVHNA KTKPREEQYNSTY RVVSVLTVLHQD WLNGKEYKCKVS NKALPAPIEKTISK AKGQPREPQVYT LPPSREEMTKNQV SLTCLVKGFYPSDI | Heavy chain of NB 1088-hIgG1 | 156 | CAGGTACAACTTGTTCAAAG TGGGGCCGAAGTGAAAAA CCAGGTGCATCCGTCAAGGT TTCTTGTAAGGCAACCGGTT ATACCTTTAGCAACTACTGG ATCGAGTGGGTCCGGCAAG CACCAGGTCAGGGACTTGA GTGGATAGGAGAAATATTCC CAGGAAGCGGCCGCATCAA CTTCAACGAAAAGTTCAAG GGCCGAGTGACTTTCACTGC TGACACATCCATTTCCACTA CTTATATGGAGCTTTCTCGTC TCCGGAGTGACGACACAGC CGTATACTACTGTGCCCGCA CCAAGATCTATGGCAACAGC TTCGACTATTGGGGACAGGG TACCTTGGTTACTGTGAGTT CTGCCTCCACTAAGGGACCT TCCGTATTTCCTCTTGCCCCT AGCAGTAAGTCAACTTCCGG AGGAACCGCAGCCCTGGGA TGCCTTGTAAAGGATTATTTT CCCGAACCCGTTACCGTGTC ATGGAATAGCGGAGCTCTCA CCTCTGGTGTGCACACTTTC CCTGCAGTGCTCCAGAGCA GCGGACTGTATAGTCTGTCT AGTGTCGTAACAGTGCCTAG CTCTTCACTGGGCACCCAGA |
| | AVEWESNGQPEN NYKTTPPVLDSDG SFFLYSKLTVDKS RWQQGNVFSCSV MHEALHNHYTQK SLSLSPGK | | | CATACATCTGTAATGTGAAC CACAAACCATCCAATACCAA GGTGGACAAGAAGGTGGAG CCCAAGAGTTGCGACAAGA CACATACTTGTCCACCTTGT CCCGCTCCTGAACTGCTCGG AGGCCCCTCCGTCTTCCTGT TCCCCCCTAAGCCTAAGGAC ACCTTGATGATCTCCAGAAC ACCCGAAGTCACCTGTGTGG TGGTGGATGTGTCTCATGAA GACCCAGAAGTGAAGTTTA ACTGGTACGTGGATGGGGTC GAAGTCCATAATGCTAAAAC TAAGCCCCGAGAGGAGCAA TATAACTCCACCTACCGTGT CGTGTCTGTTTTGACAGTGC TGCATCAGGATTGGCTTAAC GGTAAGGAGTATAAATGCAA GGTGAGCAACAAAGCTCTG CCCGCACCCATCGAGAAGA CAATATCTAAGGCAAAAGGG CAGCCACGGGAACCCCAGG TCTATACTCTCCCACCCTCTA GGGAGGAGATGACTAAGAA CCAGGTGTCTCTGACATGTC TCGTAAAAGGCTTCTACCCT TCTGACATTGCCGTTGAATG GGAATCCAACGGCCAGCCT GAGAACAACTACAAGACAA CCCCCCCCGTCCTGGATAGT GACGGCAGTTTCTTCTTGTA CAGCAAGCTGACTGTGGATA AGTCCCGATGGCAGCAGGG TAATGTATTCTCCTGCAGCGT CATGCATGAGGCACTTCATA ACCACTATACACAGAAAAGC CTGTCACTGTCCCCCGGCAA A |
| 80 | QVQLVQSGAEVK KPGASVKVSCKA TGYTFSNYWIEW VRQAPGQGLEWI GEIFPGSGRINFNE KFKGRVTFTADTS ISTTYMELSRLRS DDTAVYYCARTKI YGNSFDYWGQGT LVTVSSASTKGPS VFPLAPCSRSTSES TAALGCLVKDYFP EPVTVSWNSGAL TSGVHTFPAVLQS SGLYSLSSVVTVP SSSLGTKTYTCNV DHKPSNTKVDKR VESKYGPPCPPCP | Heavy chain of NB 1088-hIgG4 | 157 | CAGGTACAACTTGTTCAAAG TGGGGCCGAAGTGAAAAA CCAGGTGCATCCGTCAAGGT TTCTTGTAAGGCAACCGGTT ATACCTTTAGCAACTACTGG ATCGAGTGGGTCCGGCAAG CACCAGGTCAGGGACTTGA GTGGATAGGAGAAATATTCC CAGGAAGCGGCCGCATCAA CTTCAACGAAAAGTTCAAG GGCCGAGTGACTTTCACTGC TGACACATCCATTTCCACTA CTTATATGGAGCTTTCTCGTC TCCGGAGTGACGACACAGC CGTATACTACTGTGCCCGCA CCAAGATCTATGGCAACAGC TTCGACTATTGGGGACAGGG TACCTTGGTTACTGTGAGTT |
| | APEFLGGPSVFLF PPKPKDTLMISRT PEVTCVVVDVSQ EDPEVQFNWYVD GVEVHNAKTKPR EEQFNSTYRVVSV LTVLHQDWLNGK EYKCKVSNKGLP SSIEKTISKAKGQP REPQVYTLPPSQE EMTKNQVSLTCL VKGFYPSDIAVEW ESNGQPENNYKT TPPVLDSDGSFFL YSRLTVDKSRWQ EGNVFSCSVMHE ALHNHYTQKSLS LSLGK | | | CTGCTTCAACCAAAGGCCCT TCCGTATTCCCCCTCGCCCC ATGCAGCAGAAGCACCTCC GAAAGCACAGCTGCTTTGG GTTGCCTGGTCAAGGACTAC TTCCCAGAGCCCGTAACCGT TTCTTGGAACTCCGGCGCCT TGACATCTGGCGTGCACACC TTCCCTGCCGTCCTGCAAAG TAGCGGCCTGTACAGCTTGT CCTCTGTGGTTACCGTGCCA TCCTCTAGCCTCGGCACAAA GACCTACACTTGCAATGTGG ACCACAAACCCAGCAACAC AAAGGTTGACAAGAGGGTG GAGAGCAAATATGGCCCACC TTGCCCACCATGCCCAGCTC CAGAGTTTCTGGGGGGACCT TCTGTATTTCTGTTCCCACCA AAGCCTAAGGACACCCTGAT GATTTCCAGGACCCCCGAGG TGACATGCGTGGTTGTGGAT GTGTCCCAGGAAGATCCTGA AGTGCAATTTAACTGGTACG TCGACGGGGTAGAGGTCCA CAATGCTAAGACAAAGCCA CGGGAGGAGCAGTTTAACA GTACATATCGCGTTGTGTCC GTGTTGACTGTGCTGCATCA GGATTGGCTCAATGGGAAGG AGTATAAGTGCAAGGTCTCT AACAAAGGTCTGCCCTCCTC TATCGAGAAAACCATTAGCA AGGCAAAGGGTCAGCCTAG GGAACCCCAGGTTTATACCC TGCCTCCCTCTCAGGAAGAG ATGACCAAGAACCAGGTGA GCCTTACTTGCCTGGTAAAA GGCTTCTATCCTTCAGACATA GCTGTGGAGTGGGAATCCA ACGGTCAACCTGAGAACAA CTACAAAACCACTCCTCCAG TGCTGGATTCTGATGGATCC TTTTTCCTGTACAGCCGGCT GACAGTGGATAAATCAAGGT GGCAGGAAGGGAACGTGTT CTCATGTAGCGTGATGCATG AAGCACTGCACAACCACTAT ACCCAGAAGAGTCTTTCTCT GTCACTTGGCAAA |
| 81 | DIQMTQSPSSLSA SVGDRVTITCKAS QDVGTAVVWYQ QKPGKAPKLLIY WASSRHEGVPDR FSGSGSGTDFTLTI SSLQPEDFADYFC | Light chain of NB 1088-hK | 158 | GATATCCAGATGACCCAGAG CCCCTCTTCCCTCTCCGCAA GCGTTGGTGACAGGGTGAC CATTACCTGCAAGGCCAGTC AAGATGTTGGAACAGCAGTT GTGTGGTATCAGCAGAAGCC CGGGAAGGCACCTAAACTG |
| | QQYSRYPLTFGQG TKLEIKRTVAAPS VFIFPPSDEQLKSG TASVVCLLNNFYP REAKVQWKVDN ALQSGNSQESVTE QDSKDSTYSLSST LTLSKADYEKHK VYACEVTHQGLSS PVTKSFNRGEC | | | CTGATCTATTGGGCCTCAAG TCGGCATGAAGGCGTTCCAG ATAGGTTCAGTGGATCAGGT AGCGGTACAGACTTCACCCT CACTATCAGCTCCCTGCAGC CTGAAGACTTTGCCGACTAC TTCTGTCAGCAGTACTCCAG ATATCCTCTTACCTTTGGACA AGGGACCAAACTCGAGATA AAAAGAACCGTGGCCGCTC CTTCTGTGTTCATATTTCCTC CATCCGATGAGCAGCTTAAG TCTGGAACTGCTTCTGTGGT CTGTCTGCTCAACAACTTCT ACCCTAGAGAGGCCAAAGT GCAATGGAAAGTGGACAAT GCTCTGCAATCAGGAAACTC TCAAGAGAGCGTAACAGAG CAAGATTCTAAAGACTCCAC CTATAGTCTTTCCAGCACAC TGACCCTTAGTAAGGCCGAC TACGAAAAACATAAGGTGTA TGCCTGCGAGGTGACTCATC AAGGGCTGTCCAGTCCAGT GACCAAAAGCTTTAATAGGG GGGAATGT |

Hereinafter, the present disclosure will be described in detail by way of examples. In the examples or test examples, the experimental methods without specifying the specific conditions were carried out according to conventional conditions.

Hereinafter, the scheme of the present disclosure will be explained in conjunction with examples. Those skilled in the art will understand that the following examples are only used to illustrate the present disclosure and should not be considered as limiting the scope of the present disclosure. Where specific techniques or conditions are not specified in the examples, they are performed according to techniques or conditions described in the literature in the art or according to the product specification. The reagents or instruments used without specifying the manufacturer are all conventional products that can be obtained commercially.

### Example 1: Preparation of antibody

This example was used to obtain a murine monoclonal antibody against human CD155, in which the purified recombinant CD155 extracellular region Fc fusion protein (CD155-Fc) (a fusion protein of recombinant CD155 extracellular region and hIgG1-Fc, with amino acid sequence as set forth in SEQ ID NO. 75) was used as an antigen to immunize the Balb/c mice (9-week-old, purchased from Shanghai SLAC, weighing about 20 g).

Mice were immunized three times with purified antigen and a complete Freund's adjuvant, and immune responses were detected after blood collection via the tail vein. The sera were screened by ELISA and flow cytometry using conventional operating procedures to obtain mice with anti-human CD155 immunoglobulin. Splenocytes from mice with the highest anti-CD155 immunoglobulin were taken out and fused with murine myeloma SP2/0 cells (ATCC NO. CRL-1581). The fused hybridoma cells were subjected to antibody screening to obtain murine mAbs. The more detailed experimental steps are as follows.

The candidate hybridoma cells were cultured to a total number of 10⁶. The cells were collected by centrifugation at 800 rpm for 10 min, and the total RNA of the hybridoma cells was extracted with a Trizol kit (Invitrogen). The total RNA was used as a template to synthesize the cDNA library (Invitrogen) by reverse transcription, and the cDNA was used as a template to amplify the corresponding nucleic acid sequence of the variable region in hybridoma cells by PCR. The primer sequences used in the PCR amplification reaction were complementary to the first framework region of the antibody variable region or the signal peptide region and the constant region (Larrick, J.W., et al., (1990) Scand. J. Immunol., 32, 121-128 and Coloma, J.J. et al., (1991) BioTechniques, 11, 152-156). The reaction system and process of PCR amplification are as follows. In a 50 µL reaction system, 2 µL of cDNA, 5 µL of 10×PCR buffer, 2 µL of upstream and downstream primers (5 µmol), 2 µL of dNTP, 1 µL of Taq enzyme (Takara, Ex Taq), and 38 µL of H₂O were added respectively. A pre-denaturation was performed for 5 min at 95°C, and the procedure was entered into the temperature cycle for PCR amplification. The reaction conditions included: denaturation at 94°C for 30 s, annealing at 58°C for 45 s, extension at 72°C for 50 s for a total of 32 cycles, then extension at 72°C for 7 min. After sequencing the amplified products, the variable region sequences of the heavy chain and the light chain (including amino acid sequences and nucleic acid sequences) of murine mAb were obtained (the amino acid sequences were as set forth in SEQ ID NO: 53 to 54).

### Example 2: Detection of binding activity of murine CD155 antibody

The flow cytometry assay was used to detect the binding characteristics of CD155 antibody. Human CD155 protein (CHO-K1-human CD155) and monkey CD155 protein (CHO-K1-Rhesus CD155) were overexpressed in CHO-K1 cells (ATCC number). The intensity of the signal after the addition of the murine antibody obtained in Example 1 was detected to determine the binding characteristics of the antibody and CD155. The specific experimental operation was as follows.

HEK293T cells were plated in a six-well plate at 5×10⁵ cells/well and cultured overnight in a DMEM medium without double antibiotics. The medium was discarded before transfection and 1 mL of fresh DMEM medium without double antibiotics was added. pLVX-EF1a-CD155-IRES-puro (inserted with the nucleotide sequence SEQ ID NO: 45 encoding human CD155 protein) or pLVX-EF1a-cynomolgus CD155-IRES-puro (inserted with the nucleotide sequence SEQ ID NO: 46 encoding the Rhesus CD155 protein) were added to 200 µL serum-free DMEM medium with pMD2G and psPAX2 vectors (3 µg in total) according to a ratio of 2: 1: 1, and then 12 µg of polyetherimide (PEI, Polysciences Co., Ltd.) was added, wherein the restriction sites of pLVX-EF1a-IRES-puro vector were between EcoRI and BamHI. After mixing well, the mixture was stood for 16 min. Then the whole liquid was added to the above six-well plate plated with HEK293T cells. After 6 h of culture, the medium was discarded and a fresh complete DMEM medium was added for culture. 48 h after transfection, the cell culture supernatant was collected and filtered through a 0.45 µm filter (Millipore) to obtain the virus supernatant. The obtained different virus supernatants were added to a 6-well plate containing 1×10⁴ CHO-K1 cells, and polybrene (Sigma) at a final concentration of 4 µg/mL was added and cultured for 12 h. The supernatant was then discarded and a fresh complete DMEM medium was added. The obtained cells were CHO-K1-CD155 cells or CHO-K1-Rhesus CD155 cells.

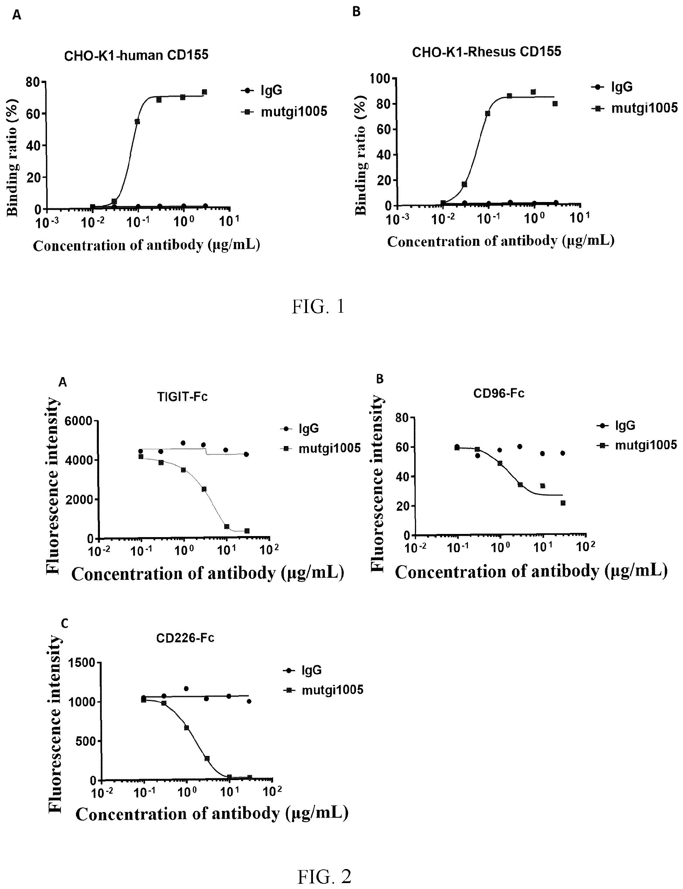

The CHO-K1-human CD155 or CHO-K1-Rhesus CD155 cells were diluted to 2×10⁶ cells/mL with PBS and added to a 1.5 mL EP tube at a volume of 100 µL/tube, to which 10 µL/tube of goat serum (purchased from Shanghai Biotech) was added and blocked at 4°C for 30 min. CD155 antibodies at different concentrations were added and incubated at 4°C for 30 min. 1 mL of PBS was added to the EP tub, and the mixture was centrifuged at 4°C, 3500 rpm for 5 min. The supernatant was discarded, and the precipitate was washed again with PBS. After centrifugation, the supernatant was discarded, and the cells were resuspended with 100 µL/tube PBS. 0.1 µL/tube Alexa-647-labeled goat anti-mouse secondary antibody (Biolegend) was added and incubated at 4°C in the dark for 30 min. The mixture was washed twice with PBS, and the supernatant was discarded after centrifugation. The cells were resuspended with 200 µL/tube PBS and detected by flow cytometry, wherein IgG (purchased from Biolegend) was used as a control. FIG. 1 shows that the murine antibody (mutgi1005) of the present disclosure can bind to human and monkey CD155 proteins.

### Example 3: Detection of blocking activity of murine CD155 antibody

The CD155 antibody blocks the signaling pathway between CD155 and its receptors TIGIT, CD96, and CD226 by binding to the extracellular region of CD155. The flow cytometry assay was used to detect the blocking effect of CD155 antibody on the binding of CD155 to its receptors. The specific experimental procedures were as follows.

The CHO-K1-human CD155 cells obtained in Example 2 (same as above) were diluted to 2×10⁶/mL with PBS and added to a 1.5 mL EP tube at a volume of 100 µL/tube, to which 10 µL/tube of mouse serum was added, and blocked at 4°C for 30 min. After blocking, CD155 antibodies at different concentrations were added and incubated at 4°C for 30 min; and then 2 µg/tube of TIGIT extracellular region Fc fusion protein (TIGIT-Fc, SEQ ID NO: 76, expressed in this laboratory), 4 µg/tube of CD96 extracellular region Fc fusion protein (CD96-Fc, SEQ ID NO: 77, expressed in this laboratory), and 2 µg/tube of CD226 extracellular region Fc fusion protein (CD226-Fc, SEQ ID NO: 78, expressed in this laboratory) were added respectively, and incubated at 4°C for 30 min. 1 mL of PBS was added to the above EP tube and centrifuged at 4°C, 3500 rpm for 5 min. The supernatant was discarded, and the precipitate was washed again with PBS. After centrifugation, the supernatant was discarded, and the cells were resuspended with 100 µL/tube PBS. 1 µL/tube 647-labeled mouse anti-human secondary antibody (HP6017, Biolegend) was added thereto and incubated at 4°C in the dark for 30 min. The mixture was washed twice with PBS, and the supernatant was discarded after centrifugation. The cells were resuspended with 200 µL/tube PBS and detected by flow cytometry, wherein IgG (purchased from Biolegend) was used as a control antibody. The results were shown in FIG. 2, and it can be seen that the murine CD155 antibody (mutgi1005) of the present disclosure can block the binding of CD155 to TIGIT, CD96, and CD226.

### Example 4: Detection assay of blocking activity of murine CD155 antibody

ELISA assay was used to detect the effect of antibodies on the binding of CD155 to its receptors TIGIT, CD96, and CD226. The specific experiments were as follows.

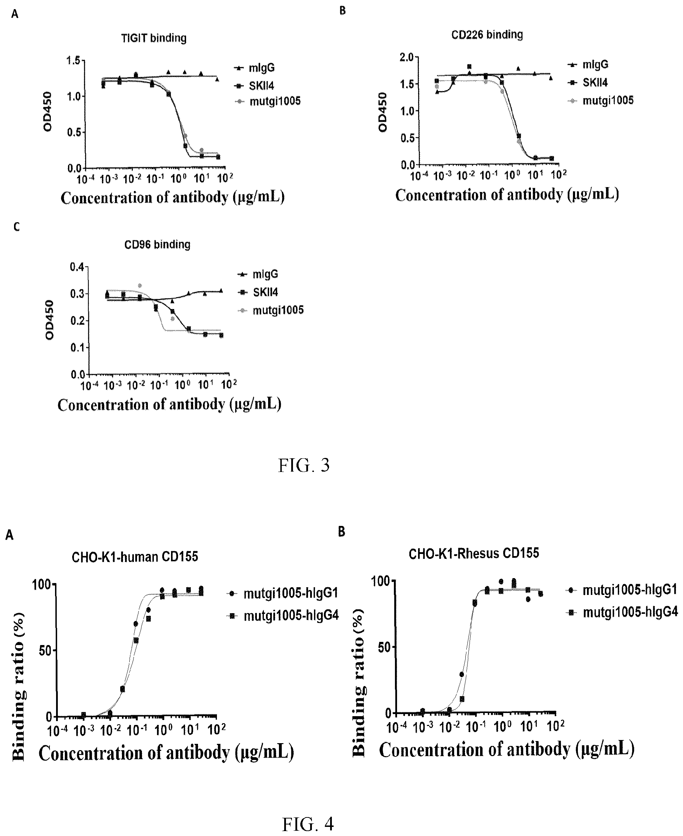

CD155-His tag fusion protein (purchased from Acro) was diluted to 5 µg/mL with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer. Then 200 µL/well PBS/10% BSA was added and incubated at 37°C for 2 h for blocking. The blocking solution was removed, and the plate was washed 6 times with PBST. Then murine CD155 antibody to be tested diluted to an appropriate concentration with PBST/0.05% BSA, 5 µg/mL TIGIT-Avi tag (purchased from Acro), 3.2 µg/mL CD96-Avi tag (purchased from Acro), or 10 µg/mL CD226-Avi tag (purchased from Acro) were added, totaling 100 µL/well, and incubated at 37°C for 1 h. The reaction system was removed, and the plate was washed 6 times with PBST. HRP (horseradish peroxidase)-labeled Strreptavidin secondary antibody (purchased from Southern biotech) was diluted with PBST/0.05% BSA at 100 µL/well and incubated at 37°C for 1 h. After washing the plate 6 times with PBST, 80 µL/well TMB (tetramethylbenzidine) was added and incubated at room temperature for 3 min. 80 µL/well 4 M sulfuric acid was added to terminate the reaction. The absorbance was read at 450 mm using a microplate reader with mIgG (purchased from Biolegend) and SKII4 (purchased from Biolegend) as controls. The results were shown in FIG. 3 and indicated that the murine CD155 antibody (Mutgi1005) of the present disclosure can block the binding of CD155 to its receptors TIGIT, CD96, and CD226.

### Example 5: Flow cytometry binding assay of chimeric CD155 antibody

The CHO-K1-human CD155 and CHO-K1-Rhesus CD155 cells obtained in Example 2 were diluted to 2×10⁶/mL with PBS and added to 1.5 mL EP tube at a volume of 100 µL/tube, to which 10 µL/tube of mouse serum was added and blocked at 4°C for 30 min. The mutgi1005-hIgG1 or mutgi1005-hIgG4 CD155 chimeric antibodies at different concentrations (fused by the light chain variable region and the heavy chain variable region of the murine antibody obtained in Example 1 with humanized wild-type IgG1 or IgG4, as set forth in SEQ ID NO: 69 to 72) were added and incubated at 4°C for 30 min. 1 mL of PBS was added to the EP tub, and the mixture was centrifuged at 4°C, 3500 rpm for 5 min. The supernatant was discarded, and the precipitate was washed again with PBS. After centrifugation, the supernatant was discarded, and the cells were resuspended with 100 µL/tube PBS. 1 µL/tube Alexa-647-labeled mouse anti-human secondary antibody (HP0617, Biolegend) was added and incubated at 4°C in the dark for 30 min. The mixture was washed twice with PBS, and the supernatant was discarded after centrifugation. The cells were resuspended with 200 µL/tube PBS and detected by flow cytometry. The results were shown in FIG. 4 and further show that the chimeric antibody of the present disclosure can bind to human or monkey CD155 protein.

### Example 6: Antibody humanization and affinity maturation assay

Referring to the heavy chain variable region sequence (as set forth in SEQ ID NO: 53) and the light chain variable region sequence (as set forth in SEQ ID NO: 54) of the CD155 antibody obtained in Example 1, MOE software was used to design and select the humanized template that best matched its non-CDR region. The CDR region of the murine antibody was transplanted onto the selected humanized template to replace the CDR region of the human template to obtain a humanized antibody. Then, based on the three-dimensional structure of the murine antibody, the buried residues, the residues that directly interact with the CDR region, and the residues in the FR region that have an important influence on the conformation of VL and VH were back mutated to obtain the humanized antibody. The sequence of the heavy chain variable region of the humanized CD155 antibody (tgi1005 V0-hIgG1/4) was as set forth in SEQ ID NO: 63, and the sequence of the light chain variable region thereof was as set forth in SEQ ID NO: 64.

Affinity-matured antibodies were obtained by random mutation of the CDR region of the CD155 antibody. The sequence of the heavy chain variable region of the affinity-matured CD155 antibody was as set forth in SEQ ID NO: 25, and the sequence of the light chain variable region thereof was as set forth in SEQ ID NO: 26; or the sequence of the heavy chain variable region of the affinity-matured CD155 antibody was as set forth in SEQ ID NO: 27, and the sequence of the light chain variable region thereof was as set forth in SEQ ID NO: 28; or the sequence of the heavy chain variable region of the affinity-matured CD155 antibody was as set forth in SEQ ID NO: 29, and the sequence of the light chain variable region thereof was as set forth in SEQ ID NO: 30; or the sequence of the heavy chain variable region of the affinity-matured CD155 antibody was as set forth in SEQ ID NO: 31, and the sequence of the light chain variable region thereof was as set forth in SEQ ID NO: 32. The specific sequences were shown in Table 1 or 2.

**[Table 2]**

| Item | HCDR1 | HCDR2 | HCDR3 | LCDR1 | LCDR2 | LCDR3 |
|---|---|---|---|---|---|---|
| Murine | GFTFSSSY | IYAGTGDT | VRHGSTGRGWYFAV | TGAVTTSN | GTN | ALWYSN HFV |
| Humaniz ed V0 | GYTFTSSY | IYAGTGDT | ARHGSTGRGWYFAV | TGAVTTSN | GTN | ALWYSN HFV |
| Affinity-matured humaniz ed V20 | GYTFTSSY | IYAGTGDT | ARHGYTGRGWYFAV | TGAVTTSN | HTN | ALWYSN KFV |
| Affinity-matured humaniz ed V21 | GYTFTSSY | IYAGTGDT | ARHGYTGRGWYFAV | TGAVTTSN | GTN | ILWYSN HFV |
| Affinity-matured humaniz ed V28 | GYTFTSSY | IYAGTGDT | ARHGLTGRHWYFAV | TGAVTTSN | GTN | ILWYSN KFV |
| Affinity-matured humaniz ed V28 | GYTFTSSY | IYAGTGDT | ARHGMTGRGWYFAV | TGAVTTSN | HTN | ILWYSN KFV |

### Example 7: Detection assay of binding activity of affinity-matured humanized CD155 antibody

ELISA assay was used to detect the binding characteristics of the affinity-matured humanized CD155 antibody obtained in Example 6. CD155-His tag fusion protein (purchased from Acro) and Rhesus CD155-His tag fusion protein (purchased from Acro) were coated into 96-well plates. After the addition of antibodies, the intensity of the detected signal was used to determine the binding characteristics of the antibodies to human and monkey CD155.

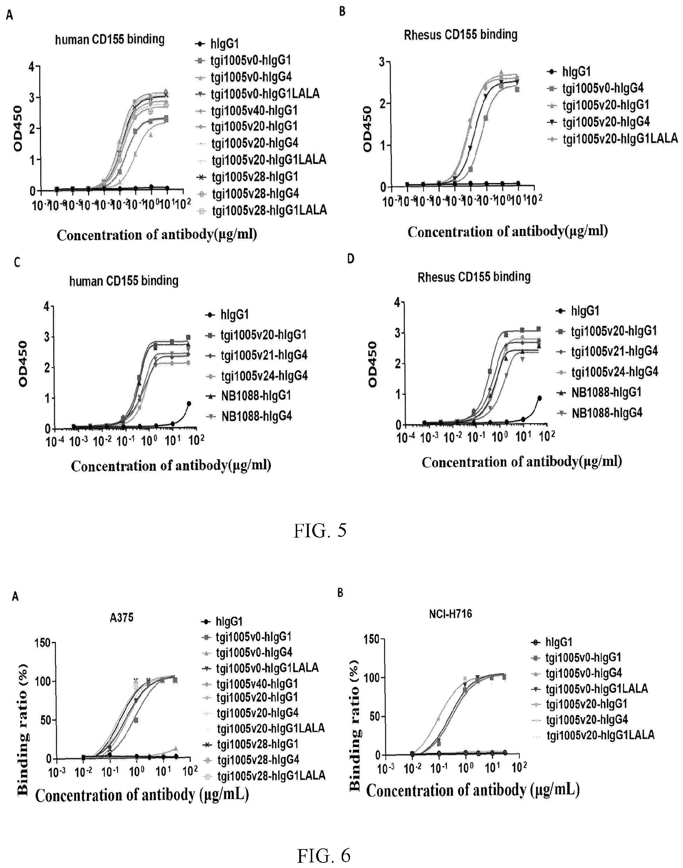

CD155-His tag fusion protein and Rhesus CD155-His tag fusion protein were diluted to 2 µg/mL with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer, and then 200 µL/well of PBS/10% BSA was added and incubated at 37°C for 2 h for blocking. After removing the blocking solution, the plate was washed 6 times with PBST, and 100 µL/well of affinity-matured humanized CD155 antibody to be tested diluted to an appropriate concentration with PBST/0.05% BSA was added and incubated at 37°C for 1 hour. The reaction system was removed, and the plate was washed 6 times with PBST, and then HRP (horseradish peroxidase)-labeled mouse anti-human IgG (Fab-specific) secondary antibody (Sigma) was diluted in PBST/0.05% BSA at 100 µL/well and incubated at 37°C for 1 h. After washing the plate six times with PBST, 80 µL/well TMB (tetramethylbenzidine) was added and incubated at room temperature for 3 min. The reaction was terminated by adding 80 µL/well 4 M sulfuric acid, wherein hIgG1 (purchased from Biolegend, and patented antibodies NB1088-hIgG1 (SEQ ID NO: 79 and 81) and NB1088-hIgG4 (SEQ ID NO: 80 and 81) expressed in our laboratory) was used as the control antibody. The absorbance was read at 450 nm using a microplate reader. The results were shown in FIG. 5, where LALA refers to the L234A and L235A mutations in the hIgG1 constant region. The affinity-matured humanized CD155 antibody of the present disclosure can bind to human and monkey CD155, and the binding activity of the affinity-matured antibody is higher than that of the antibody that has not been affinity-matured.

### Example 8: Detection assay of blocking activity of affinity-matured humanized CD155 antibody

ELISA assay was used to detect the effect of the affinity-matured humanized CD155 antibody obtained in Example 6 on the binding of CD155 to its receptor.

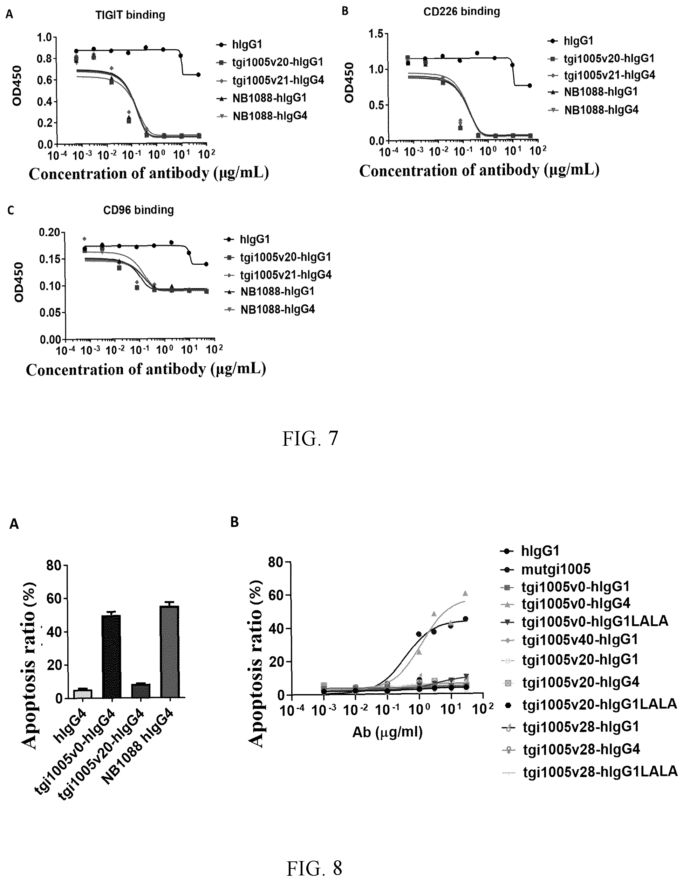

CD155-His tag fusion protein (purchased from Acro) was diluted to 5 µg/mL with PBS buffer, added to a 96-well plate at a volume of 100 µL/well, and placed at 4°C overnight. The PBS buffer in the 96-well plate was aspirated, and the plate was washed 6 times with PBST (pH 7.2 PBS containing 0.1% Tween 20) buffer, and then 200 µL/well PBS/10% BSA was added and incubated at 37°C for 2 h for blocking. The blocking solution was removed, and the plate was washed 6 times with PBST. Then, 100 µL/well of affinity-matured humanized CD155 antibody to be tested diluted to an appropriate concentration with PBST/0.05% BSA was added. 5 µg/mL TIGIT-Avi tag (purchased from Acro), 3.2 µg/mL CD96-Avi tag (purchased from Acro), and 10 µg/mL CD226-Avi tag (purchased from Acro) were added to the above antibodies, and the mixture was incubated at 37°C for 1 h. After the incubation, the reaction system was removed, the plate was washed 6 times with PBST, and then HRP (horseradish peroxidase)-labeled Avidin secondary antibody was diluted with PBST/0.05% BSA at 100 µL/well and incubated at 37°C for 1 h. After washing the plate 6 times with PBST, 80 µL/well TMB (tetramethylbenzidine) was added and incubated at room temperature for 3 min. 80 µL/well 4 M sulfuric acid was added to terminate the reaction. The absorbance was read at 450 nm using a microplate reader. The results were shown in FIG. 7, and the affinity-matured humanized CD155 antibody of the present disclosure can block the binding of CD155 to TIGIT, CD96, and CD226.

### Example 9: Flow cytometry binding assay of affinity-matured humanized CD155 antibody

The CHO-K1-human CD155 and CHO-K1-Rhesus CD155 cells obtained in Example 2 were diluted to 2×10⁶ /mL with PBS and added to a 1.5 mL EP tube at a volume of 100 µL/tube, to which 10 µL/tube of rat serum was added and blocked at 4°C for 30 minutes. The affinity-matured humanized CD155 antibodies obtained in Example 6 at different concentrations were added and incubated at 4° C for 30 min. 1 mL of PBS was added to the EP tub, and the mixture was centrifuged at 4°C, 3500 rpm for 5 min. The supernatant was discarded, and the precipitate was washed again with PBS. After centrifugation, the supernatant was discarded, and the cells were resuspended with 100 µL/tube PBS, to which 1 µL/tube Alexa-647-labeled rat anti-human secondary antibody (Biolegend) was added and incubated at 4°C in the dark for 30 min. The mixture was washed twice with PBS, and the supernatant was discarded after centrifugation. The cells were resuspended with 200 µL/tube PBS and detected by flow cytometry, wherein hIgG1 was used as a control antibody. The assay results were shown in FIG. 6, further demonstrating that the affinity-matured humanized antibody of the present disclosure can bind to CD155.

### Example 10: Effect of affinity-matured humanized CD155 antibody on apoptosis of U-937-CD155 cells

The flow cytometry assay was used to detect the functional characteristics of the affinity-matured humanized CD155 antibody obtained in Example 6. The human CD155 protein (U-937-CD155) was overexpressed in U937 cells (ATCC No. CRL-1593.2) using the pLVX-EF1a-IRES-puro plasmid. The above-mentioned antibody was added and incubated for 4 hours, and then 7-AAD was added. The intensity of the 7-AAD signal was detected to determine the functional characteristics of the CD155 antibody in affecting the apoptosis of U-937-CD155 cells.

HEK293T cells were plated in a six-well plate at 5×10⁵ cells/well and cultured overnight in a DMEM medium without double antibiotics. The medium was discarded before transfection and 1 mL of fresh DMEM medium without double antibiotics was added. pLVX-EF1a-CD155-IRES-pur (the encoding sequence (SEQ ID NO. 45) of human CD155 protein was inserted between the restriction endonuclease sites EcoRI and BamHI of the pLVX-EF1a-IRES-puro vector), pMD2G, and psPAX2 vectors (a total of 3 µg) were added according to a ratio of 2: 1: 1 to 200 µl of serum-free DMEM medium, and 12 µg of polyetherimide (PEI, Polysciences Co. Ltd.) was added. After mixing well, the mixture was stood for 16 min. Then the whole liquid was added to a six-well plate plated with HEK293T cells. After 6 h of culture, the medium was discarded and a fresh complete DMEM medium was added for culture. At 48 hours after transfection, the cell culture supernatant was collected and filtered through a 0.45 µm filter (Millipore) to obtain the virus supernatant. All virus supernatants were added to a 6-well plate containing 1×10⁴ U-937 cells, and polybrene (Sigma) at a final concentration of 4 µg/mL was added and cultured for 12 h. The supernatant was then discarded and a fresh complete DMEM medium was added. The resulting cells were U-937-CD155 cells.

U-937-CD155 cells were diluted to 2×10⁵/mL with complete IMDM and added to a 96-well round-bottom plate at a volume of 100 µL/tube. The above-mentioned affinity-matured humanized CD155 antibody was added thereto and incubated at 37°C, 5% CO₂ for 4 h. 1 µg/tube of 7-AAD was added, and the cell suspension was transferred into a flow tube and detected by flow cytometry. The results were shown in FIG. 8, further showing that the affinity-matured humanized CD155 antibodies of the present disclosure have different functional characteristics, in which v0 can promote apoptosis of U-937-CD155 cells, while v20, v28, and v40 will not induce apoptosis.

### Example 11: Effect of affinity-matured humanized CD155 antibody on anti-cancer activity in mice

The in vivo efficacy assay was used to detect the function of the affinity-matured humanized CD155 antibody obtained in Example 6 in promoting anti-cancer in immune reconstructed mice.
(1) On day 0, human PBMCs were transfused into NCG mice (purchased from Jicui Pharmaceutical) via the tail vein at 1×10⁷ cells per mouse;
(2) On day 4, NCG mice were subcutaneously injected with tumors on the right flank, with 1×10⁶ tumor cells per mouse;
(2) On days 7, 10, 13, and 16, the mice were intraperitoneally injected with the affinity-matured humanized antibody at a dose of 100 µg or 200 µg per mouse; and
(3) The tumor volume was measured every 3 days (on day 7, 10, 13, 16, 19, and 22) after injection of the above antibodies.

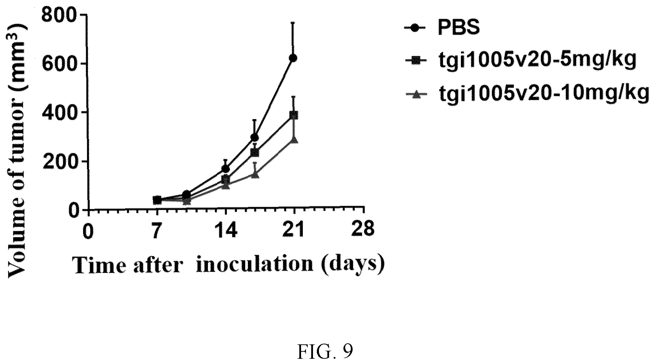

The results were shown in FIG. 9, and it can be seen that the affinity-matured humanized CD155 antibody of the present disclosure can effectively inhibit tumor growth and has anti-cancer function.

Based on the assay results of the above Examples 1 to 11, the antibodies obtained by the present disclosure can bind to human and monkey CD155 proteins; can effectively block the interaction between CD155 and its receptors TIGIT, CD96, and CD226; and can promote anti-cancer in immune reconstructed mice.

The preferred embodiments of the present disclosure are described in detail above. However, the present disclosure is not limited thereto. Within the scope of technical conception of the present disclosure, a variety of simple variations may be made to the technical solutions of the present disclosure, including the combination of various technical features in any other suitable manner. These simple variations and combinations shall be regarded as the contents disclosed by the present disclosure, and all of them fall within the scope of protection of the present disclosure.

In the specification, the description of the reference terms such as "one embodiment", "some embodiments", "example", "specific example", or "some examples" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine and integrate different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. An antibody or antigen binding fragment, comprising a CDR selected from at least one of the following sequences or amino acid sequences having at least 80% identity thereto:
heavy chain variable region CDR sequences: SEQ ID NO: 1 to 3, SEQ ID NO: 7 to 9, SEQ ID NO: 13 to 15, and SEQ ID NO: 19 to 21; and
light chain variable region CDR sequences: SEQ ID NO: 4 to 6, SEQ ID NO: 10 to 12, SEQ ID NO: 16 to 18, and SEQ ID NO: 22 to 24.

2. The antibody or antigen binding fragment according to claim 1, comprising:
a heavy chain variable region CDR1 selected from at least one of amino acid sequences as set forth in SEQ ID NO: 1, SEQ ID NO: 7, SEQ ID NO: 13, and SEQ ID NO: 19, and amino acid sequences in conservative modification form thereof;
a heavy chain variable region CDR2 selected from at least one of amino acid sequences as set forth in SEQ ID NO: 2, SEQ ID NO: 8, SEQ ID NO: 14, and SEQ ID NO: 20, and amino acid sequences in conservative modification form thereof; and
a heavy chain variable region CDR3 selected from at least one of amino acid sequences as set forth in SEQ ID NO: 3, SEQ ID NO: 9, SEQ ID NO: 15, and SEQ ID NO: 21, and amino acid sequences in conservative modification form thereof.

3. The antibody or antigen binding fragment according to claim 1, comprising:
a light chain variable region CDR1 selected from at least one of amino acid sequences as set forth in SEQ ID NO: 4, SEQ ID NO: 10, SEQ ID NO: 16, and SEQ ID NO: 22, and amino acid sequences in conservative modification form thereof;
a light chain variable region CDR2 selected from at least one of amino acid sequences as set forth in SEQ ID NO: 5, SEQ ID NO: 11, SEQ ID NO: 17, and SEQ ID NO: 23, and amino acid sequences in conservative modification form thereof; and
a light chain variable region CDR3 selected from at least one of amino acid sequences as set forth in SEQ ID NO: 6, SEQ ID NO: 12, SEQ ID NO: 18, and SEQ ID NO: 24, and amino acid sequences in conservative modification form thereof.

4. The antibody or antigen binding fragment according to any one of claims 2 to 3, comprising:
1) a heavy chain variable region CDR1 as set forth in SEQ ID NO: 1, a heavy chain variable region CDR2 as set forth in SEQ ID NO: 2, a heavy chain variable region CDR3 as set forth in SEQ ID NO: 3, a light chain variable region CDR1 as set forth in SEQ ID NO: 4, a light chain variable region CDR2 as set forth in SEQ ID NO: 5, and a light chain variable region CDR3 as set forth in SEQ ID NO: 6; or
2) a heavy chain variable region CDR1 as set forth in SEQ ID NO: 7, a heavy chain variable region CDR2 as set forth in SEQ ID NO: 8, a heavy chain variable region CDR3 as set forth in SEQ ID NO: 9, a light chain variable region CDR1 as set forth in SEQ ID NO: 10, a light chain variable region CDR2 as set forth in SEQ ID NO: 11, and a light chain variable region CDR3 as set forth in SEQ ID NO: 12; or
3) a heavy chain variable region CDR1 as set forth in SEQ ID NO: 13, a heavy chain variable region CDR2 as set forth in SEQ ID NO: 14, a heavy chain variable region CDR3 as set forth in SEQ ID NO: 15, a light chain variable region CDR1 as set forth in SEQ ID NO: 16, a light chain variable region CDR2 as set forth in SEQ ID NO: 17, and a light chain variable region CDR3 as set forth in SEQ ID NO: 18; or
4) a heavy chain variable region CDR1 as set forth in SEQ ID NO: 19, a heavy chain variable region CDR2 as set forth in SEQ ID NO: 20, a heavy chain variable region CDR3 as set forth in SEQ ID NO: 21, a light chain variable region CDR1 as set forth in SEQ ID NO: 22, a light chain variable region CDR2 as set forth in SEQ ID NO: 23, and a light chain variable region CDR3 as set forth in SEQ ID NO: 24.

5. The antibody or antigen binding fragment thereof according to claim 4, comprising:
i) a heavy chain variable region having an amino acid sequence with at least 80% homology to at least one of amino acid sequences as set forth in SEQ ID NO. 25, SEQ ID NO. 27, SEQ ID NO. 29, and SEQ ID NO. 31 and amino acid sequences in conservative modification form thereof; and/or
ii) a light chain variable region having an amino acid sequence that with at least 80% homology to at least one of amino acid sequences as set forth in SEQ ID NO. 26, SEQ ID NO. 28, SEQ ID NO. 30, and SEQ ID NO. 32 and amino acid sequences in conservative modification form thereof.

6. The antibody or antigen binding fragment thereof according to any one of claim 5, wherein:
the heavy chain variable region has an amino acid sequence with at least 90%, at least 95%, at least 96%, at least 97%, at least 98%, at least 99%, or 100% homology to the heavy chain variable region selected from i); and
the light chain variable region has an amino acid sequence with at least 90%, at least 95%, at least 96%, at least 97%, at least 98%, at least 99%, or 100% homology to the light chain variable region selected from ii).

7. The antibody or antigen binding fragment thereof according to claim 6, comprising:
1) a heavy chain variable region as set forth in SEQ ID NO: 25 and a light chain variable region as set forth in SEQ ID NO: 26; or
2) a heavy chain variable region as set forth in SEQ ID NO: 27 and a light chain variable region as set forth in SEQ ID NO: 28; or
3) a heavy chain variable region as set forth in SEQ ID NO: 29 and a light chain variable region as set forth in SEQ ID NO: 30; or
4) a heavy chain variable region as set forth in SEQ ID NO: 31 and a light chain variable region as set forth in SEQ ID NO: 32.

8. The antibody or antigen binding fragment according to claim 1, wherein the antibody comprises at least one of a heavy chain constant region and a light chain constant region, and wherein at least a portion of the at least one of the heavy chain constant region and the light chain constant region is derived from at least one of a humanized antibody, a primate-derived antibody, or a mutant thereof.

9. The antibody or antigen binding fragment according to claim 8, wherein the light chain constant region and the heavy chain constant region of the antibody are both derived from a humanized IgG antibody or a mutant thereof.

10. The antibody or antigen binding fragment according to claim 8, wherein the light chain constant region and the heavy chain constant region of the antibody are both derived from a humanized IgG1 antibody, a humanized IgG4 antibody, or a mutant thereof.

11. The antibody or antigen binding fragment according to claim 8, wherein the antibody comprises:
a heavy chain constant region having an amino acid sequence as set forth in SEQ ID NO: 41, SEQ ID NO: 42, or SEQ ID NO: 43; and/or
a light chain constant region having an amino acid sequence as set forth in SEQ ID NO: 44.

12. The antibody or antigen binding fragment according to claim 8, wherein the antibody comprises:
a heavy chain having an amino acid sequence as set forth in any one of SEQ ID NO:33, SEQ ID NO: 35, SEQ ID NO: 37, and SEQ ID NO: 39; and
a light chain having an amino acid sequence as set forth in any one of SEQ ID NO:34, SEQ ID NO: 36, SEQ ID NO: 38, and SEQ ID NO: 40.

13. The antibody or antigen binding fragment according to claim 1, wherein the antibody is a monoclonal antibody, a murine antibody, a chimeric antibody, a humanized antibody, an engineered human antibody, a human antibody, Fv, a single-chain antibody (scFv), Fab, Fab', Fab'-SH, or F(ab')₂.

14. The antibody or antigen binding fragment thereof according to claim 1, wherein the antibody or antigen binding fragment thereof is capable of binding to an amino acid sequence as set forth in SEQ ID NO: 73 and/or SEQ ID NO: 74.

15. A nucleic acid molecule, encoding the antibody or antigen binding fragment according to any one of claims 1 to 14.

16. The nucleic acid molecule according to claim 15, wherein the nucleic acid molecule is DNA.

17. An expression vector, carrying the nucleic acid molecule according to any one of claims 15 to 16.

18. A recombinant cell, carrying the nucleic acid molecule according to any one of claims 5 to 16 or the expression vector according to claim 17; or being capable of expressing the antibody or antigen binding fragment according to any one of claims 1 to 14.

19. The recombinant cell according to claim 18, wherein the recombinant cell is obtained by introducing the expression vector according to claim 17 into a host cell.

20. The recombinant cell according to claim 19, wherein the expression vector is introduced into the host cell by electroporation.

21. The recombinant cell according to claim 19, wherein the recombinant cell is a eukaryotic cell.

22. The recombinant cell according to claim 19, wherein the recombinant cell is a mammalian cell.

23. An immunoconjugate, comprising:
the antibody or antigen binding fragment according to any one of claims 1 to 14; and
a therapeutic agent.

24. A composition, comprising:
the antibody according to any one of claims 1 to 14;
the nucleic acid molecule according to any one of claims 15 to 16;
the expression vector according to any one of claims 17; or
the recombinant cell according to any one of claims 18 to 22.

25. Use of the antibody or antigen binding fragment thereof according to any one of claims 1 to 14, the nucleic acid molecule according to any one of claims 15 to 16, the expression vector according to claim 17, the recombinant cell according to any one of claims 18 to 22, or the composition according to claim 24 in the manufacture of a medicament for preventing and/or treating a CD155-related disease.

26. The use according to claim 25, wherein the CD155-related disease is cancer, autoimmune disease, transplant rejection, or infectious disease.

27. The use according to claim 26, wherein the cancer comprises at least one of melanoma, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, and head-and-neck cancer.

28. A medicament, comprising:
the antibody according to any one of claims 1 to 14;
the nucleic acid molecule according to any one of claims 15 to 16;
the expression vector according to claim 17;
the recombinant cell according to any one of claims 18 to 22; or
the composition according to claim 24.

29. Use of the antibody or antigen binding fragment according to any one of claims 1 to 14 in the manufacture of a kit for detecting CD155.

30. A kit for detecting CD155, comprising the antibody or antigen binding fragment according to any one of claims 1 to 14.

31. A method for preventing and/or treating a CD155-related disease, the method comprising:
administering, to a subject, at least one of:
the antibody or antigen binding fragment according to any one of claims 1 to 14;
the nucleic acid molecule according to any one of claims 15 to 16;
the expression vector according to claim 17;
the recombinant cell according to any one of claims 18 to 22;
the immunoconjugate according to claim 23; or
the composition according to claim 24.

32. The method according to claim 31, wherein the CD155-related disease is cancer, autoimmune disease, transplant rejection, or infectious disease.

33. The method according to claim 32, wherein the cancer comprises at least one of melanoma, lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, and head-and-neck cancer.
